# EUROPEAN PATENT APPLICATION

(11) **EP 4 800 023 A1**
(43) Date of publication of application: **02.09.2026**
(21) Application number: 25160386.6
(22) Date of filing: 26.02.2025
(51) Int. Cl.: C07K 7/06, A61K 38/00

(54) **NOVEL DNAN-TARGETING, ANTI-TUBERCULAR MYCOPLANECIN DERIVATIVES**

(71) Applicant: Helmholtz-Zentrum für Infektionsforschung GmbH, 38124 Braunschweig (DE); Universität des Saarlandes, eine Körperschaft des Öffentlichen Rechts, 66123 Saarbrücken (DE)
(72) Inventor: Rasheed, Sari, 38124 Braunschweig (DE); Herrmann, Jennifer, 38124 Braunschweig (DE); Müller, Rolf, 38124 Braunschweig (DE); Papadopoulos, Emanuel, 66123 Saarbrücken (DE); Kazmaier, Uli, 66123 Saarbrücken (DE); Junk, Lukas, 38124 Braunschweig (DE)
(74) Representative: Forstmeyer, Dietmar

(57) **Abstract**

The present invention relates to novel mycoplanecine derivatives of formula (I) and their use in the treatment of tuberculosis.

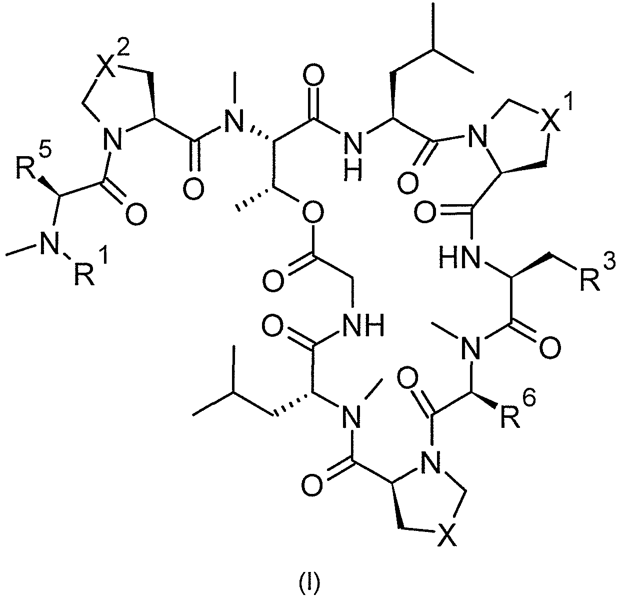

## Description

The present invention relates to novel mycoplanecin derivatives and their use in the treatment of tuberculosis.

Tuberculosis (TB) continues to present a global health obstacle as indicated by the 2022 WHO Global TB Report, with an estimated incidence of 10.6 million new cases in 2021, an increase of 4.5% from 10.1 million in 2020. Additionally, the increasing prevalence of multidrug-resistant *Mycobacterium tuberculosis (MDR-Mtb)* necessitates the development of novel drugs against TB. Currently, only three recently developed novel anti-tubercular agents (bedaquiline (BDQ; Janssen Therapeutics), delamanid (Otsuka Pharmaceutical Co.), and pretomanid (TB Alliance)) have obtained marketing authorization, marking the initial approval of new TB drugs in more than four decades. However, even for these new TB drugs, resistance is on the rise and report showed that bedaquiline resistance acquisition was documented in > 15% of all MDR-TB patients receiving bedaquiline as part of their treatment regimen. Consequently, new drugs addressing novel targets in *Mycobacterium tuberculosis (Mtb)* are desired.

DNA polymerase III sliding clamp (DnaN) is considered as an attractive (and clinically validated) target in *Mtb* since compounds binding to the DNA sliding clamp have a very low risk of resistance development as mutants observed so far are highly impaired in their growth and show a reversible phenotype.

In was an object of the present invention to provide novel compounds for use in the treatment of tuberculosis. It was a further object to provide compounds targeting DnaN.

The present invention provides compounds of formula (I): 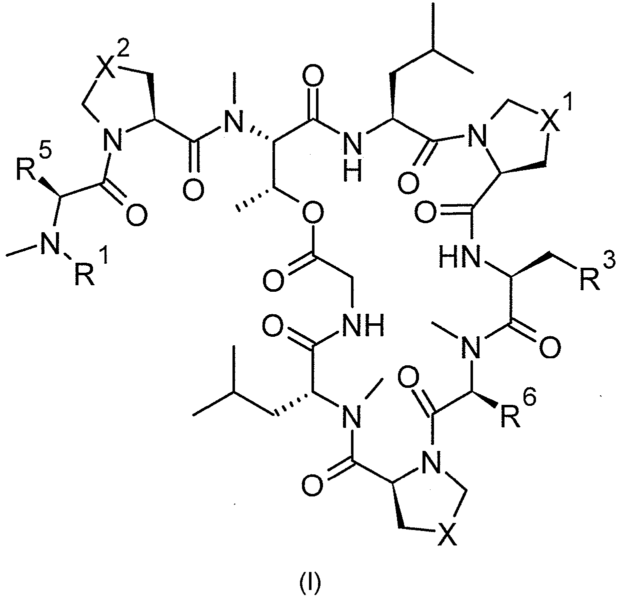 wherein
X is SO₂ or CR⁴R⁴a;
X¹ is SO₂ or CR⁷R^{7a};
X² is SO₂ or CR²R²³;
R¹ is a group of formula -C(=O)-C(=O)-R^{1a}; -C(=O)-R^{1a}; or -C(=O)-O-R^{1a};
R^{1a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R² is hydrogen; fluorine; a C₁₋₄ alkyl group; a C₁₋₄ alkyloxy group; a C₁₋₄ alkenyloxy group; or a cyclohexyl group; and R²⁵ is hydrogen; or fluorine; or R² and R^{2a} together with the carbon atom to which they are bound, form a cyclopropyl group;
R³ is a C₁₋₆ alkyl group;
R⁴ is hydrogen; fluorine; a C₁₋₄ alkyl group; a C₁₋₄ alkyloxy group; a C₁₋₄ alkenyloxy group; or a cyclohexyl group; and R^{4a} is hydrogen; or fluorine; or R⁴ and R^{4a} together with the carbon atom to which they are bound, form a cyclopropyl group;
R⁵ is a C₁₋₆ alkyl group;
R⁶ is a C₁₋₆ alkyl group; a group of formula -(CH₂)₄-N₃; or a group of the following formula: 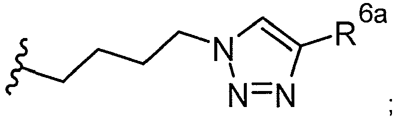
R^{6a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R⁷ is hydrogen; fluorine; a C₁₋₄ alkyl group; a C₁₋₄ alkyloxy group; a C₁₋₄ alkenyloxy group; or a cyclohexyl group; and R^{7a} is hydrogen; or fluorine; or R⁷ and R^{7a} together with the carbon atom to which they are bound, form a cyclopropyl group;
or a salt thereof.

According to a preferred embodiment, the present invention provides compounds of formula (la): 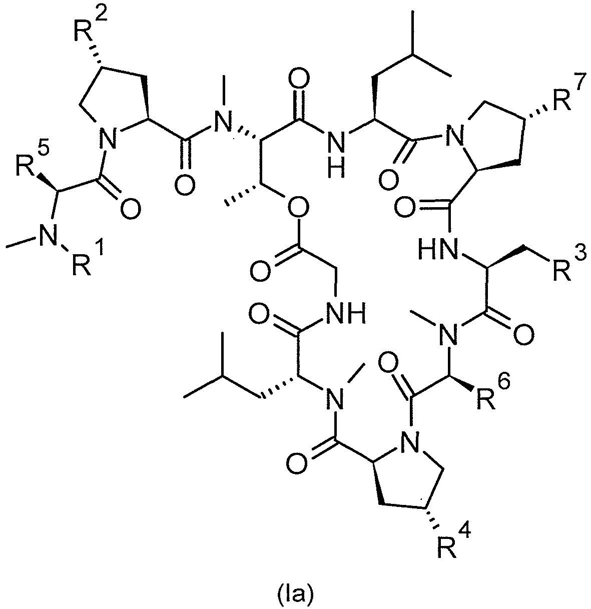 wherein
R¹ is a group of formula -C(=O)-C(=O)-R^{1a}; -C(=O)-R^{1a}; or -C(=O)-O-R^{1a};
R^{1a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R² is hydrogen; a C₁₋₄ alkyl group; a C₁₋₄ alkyloxy group; or a C₁₋₄ alkenyloxy group;
R³ is a C₁₋₆ alkyl group;
R⁴ is hydrogen; a methyl group; a C₁₋₄ alkyloxy group; or a cyclohexyl group;
R⁵ is a C₁₋₆ alkyl group;
R⁶ is a C₁₋₆ alkyl group; a group of formula -(CH₂)₄-N₃; or a group of the following formula: 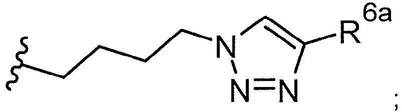
R^{6a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R⁷ is hydrogen; or a methyl group;
or a salt thereof.

Preferably, R^{1a} is a C₁₋₁₂ alkyl group; a C₁₋₁₂ alkenyl group; a C₃₋₇ cycloalkyl group; a C₁₋₆ alkyl-C₃₋₇ cycloalkyl group; an optionally substituted phenyl group; a C₁₋₆ alkyl-phenyl group, wherein the phenyl may optionally be substituted; or a C₁₋₆ alkyl-heterocycloalkyl group, wherein the heterocycloalkyl group contains 5 or 6 ring atoms that are independently selected from C, N, O and S

Further preferably, R^{1a} is a C₁₋₁₂ alkyl group; a C₁₋₁₂ alkenyl group; a C₃₋₇ cycloalkyl group; a C₁₋₆ alkyl-C₃₋₇ cycloalkyl group; or an optionally substituted phenyl group.

Moreover preferably, R^{1a} is a C₁₋₉ alkyl group; a cyclohexyl group; a group of formula - CH₂-CH₂-CH₂-cyclohexyl; or a 4-fluorophenyl group.

Further preferably, R^{1a} is a bicyclo(1.1.1)pentane group.

Further preferably, R^{1a} is an ethyl group.

Moreover preferably, R^{1a} is group of formula -CH₂-CH=CH₂.

Further preferably, R¹ a group of formula -C(=O)-C(=O)-R^{1a}.

Moreover preferably, R¹ a group of formula -C(=O)-C(=O)-R^{1a}, wherein R^{1a} is an ethyl group.

Further preferably, R¹ is a group of formula -C(=O)-R^{1a}.

Moreover preferably, R¹ is a group of formula -C(=O)-R^{1a}, wherein R^{1a} is a C₁₋₉ alkyl group; a cyclohexyl group; a group of formula -CH₂-CH₂-CH₂-cyclohexyl; or a 4-fluorophenyl group.

Further preferably, R¹ is a group of formula -C(=O)-O-R^{1a}.

Moreover preferably, R¹ is a group of formula -C(=O)-O-R^{1a}, wherein R^{1a} is group of formula -CH₂-CH=CH₂.

Further preferably, R² is hydrogen; a methyl group; an ethyl group; a methoxy group; or an ethoxy group.

Especially preferably, R² is a methyl group; or an ethyl group.

Moreover preferably, R³ is an isobutyl group; or an isopropyl group.

Further preferably, R⁵ is a group of formula -CH(CH₃)₂ or -(CH₂)₃CH₃.

Moreover preferably, R⁶ is a C₁₋₆ alkyl group.

Further preferably, R⁶ is a group of formula -CH(CH₃)₂.

Moreover preferably, R⁶ is a group of formula -(CH₂)₄-N₃.

Further preferably, R⁶ is a group of the following formula:

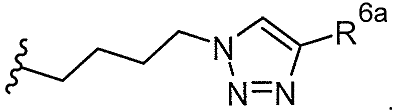

Moreover preferably, R⁷ is hydrogen.

Further preferably, R⁴ is a methyl group; or a cyclohexyl group.

The most preferred compounds of the present invention are the compounds disclosed in the examples, or a salt thereof.

It is further preferred to combine the preferred embodiments of the present invention in any desired manner (e.g., any embodiment for R^{1a} may be combined with any embodiment of R⁵).

The term "optionally substituted" refers to a group which is unsubstituted or substituted by one or more (especially by one, two or three; preferably by one or two) substituents.

If a group (e.g., group R^{1a}) comprises more than one substituent, these substituents are independently selected, i.e., they may be the same or different.

If a group (e.g., group R^{1a}) is substituted by a cyclic group, such as e.g., a cycloalkyl group or a heterocycloalkyl group, this cyclic group may be bonded to this group via a single or double bond or this cyclic group may be annulated or fused to said group.

Examples for substituents are fluorine, chlorine, bromine and iodine and OH, =O, SH, NH₂, -SO₃H, -SO₂NH₂, -COOH, -COOMe, -COMe (Ac), -NHSO₂Me, -SO₂NMe₂, - CH₂NH₂, -NHAc, -SO₂Me, -CONH₂, -CN, -NHCONH₂, -NHC(NH)NH₂, -NOHCH₃, -N₃ and -NO₂ groups. Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₉ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g., F, Cl, Br, I) and groups of formula -OH, =O, -O-C₁₋₆ alkyl (e.g., -OMe, -OEt, -O-*n*Pr, -O-*i*Pr, -O-*n*Bu, -O-*i*Bu and -O-*t*Bu), -NH₂, - NHC₁₋₆ alkyl, -N(C₁₋₆ alkyl)₂, -COOH, -COO-C₁₋₆ alkyl (e.g., -COOMe), -CO-C₁₋₆ alkyl (e.g., -COMe), -COCF₃, -NHSO₂Me, -SO₂NMe₂, -SO₃H, -SO₂NH₂, -CONH₂, -CH₂NH₂, -CN, - C₁₋₆ alkyl (e.g., -Me, -Et, -*n*Pr, -*i*Pr, -*n*Bu, -*i*Bu, -*t*Bu and -CF₃), -C₁₋₆ heteroalkyl, -SH, -S-CO-C₁₋₆ alkyl, -S-C₁₋₆ alkyl, -NHAc, -NO₂, -C≡CH, -NHCONH₂, -SO₂Me, -SO₂CF₃, phenyl, -CO-4-fluorophenyl, -C₃₋₆ cycloalkyl (e.g. cyclopropyl, cyclobutyl) and heterocycloalkyl containing 3 to 6 ring atoms selected from C, N, S and O.

Further preferred substituents are halogen atoms (e.g., F, Cl, Br) and groups of formula - OH, =O, -O-C₁₋₆ alkyl (e.g., -OMe, -OEt, -O-*n*Pr, -O-*i*Pr, -O-*n*Bu, -O-*i*Bu and -O-*t*Bu), - NH₂, -NHC₁₋₆ alkyl, -N(C₁₋₆ alkyl)₂, -COOH, -COO-C₁₋₆ alkyl (e.g., -COOMe), -CO-C₁₋₆ alkyl (e.g., -COMe), -NHSO₂Me, -SO₂NMe₂, -SO₃H, -SO₂NH₂, -CONH₂, -CH₂NH₂, -CN, -C₁₋₆ alkyl (e.g., -Me, -Et, -*n*Pr, -*i*Pr, *-nBu,* -*i*Bu, -*t*Bu and -CF₃), -SH, -S-CO-C₁₋₆ alkyl, -S-C₁₋₆ alkyl, -NHCOO*t*Bu, -NHAc, phenyl, -NO₂, -C≡CH, -NHCONH₂, -SO₂Me and cyclopropyl.

Especially preferred substituents are selected from halogen, -NH₂, -N₃, -N⁺(CH₃)₃, - CH₂NH₂, -CH₃, -COCH₃, -OCOCH₃, -N(CH₃)-CO-CH₃, -NH-CO-CH₃, -COOH, -NO₂, -OH, -NHCH₃, -N(CH₃)₂, -SCH₃, and -CH₂NHCOCH₃.

The suffix "-ene" like e.g., in "phenylene" refers to the corresponding divalent group.

The expression alkyl refers to a saturated, straight-chain or branched hydrocarbon group that contains from 1 to 20 carbon atoms, preferably from 1 to 15 carbon atoms, especially from 1 to 10 (e.g., 1, 2, 3 or 4) carbon atoms, for example a methyl (Me, CH₃), ethyl (Et), *n*-propyl (*n*Pr), *iso*-propyl (*i*Pr), *n*-butyl (*n*Bu), *iso*-butyl (*i*Bu), *sec*-butyl (*s*Bu), *tert*-butyl (*t*Bu), *n*-pentyl, *iso*-pentyl, *n*-hexyl, 2,2-dimethylbutyl or *n*-octyl group.

Especially preferred alkyl groups are C₁₋₆ alkyl groups; moreover preferred alkyl groups are C₁₋₄ alkyl groups.

The expression C₁₋₁₂ alkyl refers to a saturated, straight-chain or branched hydrocarbon group that contains from 1 to 12 carbon atoms. The expression C₁₋₉ alkyl refers to a saturated, straight-chain or branched hydrocarbon group that contains from 1 to 9 carbon atoms. The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl, ethyl, *n*-propyl, *iso*-propyl, *n*-butyl, *iso*-butyl, *sec*-butyl or *tert*-butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g., 2, 3 or 4) carbon atoms, for example an ethenyl (vinyl), propenyl (allyl), isopropenyl, butenyl, ethynyl (acetylenyl), propynyl (e.g., propargyl), butynyl, isoprenyl or hex-2-enyl group. Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or Cl) such as, for example, a 2,2,2-trichloroethyl or a trifluoromethyl group.

The expression heteroalkyl refers to an alkyl, alkenyl or alkynyl group in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or CI).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g., 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-C₁₀ heteroalkyl refers to a heteroalkyl group containing from 1 to 10 carbon atoms and 1, 2, 3, 4, 5 or 6 heteroatoms selected from O, S and/or N (especially O and/or N). The term C₁-C₆ heteroalkyl refers to a heteroalkyl group containing from 1 to 6 carbon atoms and 1, 2, 3 or 4 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N).

Further preferably, the expression heteroalkyl refers to an alkyl group as defined above (straight-chain or branched) in which one or more (preferably 1 to 6; especially preferably 1, 2, 3 or 4) carbon atoms have been replaced by an oxygen, sulfur or nitrogen atom or a CO group or a SO group or a SO₂ group; this group preferably contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen); this group may preferably be substituted by one or more (preferably 1 to 6; especially preferably 1, 2, 3 or 4) fluorine, chlorine, bromine or iodine atoms or OH, =O, SH, =S, NH₂, =NH, N₃, CN or NO₂ groups.

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-SO₂-Y^{a}-, R^{a}-SO₂-N(R^{b})-Y^{a}-, R^{a}-N(R^{b})-Y^{a}-, R^{a}-CO-Y^{a}-, R^{S}-O-CO-Y^{a}-, R^{S}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{a})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, *n*-propyloxy, iso-propyloxy, *n*-butoxy, *tert*-butyloxy, methoxymethyl, ethoxymethyl, -CH₂CH₂OH, -CH₂OH, -SO₂Me, -NHAc, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, propellane (e.g., [1.1.1]propellane), bicyclo(1.1.1)pentane, tetraline, cyclopentylcyclohexyl, fluorocyclohexyl or cyclohex-2-enyl group. Preferably, the expression cycloalkyl refers to a saturated cyclic group that contains one or more rings (preferably 1 or 2), and contains from 3 to 14 ring carbon atoms, preferably from 3 to 10 (especially 3, 4, 5, 6 or 7) ring carbon atoms.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) and 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g., -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings and from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings and from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl (Ph), naphthyl, biphenyl, 2-fluorophenyl, anilinyl, 3-nitrophenyl or 4-hydroxyphenyl group.

The expression heteroaryl refers to an aromatic group that contains one or more rings and from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g., 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g., 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are phenylcyclopentyl, cyclohexylphenyl as well as groups derived from toluene, xylene, mesitylene, styrene, benzyl chloride, o-fluorotoluene, 1H-indene, tetraline, dihydronaphthalene, indanone, cumene, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or a cycloalkyl group containing 3, 4, 5, 6 or 7 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group comprises one or more heteroatoms (preferably selected from O, S or N). A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 3, 4, 5, 6 or 7 ring carbon atoms and/or one or two heterocycloalkyl groups, each containing 3, 4, 5, 6 or 7 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroaryl heterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

The term halogen refers to F, Cl, Br or I.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated or fused or bridged.

The present invention further provides pharmaceutical compositions comprising one or more compounds described herein or a pharmaceutically acceptable salt thereof, optionally in combination with one or more carrier substances and/or one or more adjuvants.

The present invention furthermore provides compounds or pharmaceutical compositions as described herein for use in the treatment of bacterial infections, especially for use in the treatment of tuberculosis.

The present invention further provides a compound as described herein or a pharmaceutical composition as defined herein for the preparation of a medicament for use in the treatment of bacterial infections, especially for use in the treatment of tuberculosis.

According to a moreover preferred embodiment, the present invention provides a method for treating a bacterial infection (especially for treating tuberculosis), which comprises administering to a subject in need of such treatment a therapeutically effective amount of a compound as described herein or a pharmaceutical composition as defined herein.

Examples of salts (especially of pharmacologically acceptable salts) of sufficiently basic compounds are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The solvation/ hydration may occur during the process of production or as a consequence of the hygroscopic nature of the initially water-free compounds. The solvates and/or hydrates may e.g. be present in solid or liquid form.

The therapeutic use of the compounds described herein, their pharmacologically acceptable salts, solvates and hydrates, respectively, as well as formulations and pharmaceutical compositions also lie within the scope of the present invention.

In general, the compounds and pharmaceutical compositions described herein will be administered by using the established and acceptable modes known in the art.

For oral administration, such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g., liquid aerosol), transdermal, for example via an transdermal drug delivery system (TDDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules, one may use excipients as are e.g., vegetable, petroleum, animal or synthetic oils, wax, fat, and polyols. For the production of liquid solutions, emulsions or suspensions or syrups one may use as excipients e.g., water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories, one may use excipients as e.g. vegetable, petroleum, animal or synthetic oils, wax, fat and polyols. For aerosol formulations, one may use compressed gases suitable for this purpose, e.g., oxygen, nitrogen and carbon dioxide. The pharmaceutically useful agents may also contain additives for conservation, stabilization, e.g., UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

### EXAMPLES

### List of Abbreviations

- Ac: Acetyl
- Bn: Benzyl
- Boc: *tert*-Butyloxycarbonyl
- Cbz: Benzyloxycarbonyl
- cHex: Cyclohexane
- Cl: Chemical ionization
- COMU: (1-Cyano-2-ethoxy-2-oxoethylidenaminooxy)dimethylamino-morpholino-carbenium-hexafluorophosphate
- Cy: Cyclohexyl
- CyPro: trans-4-Cyclohexylproline
- DICHED: 1,2-Dicyclohexyl-1,2-ethandiol
- DIPA: Diisopropylamine
- DIPEA: N,N-Diisopropylethylamine
- DMAc: Dimethylacetamide
- DMBA: Dimethylbarbituric acid
- DMF: Dimethylformamide
- DMSO: Dimethylsulfoxide
- EDC: 1-(3-Dimethylaminopropyl)-3-ethylcarbodiimide hydrochloride
- ESI: Electrospray ionization
- Et: Ethyl
- EtOPro: trans-4-Ethoxyproline
- EtPro: trans-4-Ethylproline
- FDPP: Pentafluorphenyl diphenylphosphinate
- Fmoc: Fluorenylmethoxycarbonyl
- Gly: Glycine
- HATU: *O*-(7-Azabenzotriazol-1-yl)-*N*,*N*,*N*'*N*'-tetramethyluronium hexafluorphosphate
- HOAt: 1-Hydroxy-7-azabenzotriazole
- HOBt: 1-Hydroxybenzotriazole
- HoLeu: Homoleucine
- HPLC: high performance liquid chromatography
- HRMS: high resolution mass spectrometry
- IBCF: *iso*-Butylchlorformiate
- IPCF: *iso*-Propylchloroformiate
- LC: liquid chromatography
- LDA: Lithiumdiisopropylamide
- Leu: Leucine
- Lit.: Literature value
- Lys: Lysine
- Me: Methyl
- MeOPro: *trans*-4-Methoxyproline
- MePro: *trans*-4-Methylproline
- MNBA: 2-Methyl-6-nitrobenzoic anhydride
- MS: mass spectrometry
- *n*-Bu: *n*-Butyl
- NMI: *N*-Methylimidazole
- NMM: *N*-Methylmorpholine
- NMR: nuclear magnetic resonance
- Ph: Phenyl
- PPY: 4-Pyrrolidinylpyridine
- Pro: Proline
- PyAOP: (7-Azabenzotriazol-1-yloxy)tripyrrolidino-phosphonium hexafluorophosphate
- R_{f}: Retention factor
- sat.: saturated
- TBS: *tert*-Butyldimethylsilyl
- TBTU: *O*-(Benzotriazol-1-yl)-*N*,*N*,*N*',*N*'-tetramethyl-uronium tetrafluoroborate
- *t*-Bu: *tert*-Butyl
- Tf: Triflyl
- TFA: Trifluoroacetic acid
- THF: Tetrahydrofuran
- Thr: Threonine
- TLC: Thin layer chromatography
- Tren: Tris(2-aminoethyl)amine
- Trt: Trityl
- Ts: Tosyl
- Val: Valine

### General Information

All air and moisture sensitive reactions were carried out in dried glassware (> 100 °C) under N₂ or Ar atmosphere. Anhydrous solvents were purchased from Acros Organics or dried before use (THF was distilled over sodium/benzophenone) and stored under nitrogen atmosphere. The products were purified by column chromatography on silica gel columns (Machery-Nagel 60, 0.063-0.2 mm) or a Grace *Reveleris PREP Chromatography* system or a Büchi *Pure C-815 Flash* system using prepacked columns *RediSep*^{®} *Rf* from *Teledyne Isco.* For reverse-phase chromatography (indicated by C₁₈-SiO₂), a Grace *Reveleris PREP Chromatography* system was used with *Buchi FlashPure Select C18* columns and MeCN/H₂O solvents. Analytical TLC was performed on precoated silica gel plates (Machery-Nagel, Polygram Sil G/UV₂₅₄). Detection was accomplished with UV light (254 nm), KMnO₄ solution, ninhydrin solution or cerium(IV)/ ammonium molybdate solution. Melting points were determined with a MEL-TEMP II (Laboratory devices) apparatus and are uncorrected. ¹H and ¹³C NMR spectra were recorded at 293 or 298 K on a Bruker Avance II 400 MHz spectrometer [¹H 400 MHz and ¹³C 100 MHz], a Bruker Avance I 500 MHz spectrometer [¹H 500 MHz and ¹³C 126 MHz] or a Bruker AV 500 Neo spectrometer [¹H 500 MHz and ¹³C 126 MHz]. Chemical shifts (δ) are reported in parts per million (ppm) relative to TMS or internal solvent signal. Peaks were assigned using (¹H, ¹H)-COSY, (¹H,¹³C)-HSQC and (¹H,¹³C)-HMBC spectra. LC/MS measurements were performed on a Shimadzu system (system controller: SCL-10A, liquid chromatograph: LC-2030C ED Plus, autosampler: SCL-6B, mass spectrometer: LCMS-2020), using an Onyx C₁₈₍₂₎ column (50×4.6 mm, 3 µm particle size) from Phenomenex as the stationary phase. Detection was performed using a diode array detector (190-300 nm) and a mass detector (Shimadzu LCMS-2020) via ESI. Mass spectra were recorded with a Finnigan MAT 95 spectrometer (quadrupole) (CI), a Bruker Daltonics maXis 4G (ESI) and a UHPLC Quadrupole Orbitrap (Q Exactive) mass spectrometer from Thermo Scientific (ESI). Optical rotations were measured with a Jasco P-2000 polarimeter or a Krüss P8000-T80 polarimeter in a thermostated (20 °C ± 1 °C) cuvette, using a sodium vapor lamp (λ = 589 nm) as radiation source. ${\left[α\right]}_{D}^{20}$ values are given in 10⁻¹deg cm² g⁻¹. (*R*,*R*)-DICHED was prepared according to known literature protocols.^{[1,2]}

### Reaction Schemes

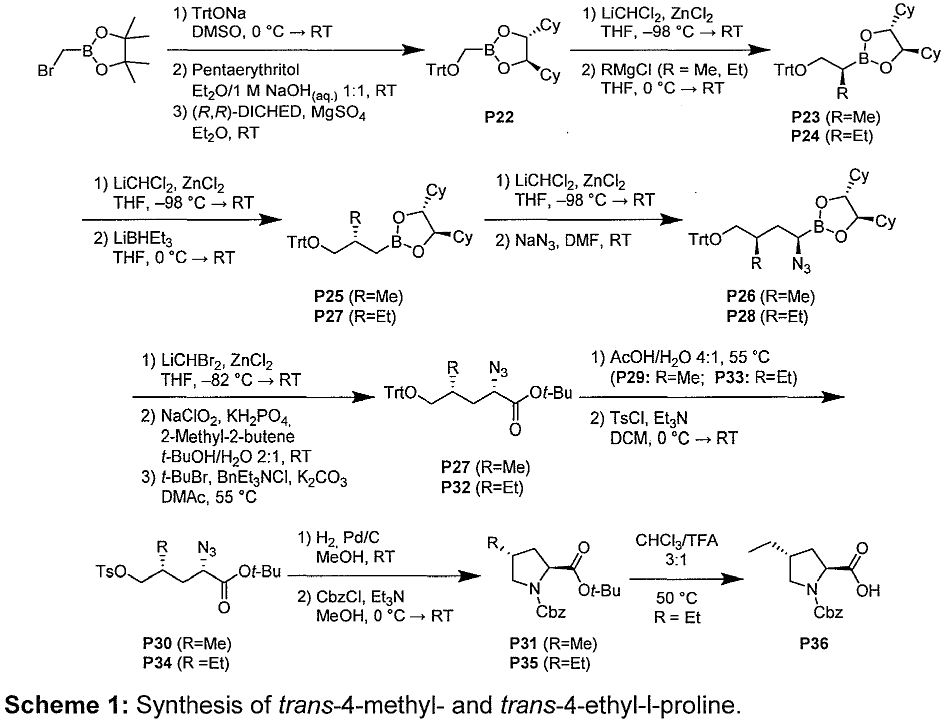

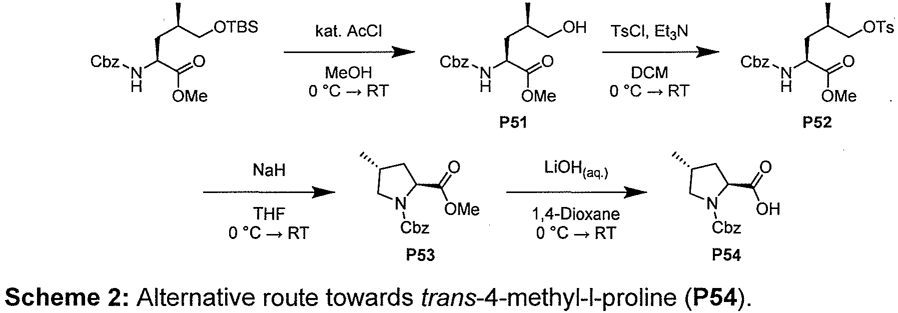

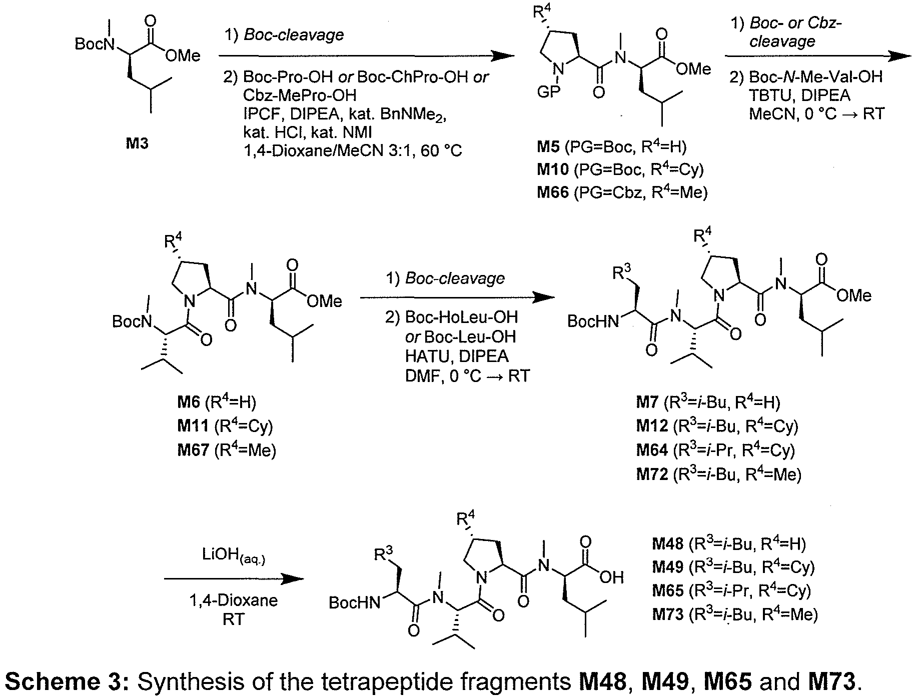

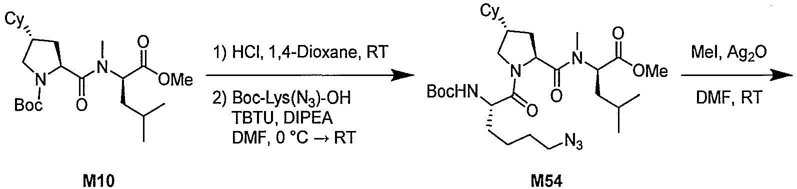

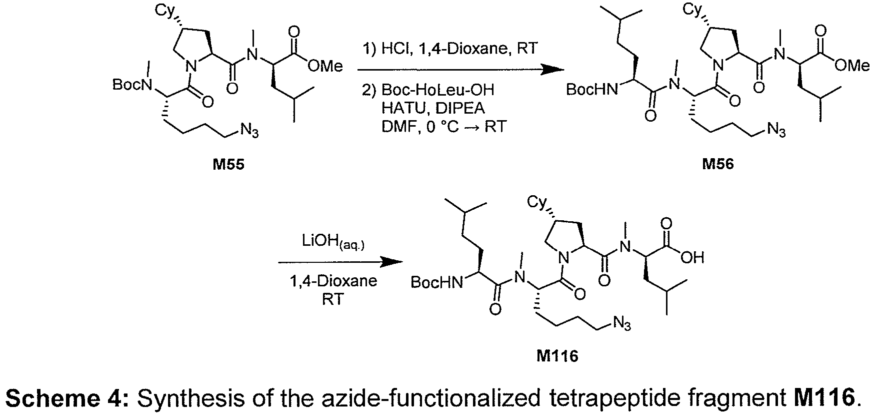

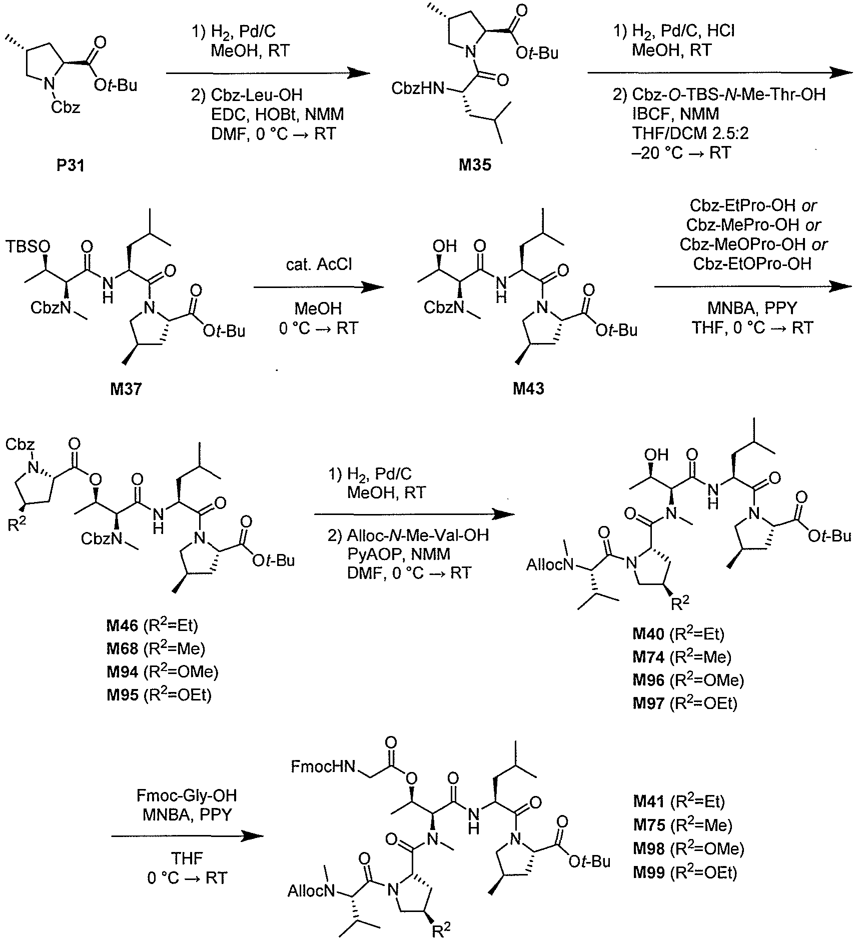

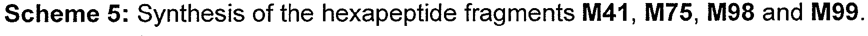

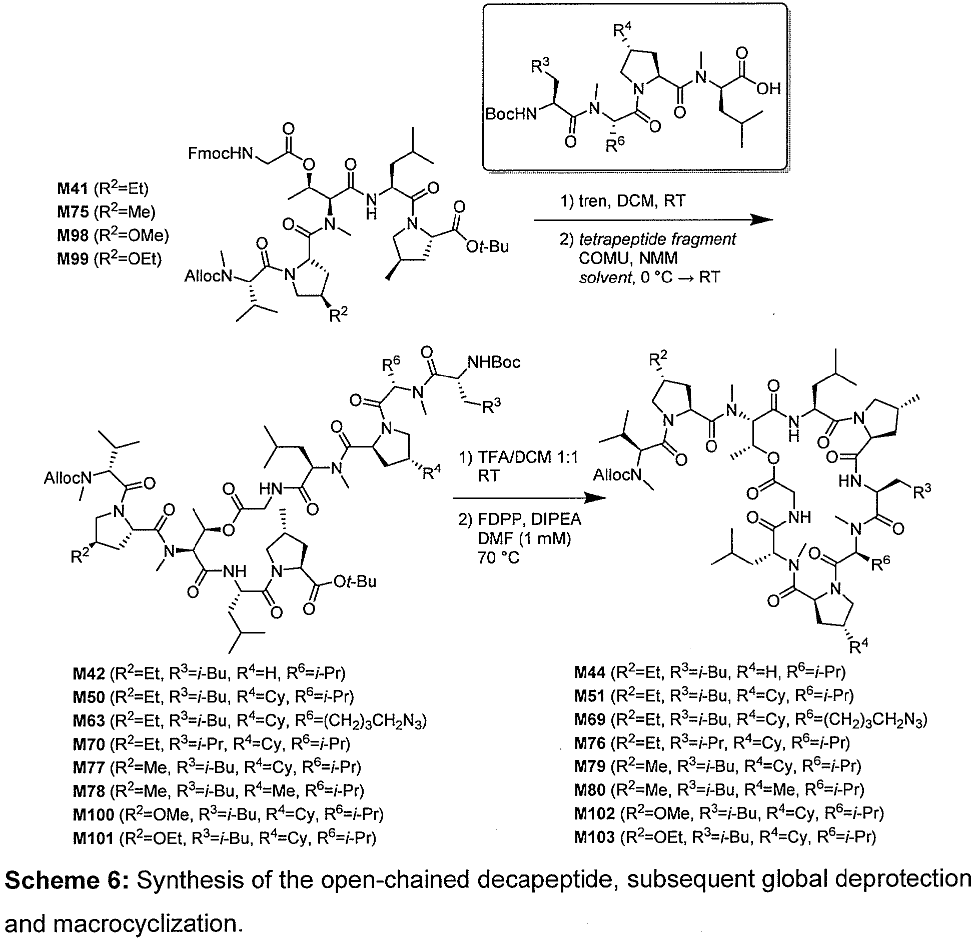

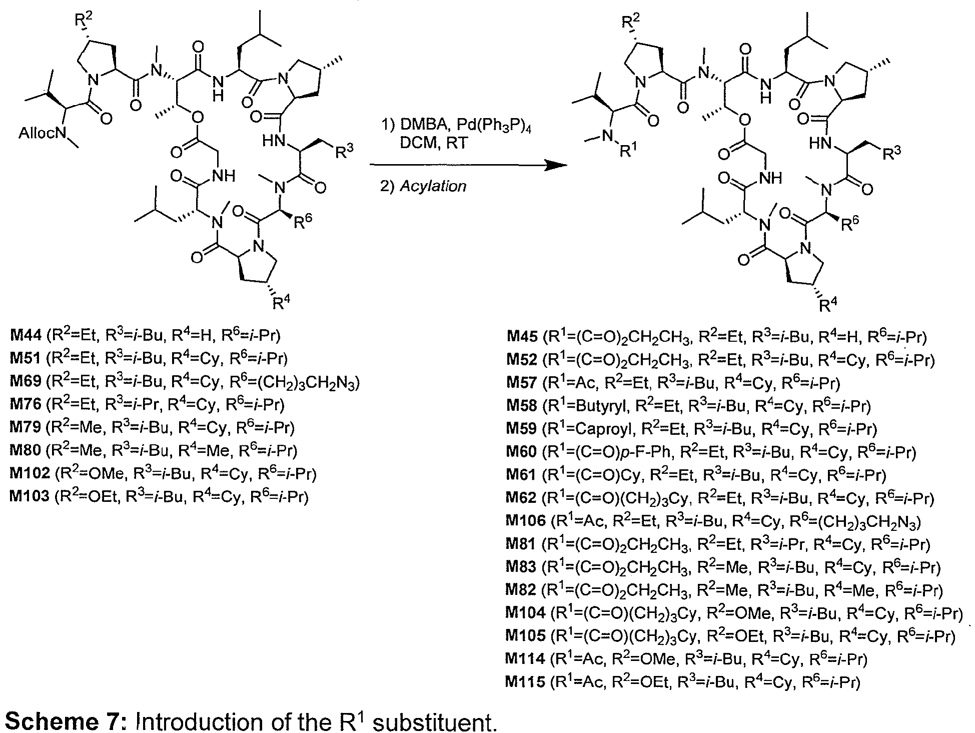

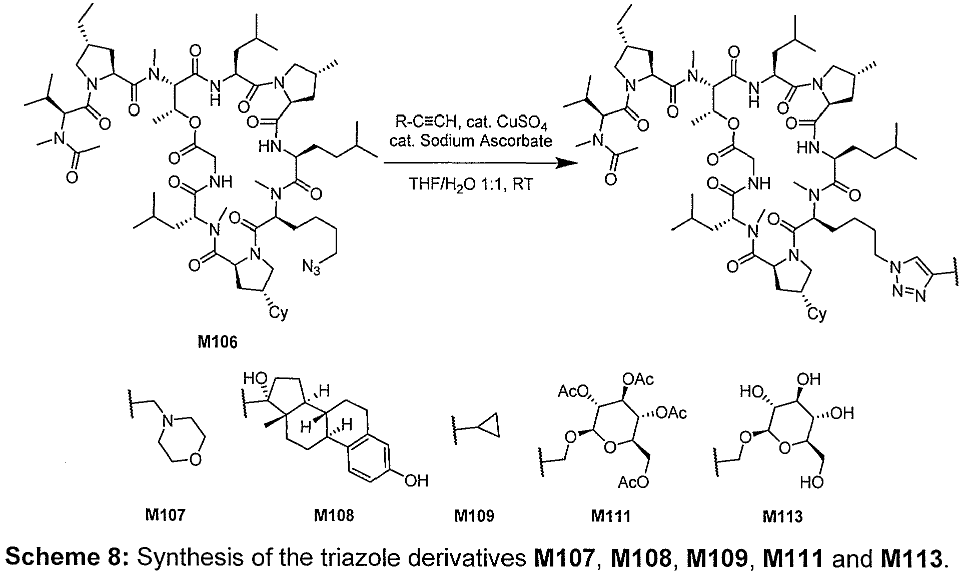

### General Procedures (GP)

### GP1: Matteson Homologation

Under N₂ atmosphere, anhydrous DCM (3.0 eq.) was added to anhydrous THF (1.5 mL/mmol *n*-BuLi) and cooled to -98 °C (MeOH/N₂₍ₗ₎). Subsequently, *n*-BuLi (1.1-1.3 eq., 2.5 M in *n*-hexane) was added slowly along the inner wall of the flask and rinsed with anhydrous THF. After 20 min, a solution of the boronic acid ester (1.0 eq.) in anhydrous THF (1.4 mL/mmol boronic acid ester) was slowly added dropwise at -98 °C, rinsing twice with anhydrous THF. After another 20 min, a suspension of in a high vacuum oven-dried ZnCl₂ (3.0 eq.) in anhydrous THF (0.6 mL/mmol ZnCl₂) was added, followed by warming to RT. The conversion to the α-chloroboronic ester was monitored via ¹H-NMR spectroscopy (usually 2-3 h).

Variant a): One-pot reaction of the α-chloroboronic ester: The reaction mixture was cooled to the indicated temperature and the corresponding nucleophile solution was added dropwise. After complete conversion was observed via ¹H-NMR spectroscopy, the mixture was quenched with sat. NH₄Cl solution and diluted with *n*-pentane. Next, the phases were then separated, and the aqueous phase was extracted once with *n*-pentane. The combined organic phases were dried over MgSO₄, the solvent was removed in vacuo, and the resulting residue was purified by column chromatography.

Variant b): Reaction of the isolated α-chloroboronic ester: The reaction mixture was quenched with sat. NH₄Cl solution and diluted with *n*-pentane. Afterwards, the phases were then separated, and the aqueous phase was extracted once with *n*-pentane. The combined organic phases were dried over MgSO₄, and the solvent was removed in vacuo. Then the resulting residue was dissolved in anhydrous THF under N₂ atmosphere and cooled to the indicated temperature, whereupon the nucleophile solution was added dropwise. After complete conversion was observed via ¹H-NMR spectroscopy, the mixture was quenched with sat. NH₄Cl solution and diluted with *n*-pentane. The phases were then separated, and the aqueous phase was extracted once with *n*-pentane. Lastly, the combined organic phases were dried over MgSO₄, the solvent was removed in vacuo, and the resulting residue was purified by column chromatography.

### GP2: Azide reduction and Cbz cleavage with Pd/C under H₂ atmosphere

In a round-bottom flask, the corresponding azide or *N*-Cbz-protected amino acid/peptide was dissolved in MeOH and Pd/C (10 wt%) was added. The reaction vessel was evacuated six times with a water aspirator vacuum pump until the black suspension began to boil, before the vessel was then flushed with H₂ (balloon). The reaction mixture was stirred under H₂ atmosphere (balloon pressure) until complete conversion was observed via TLC or LC/MS. Subsequently, it was filtered through Celite, rinsed with MeOH and the solvent was removed in vacuo.

### GP3: Methyl Ester Saponification with LiOH

A solution of the corresponding methyl ester (1.0 eq.) in 1,4-dioxane was treated with 1 M LiOH_{(aq.)} (1.1-1.5 eq.) at RT and stirred for 3-18.5 h until complete conversion was observed via LC/MS. Subsequently, the pH was adjusted to approx. 1 with 1 M HCl(aq.), causing a colorless solid to precipitate. Afterwards, the aqueous phase was extracted three times with EtOAc, before the combined organic phases were dried over MgSO₄ and the solvent was removed in vacuo. The resulting residue was purified by column chromatography.

### GP4: N- or O-Methylation of Carbamate-Protected Amino Acids^{[3]}

Under N₂ atmosphere, the *N*-protected amino acid (1.0 eq.) was dissolved in anhydrous THF and cooled to 0 °C. Subsequently, the solution was treated with methyl iodide (3-5 eq.), followed by portionwise addition of NaH (3-6 eq.). After the addition was complete, the reaction mixture was slowly warmed to RT overnight (for the introduction of an ethyl group, ethyl iodide was used instead of methyl iodide).

The reaction mixture was then carefully quenched with H₂O until no further gas evolution was observed. Subsequently, the mixture was extracted once with *n*-pentane, and the aqueous phase was acidified to a pH of approx. 2-3 with 1 M KHSO4_{(aq.)}, resulting in the formation of a colorless precipitate. Next, the aqueous phase was extracted three times with EtOAc, and the combined EtOAc phases were washed once with sat. Na₂S₂O_{3(aq.)} and once with sat. NaCl_{(aq.)} solution, before the now nearly colorless organic phase was dried over MgSO₄ and the solvent was removed in vacuo. If necessary, the resulting residue was purified by column chromatography.

### GP5: Boc-Cleavage of N-Boc-Protected Amino Acid Esters and Peptides

In a round-bottom flask, the N-Boc-protected amine (1.0 eq.) was dissolved in MeOH and cooled to 0 °C. Acetyl chloride (7.5-10 eq.) was added dropwise, and the mixture was subsequently warmed to RT. Alternatively, 1,4-dioxane with the addition of MeOH (7.5-10 eq.) or a 4.0 M solution of HCl in 1,4-dioxane was used as the reaction medium instead of MeOH. After complete conversion was confirmed by TLC or LC/MS (1-6 h), the solvent was removed in vacuo, and the residue was dried under high vacuum.

### GP6: Peptide Coupling with TBTU/HATU/HOBt+EDC/PyAOP/COMU

The carboxylic acid (1.0-2.5 eq.) and amine component (1.0 eq.) were dissolved in the indicated solvent under N₂ or Ar atmosphere and cooled to 0 °C. Subsequently, HOBt/HOAt (1.0-1.4 eq., if necessary), the coupling reagent (1.1-2.5 eq.), and the tertiary amine base (2.2-4.0 eq.) were added sequentially, before the reaction mixture was slowly warmed to RT overnight.

Afterwards, the mixture was diluted with EtOAc and washed with 1 M KHSO_{4(aq.)}, followed by water, sat. NaHCO₃ solution, and sat. NaCl solution, before the organic phase was dried over MgSO₄ and the solvent was removed in vacuo. If DMF was used as the solvent, it was washed with 1 M LiCl_{(aq.)} instead of water. Finally, the resulting residue was purified by column chromatography.

### GP7: MNBA Esterification

A solution of the corresponding alcohol (1.0 eq.) and the corresponding carboxylic acid (1.4-2.0 eq.) in anhydrous THF was treated with PPY (1.1-1.4 eq.) followed by MNBA (1.4-2.0 eq.) under Ar atmosphere at 0 °C. After addition, the reaction mixture was warmed to RT overnight and then concentrated in vacuo. Finally, the resulting residue was purified by column chromatography.

### GP8: Fmoc Deprotection with tren^{[4]}

A solution of the corresponding Fmoc-protected peptide in DCM was treated with tren (10 eq.) at RT and stirred for 1-3 h. Subsequently, the now colorless suspension was diluted with EtOAc and washed twice with water and twice with a phosphate buffer (pH = 5.5). Afterwards, the organic phase was dried over MgSO₄ and the solvent was removed in vacuo.

### GP9: Boc and t-Bu Ester Cleavage with Subsequent Macrocyclization

The corresponding linear peptide (1.0 eq.) was dissolved in a 1:1 mixture of DCM and TFA and stirred at RT for 2-3 h. After complete conversion was observed via LC/MS, the yellow solution was concentrated in vacuo and the residue was co-evaporated three times with CHCl₃.

Subsequently, the globally deprotected peptide was dissolved in anhydrous DMF and added dropwise via syringe pump under N₂ atmosphere to a solution of FDPP (5-10 eq.) and DIPEA (10-21 eq.) in anhydrous DMF at 70 °C (target concentration = 1 mM, V(DMF_{FDPP})/ V(DMF_{peptide}) = 8:2). After complete conversion was observed via LC/MS, the mixture was cooled to RT and concentrated in vacuo. The resulting residue was taken up in EtOAc and washed successively with 1 M HCl_{(aq.)}, 1 M LiCl_{(aq)}, sat. NaHCO₃, and sat. NaCl solution, before being dried over MgSO₄. After the solvent was removed in vacuo, the crude product was purified by column chromatography.

### GP10: Alloc Deprotection

A solution of Alloc-protected amine (1.0 eq.) and DMBA (5-25 eq.) in anhydrous DCM was treated with Pd(PPh₃)₄ (2-7 mol%) under N₂ atmosphere at RT and stirred for 1.5-4 h. The yellow solution was then diluted with EtOAc, washed three times with sat. NaHCO_{3(aq.)} solution, and dried over MgSO₄, before the solvent was removed in vacuo.

### GP11: Acylation via Acid Chloride

To a solution of the corresponding carboxylic acid (6-20 eq.) in anhydrous DCM, oxalyl chloride (6-20 eq.), followed by one drop of anhydrous DMF, was added under N₂ atmosphere, resulting in a vigorous gas evolution. After stirring for 2.5-4 h, this was slowly added dropwise at 0 °C to a solution of the amine deprotected according to **GP10** (1.0 eq.) and DIPEA (10-40 eq.) in anhydrous DCM, forming a white smoke. The reaction mixture was then slowly warmed to RT overnight.

Subsequently, it was diluted with EtOAc and washed successively with 1 M HCl_{(aq.)}, H₂O, sat. NaHCO_{3(aq.)} and sat. NaCl_{(aq.)} solution, before the organic phase was dried over MgSO₄. The solvent was then removed in vacuo and the residue was purified by column chromatography.

### Synthesis of the Amino Acid Building Blocks

**Synthesis of the 4-Alkylprolines^{[5]}**

### (4R,5R)-4,5-Dicyclohexyl-2-[(trityloxy)methyl]-1,3,2-dioxaborolane[P22]^{[6]}

Under N₂ atmosphere, a solution of 49.5 g (190 mmol, 1.05 eq.) of triphenylmethanol in 360 mL anhydrous DMSO was treated portionwise with 8.72 g (60 wt% in mineral oil, 223 mmol, 1.2 eq.) of NaH. After stirring the reaction mixture for 16 h, 40.0 g (181 mmol, 1.0 eq.) of bromomethylboronic acid pinacol ester were added via a transfer cannula at 0 °C, warming to RT after complete addition. After 3 d, 380 mL of sat. NH₄Cl_{(aq.)} solution were added to the reaction mixture (exothermic) and it was extracted twice with 250 mL of Et₂O. The combined organic phases were washed with water and the solvent was removed in vacuo. Next, the resulting residue was dissolved in 500 mL of Et₂O, treated with 500 mL of 1 M NaOH_{(aq.)}, followed by 61.6 g (452 mmol, 2.5 eq.) of pentaerythritol, and then stirred vigorously. After 21 h, the phases were separated and the aqueous phase was carefully neutralized with 1 M HCl_{(aq.)} at 0 °C (pH = 6). The precipitated white solid was filtered off, washed with water, dried under high vacuum, and then suspended in 380 mL of *n*-pentane and treated with 24.3 g (107 mmol, 0.7 eq.) of (*R,R*)-DICHED, followed by 37.7 g (306 mmol, 2.0 eq.) of anhydrous MgSO₄. After 4 h, an additional 3.46 g (15.3 mmol, 0.1 eq.) and after 16.5 h another 3.45 g (15.3 mmol, 0.1 eq.) of (*R,R*)-DICHED were added to the mixture. After complete conversion was observed via TLC (25.5 h), the suspension was filtered and the filter cake was rinsed with Et₂O. The filtrate was freed from the solvent in vacuo and the residue was purified by column chromatography (*n*-Pentane/Et₂O 9:1). In total, 66.6 g (131 mmol, 86 %) of the boronic ester **P22** were isolated as a colorless, crystalline solid.
${\left[α\right]}_{D}^{20} = + 53.9$ (c = 1.0, CHCl₃); Lit.: ${\left[α\right]}_{D}^{20} = + 49.7$ (c = 1.0, CHCl₃)^{[7]}
**Melting range**: 113-116 °C; Lit.: 108-110 °C^{[7]}
**R_{f}** = 0.55 (*n*-Pentane/EtOAc 95:5) 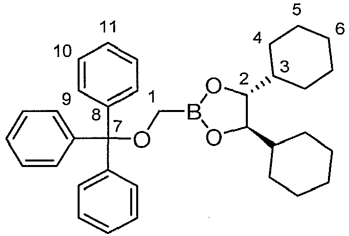
**HRMS** (Cl): Calculated for C₃₄H₄₁BO₃⁺ [M]⁺: 508.3143, found: 508.3147.

### (4R,5R)-4,5-Dicyclohexyl-2-[(S)-1-(trityloxy)propan-2-yl]-1,3,2-dioxaborolane [P23]

According to **GP1a,** 10.0 g (19.7 mmol, 1.0 eq.) of boronic acid ester **P22** were reacted with 3.8 mL (59.1 mmol, 3.0 eq., *ρ* = 1.320 g/mL) anhydrous DCM, 9.0 mL (2.5 M in *n-*hexane, 22.5 mmol, 1.1 eq.) *n*-BuLi, and 8.06 g (59.1 mmol, 3.0 eq.) ZnCl₂. After 2.5 h, the mixture was cooled to 0 °C and 16.4 mL (3.0 M in THF, 49.2 mmol, 2.5 eq.) MeMgCl were slowly added dropwise. After the addition was complete, the mixture was slowly warmed to RT. After 1.5 days, the mixture was cooled again to 0 °C, and another 3.2 mL (3.0 M in THF, 9.60 mmol, 0.5 eq.) of MeMgCl were slowly added dropwise before slowly warming to RT. After 4 h, the mixture was worked up according to **GP1a.** Automated column chromatography (SiO₂, *n*-Pentane/Et₂O 10:0 → 8:2) yielded 9.19 g (17.1 mmol, 87 %) of the boronic acid ester **P23** as a colorless resin.
${\left[α\right]}_{D}^{20} = + 42.2$ (c = 1.0, CHCl₃); Lit.: ${\left[α\right]}_{D}^{20} = + 36.4$ (c = 1.0, CHCl₃)^{[7]}
**R_{f}** = 0.55 (*n*-Pentane/Et₂O 9:1) 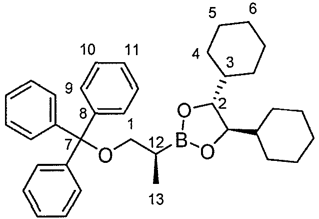
**HRMS** (CI): Calculated for C₃₆H₄₅BO₃⁺ [M]⁺: 536.3456, found: 536.3459.

### (4R,5R)-4,5-Dicyclohexyl-2-[(R)-2-methyl-3-(trityloxy)propyl]-1,3,2-dioxaborolane [P25]

According to **GP1b,** 8.51 g (15.9 mmol, 1.0 eq.) of boronic acid ester **P23** were reacted with 3.0 mL (46.6 mmol, 2.9 eq., *ρ* = 1.320 g/mL) anhydrous DCM, 7.5 mL (2.5 M in *n-*hexane, 18.8 mmol, 1.2 eq.) *n*-BuLi and 6.60 g (48.4 mmol, 3.1 eq.) ZnCl₂. After 3.5 h, aqueous work-up was carried out accordingly and the residue obtained was reacted according to **GP1b** in 40 mL anhydrous THF with 19.0 mL (1.0 M in THF, 19.0 mmol, 1.2 eq.) Superhydride^{®} at 0 °C, adding after 1.5 h another 1.6 mL (1.0 M in THF, 1.60 mmol, 0.1 eq.) Superhydride^{®} at 0 °C. After a further 1.5 h of stirring at RT, the reaction was worked up accordingly. Column chromatographic purification (SiO₂, *n-*Pentane/Et₂O 95:5) yielded 8.27 g (15.0 mmol, 95 %) of the boronic acid ester **P25** as a colorless solid.
${\left[α\right]}_{D}^{20} = + 20.4$ (c = 1.0, CHCl₃)
**Melting range:** 82-85 °C
**R_{f}** = 0.54 (*n*-Pentane/Et₂O 9:1) 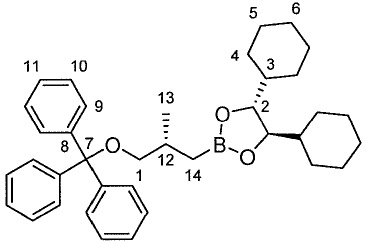
**HRMS** (ESI): Calculated for C₃₇H₄₇BNaO₃⁺ [M+Na]⁺: 573.3510, found: 573.3511.

### (4R,5R)-2-[(1R,3R)-1-Azido-3-methyl-4-(trityloxy)butyl]-4,5-dicyclohexyl-1,3,2-di-oxaborolane [P26]

According to **GP1b,** 8.25 g (15.0 mmol, 1.0 eq.) of boronic acid ester **P25** were reacted with 3.0 mL (46.6 mmol, 3.1 eq., *ρ* = 1.320 g/mL) anhydrous DCM, 7.0 mL (2.5 M in *n-*hexane, 17.5 mmol, 1.2 eq.) *n*-BuLi and 6.23 g (45.7 mmol, 3.1 eq.) ZnCl₂. After 2.5 h, the reaction was worked up aqueously. The residue obtained was dissolved in 74 mL of anhydrous DMF under N₂ atmosphere, to which 5.36 g (82.5 mmol, 5.5 eq.) NaN₃ were added and stirred for 19.5 h.

Contrary to **GP1b**, the yellow suspension was then quenched with sat. NH₄Cl_{(aq.)} solution and diluted with n-pentane and a little H₂O until two clear phases were obtained. After the phases were separated, the aqueous phase was extracted once with *n*-pentane and the combined organic phases were washed once with 1 M LiCl_{(aq.)} before drying over MgSO₄. The solvent was then removed in vacuo and the residue was purified by column chromatography (SiO₂, *n*-Pentane/Et₂O 9:1). A total of 7.44 g (12.3 mmol, 82 %) of the α-azidoboronic acid ester **P26** could be isolated as a colorless, opaque resin.
${\left[α\right]}_{D}^{20} = + 16.0$ (c = 1.0, CHCl₃)
**R_{f}** = 0.35 (*n*-Pentane/EtOAc 95:5) 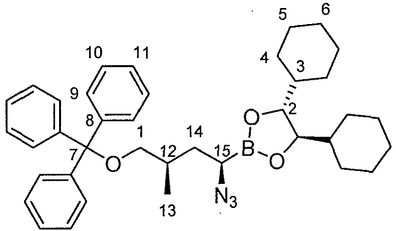
**HRMS** (CI): Calculated for C₃₈H₅₀BNO₃⁺ [M+2H-N₂]⁺: 579.3878, found: 579.3850.

### tert-Butyl (2S,4R)-2-Azido-4-methyl-5-(trityloxy)pentanoate [P27]

Homologation: Under N₂ atmosphere, 2.6 mL (18.4 mmol, 1.5 eq., *ρ* = 0.717 g/mL) DIPA was dissolved in 5.0 mL anhydrous THF and cooled to -40 °C, before 6.5 mL (2.5 M in *n*-hexane, 16.3 mmol, 1.3 eq.) *n*-BuLi was added. Stirring was then continued for 10 min at -40 °C and then for 20 min at RT. The LDA solution thus prepared was slowly added by transfer cannula to a solution of 7.44 g (12.3 mmol, 1.0 eq.) boronic acid ester **P26** and 2.6 mL (37.2 mmol, 3.0 eq., *ρ* = 2.490 g/mL) CH₂Br₂ in 17 mL anhydrous THF at - 82 °C (EtOAc/N₂₍ₗ₎). After stirring further at -82 °C for 1 h, a suspension of 5.12 g (37.6 mmol, 3.1 eq.) of ZnCl₂, heated beforehand under high vacuum, in 23 mL of anhydrous THF was added before subsequent slow warming to RT overnight. After 18 h, sat. NH₄Cl_{(aq.)} solution was added to the reaction mixture and diluted with water and *n-*pentane. The phases were then separated, and the aqueous phase was extracted once with *n*-pentane before the combined organic phases were dried over MgSO4 and the solvent was removed in vacuo.

Oxidation: The previously obtained α-bromoboronic acid ester was dissolved in 238 mL *t*-BuOH and mixed with 52 mL (491 mmol, 40 eq., *ρ* = 0.662 g/mL) 2-methyl-2-butene. A solution of 13.9 g (80 wt%, 123 mmol, 10 eq.) NaClO₂ and 16.8 g (123 mmol, 10 eq.) KH₂PO₄ in 118 mL water was then added dropwise. After 4 d, the yellow emulsion was concentrated in vacuo and acidified with 10 wt% aqueous citric acid solution to a pH of about 3 before extracting three times with Et₂O. The combined etheric phases were washed with sat. Na₂S₂O_{3(aq.)} solution, dried over MgSO₄ and the solvent was removed in vacuo. The residue was then purified by automated column chromatography (SiO₂, *n-*Pentane/EtOAc 10:0 → 7:3).

Esterification:^{[8]} The previously isolated α-azidocarboxylic acid was dissolved in 122 mL DMAc and 56 mL (49.5 mmol, 40 eq., *p* = 1.210 g/mL) *t*-BuBr, 2.80 g (12.3 mmol, 1.0 eq.) BnNEt₃Cl and 44.1 g (319 mmol, 26 eq.) K₂CO₃ were added. The suspension was then stirred for 5 h at 55 °C. After cooling to RT, water was added to the mixture until a clear solution was obtained. This was extracted three times with Et₂O, the combined etheric phases were washed twice with water and once with sat. NaCl_{(aq.)} solution before drying over MgSO₄ and removing the solvent in vacuo. Automated column chromatography (SiO₂, cHex/EtOAc 10:0 → 8:2) afforded 3.60 g (7.63 mmol, 62 %) of the α-azido-*tert*-butyl ester **P27** as a colorless oil.
${\left[α\right]}_{D}^{20} = - 1.6$ (c = 1.0, CHCl₃)
**R_{f}** = 0.69 (*n*-Pentane/EtOAc 1:1) 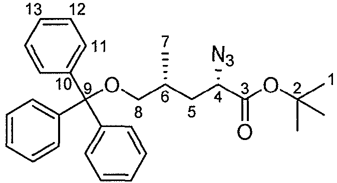
**HRMS** (Cl): Calculated for C₂₉H₃₃N₃O₃⁺ [M-N₂]⁺: 443.2455, found: 443.2475.

### tert-Butyl (2S,4R)-2-Azido-5-hydroxy-4-methylpentanoate [P29]^{[9]}

3.59 g (7.61 mmol, 1.0 eq.) of the α-azido-*tert*-butyl ester **P27** were dissolved in 30 mL AcOH, 7.5 mL water were added and the mixture was heated to 55 °C. After 2 h, the reaction was cooled to RT and carefully neutralized (strong gas evolution) with sat. NaHCO_{3(aq.)} solution. The aqueous phase was extracted three times with Et₂O, and the combined organic phases were dried over MgSO₄. The solvent was then removed in vacuo and the residue was purified by automated column chromatography (cHex/EtOAc 1:0 → 6:4). 1.50 g (6.54 mmol, 86 %) of the alcohol **P29** was isolated as a pale-yellow liquid.
${\left[α\right]}_{D}^{20} = - 12.0$ (c = 1.0, CHCl₃)
**R_{f}** = 0.30 (*n*-Pentane/EtOAc 1:1) 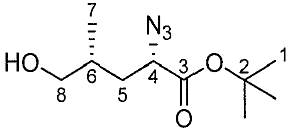
**HRMS** (Cl): Calculated for C₁₀H₁₉N₃NaO₃⁺ [M+Na]⁺: 252.1319, found: 252.1318.

### tert-Butyl (2S,4R)-2-Azido-4-methyl-5-(tosyloxy)pentanoate [P30]

To a solution of 1.50 g (6.52 mmol, 1.0 eq.) of alcohol **P29** in 32 mL DCM, 2.73 mL (19.6 mmol, 3.0 eq., *ρ* = 0.726 g/mL) Et₃N followed by 2.49 g (13.0 mmol, 2.0 eq.) tosyl chloride were added at 0 °C and slowly warmed to RT after the addition was complete. After 3 d, the reaction mixture was washed with sat. NaHCO_{3(aq.)} solution and the aqueous phase was extracted three times with DCM before the combined organic phases were dried over MgSO₄. The solvent was then removed in vacuo and the residue was purified by column chromatography (*n*-Pentane/Et₂O 8:2). 2.48 g (6.47 mmol, 99 %) of the tosylate **P30** was isolated as a yellow oil.
${\left[α\right]}_{D}^{20} = - 19.4$ (c = 1.0, CHCl₃)
**R_{f}** = 0.64 (*n*-Pentane/EtOAc 1:1) 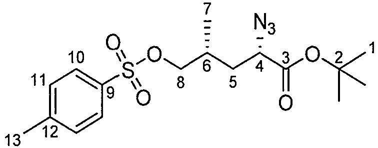
**HRMS** (Cl): Calculated for C₁₇H₂₆N₃O₅S⁺ [M+H]⁺: 384.1588, found: 384.1587.

### tert-Butyl (2S,4R)-N-Benzyloxycarbonyl-4-Methyl-prolinate [P31]

According to **GP2,** 1.11 g (2.89 mmol, 1.0 eq.) of the tosylate **P30** were reacted in 12 mL MeOH with 111 mg Pd/C (10 wt%) under H₂ atmosphere. After 23 h, the residue obtained was dissolved in 12 mL MeOH and cooled to 0 °C. Subsequently, 1.21 mL (8.67 mmol, 3.0 eq., *ρ* = 0.726 g/mL) Et₃N and dropwise 0.46 mL (3.22 mmol, 1.1 eq., *p* = 1.195 g/mL) CbzCl were added to the solution and slowly warmed to RT after the addition was completed. After 3 d, the solvent was removed in vacuo and the residue was taken up in EtOAc. The organic phase was washed three times with 10 wt% aqueous citric acid solution and twice with water. It was then dried over MgSO₄ and the solvent was removed in vacuo. The residue obtained was purified by automated column chromatography (cHex/EtOAc 1:0 → 8:2), resulting in the isolation of 787 mg (1.17 mmol, 85 %) of the proline derivative **P31** as a colorless oil.
${\left[α\right]}_{D}^{20} = - 30.7$ (c = 1.0, CHCl₃)
**R_{f}** = 0.60 (*n*-Pentane/EtOAc 1:1) 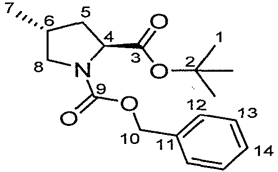
**HRMS** (CI): Calculated for C₁₈H₂₆NO₄⁺ [M+H]⁺: 320.1856, found: 320.1868.

### (4R,5R)-4,5-Dicyclohexyl-2-[(S)-1-(trityloxy)butan-2-yl]-1,3,2-dioxaborolane [P24]

According to **GP1a,** 10.0 g (19.7 mmol, 1.0 eq.) of boronic acid ester **P22** were reacted with 3.8 mL (59.1 mmol, 3.0 eq., *ρ* = 1.320 g/mL) anhydrous DCM, 9.0 mL (2.5 M in *n-*hexane, 22.5 mmol, 1.1 eq.) *n*-BuLi and 8.04 g (59.0 mmol, 3.0 eq.) ZnCl₂. After 3 h, the reaction was cooled to 0 °C and 18.2 mL (2.7 M in THF, 49.2 mmol, 2.5 eq.) EtMgCl was slowly added dropwise. After the addition was completed, the reaction was warmed to RT and cooled back to 0 °C after 4.5 d to slowly add another 5.0 mL (2.0 M in THF, 10.0 mmol, 0.5 eq.) EtMgCl dropwise before warming to RT. After 2 d, the reaction was worked up accordingly. Column chromatographic purification (SiO₂, *n*-Pentane/Et₂O 9:1) afforded 9.60 g (17.4 mmol, 89 %) of the boronic acid ester **P24** as a colorless, waxy solid.
${\left[α\right]}_{D}^{20} = + 44.7$ (c = 1.0, CHCl₃)
**R_{f}** = 0.37 (*n*-Pentane/EtOAc 95:5) 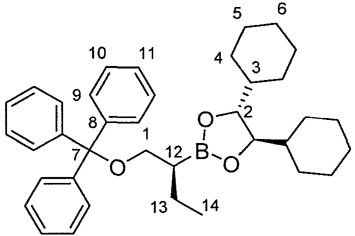
**HRMS** (CI): Calculated for C₃₇H₄₈BO₃⁺ [M+H]⁺: 551.3691, found: 551.3676.

### (4R,5R)-4,5-Dicyclohexyl-2-[(R)-2-methyl-3-(trityloxy)propyl]-1,3,2-dioxaborolane [P27]

According to **GP1b,** 9.59 g (17.4 mmol, 1.0 eq.) boronic acid ester **P24** were reacted with 3.4 mL (52.8 mmol, 3.0 eq., *ρ* = 1.320 g/mL) anhydrous DCM, 8.5 mL (2.5 M in *n*-hexane, 21.3 mmol, 1.2 eq.) *n*-BuLi and 7.25 g (53.2 mmol, 3.1 eq.) ZnCl₂. After 4 h was worked up accordingly and the residue obtained was reacted according to **GP1b** in 44 mL anhydrous THF with 21.0 mL (1.0 M in THF, 21.0 mmol, 1.2 eq.) Superhydride^{®} at 0 °C. After 2.5 h the reaction was worked up accordingly. Column chromatographic purification (SiO₂, *n*-Pentane/Et₂O 9:1) afforded 9.15 g (16.2 mmol, 93 %) of the boronic acid ester **P27** as a colorless, opaque resin.
${\left[α\right]}_{D}^{20} = + 15.3$ (c = 1.0, CHCl₃)
**R_{f}** = 0.41 (*n*-Pentane/EtOAc 95:5) 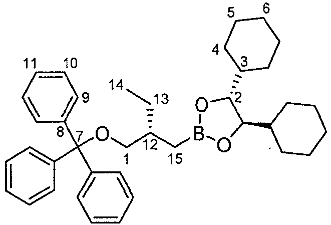
**HRMS** (CI): Calculated for C₁₉H₃BO₃⁺ [M+2H-C₁₉H₁₅]⁺: 323.2752, found: 323.2767.

### (4R,5R)-2-[(1R,3R)-1-Azido-3-((trityloxy)methyl)pentyl]-4,5-dicyclohexyl-1,3,2-dioxaborolane [P28]

According to **GP1b,** 9.14 g (16.2 mmol, 1.0 eq.) boronic acid ester **P27** were reacted with 3.2 mL (49.7 mmol, 3.1 eq., *ρ* = 1.320 g/mL) anhydrous DCM, 8.0 mL (2.5 M in *n*-hexane, 20.0 mmol, 1.2 eq.) *n*-BuLi and 6.73 g (49.4 mmol, 3.1 eq.) ZnCl₂. After 2.5 h, the reaction was worked up aqueously. The residue obtained was dissolved in 80 mL anhydrous DMF under N₂ atmosphere, to which 5.79 g (89.1 mmol, 5.5 eq.) NaN₃ was added and stirred for 16 h.

Contrary to **GP1b,** the yellow-orange suspension was quenched with sat. NH₄Cl(_{aq}.) solution and diluted with *n*-pentane and a little water until two clear phases were obtained. After the phases were separated, the aqueous phase was extracted once with *n*-pentane and the combined organic phases were washed once with 1 M LiCl_{(aq.)} before drying over MgSO₄. The solvent was then removed in vacuo and the residue was purified by column chromatography (SiO₂, *n*-Pentane/Et₂O 9:1). A total of 8.65 g (14.0 mmol, 86 %) of the α-azido-boronic acid ester **P28** was isolated as a pale-yellow, opaque resin.
${\left[α\right]}_{D}^{20} = + 10.5$ (c = 1.0, CHCl₃)
**R_{f}** = 0.38 (*n*-Pentane/EtOAc 95:5) 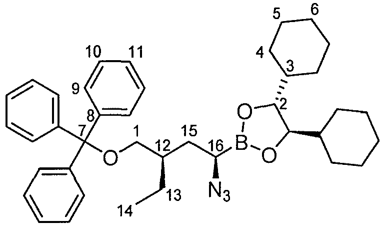
**HRMS** (ESI): Calculated for C₃₉H₅₀BN₃NaO₃⁺ [M+Na]⁺: 642.3837, found: 642.3837.

### tert-Butyl (2S,4R)-2-Azido-4-((trityloxy)methyl)hexanoate [P32]

Homologation: Under N₂ atmosphere, 2.5 mL DIPA (17.7 mmol, 1.5 eq., *ρ* = 0.717 g/mL) were dissolved in 4.9 mL anhydrous THF and cooled to -40 °C before 6.5 mL (2.5 M in *n*-hexane, 16.3 mmol, 1.3 eq.) of *n*-BuLi was added. Stirring was then continued for 10 min at -40 °C and then for 20 min at RT. The LDA solution thus prepared was slowly added by transfer cannula to a solution of 7.37 g (11.9 mmol, 1.0 eq.) of boronic acid ester **P28** and 2.5 mL (35.8 mmol, 3.0 eq., *ρ* = 2.490 g/mL) CH₂Br₂ in 17 mL anhydrous THF at -82 °C (EtOAc/N_{2(I)}). After stirring further at -82 °C for 1 h, a suspension of 4.96 g (36.4 mmol, 3.1 eq.) of ZnCl₂, heated under high vacuum beforehand, in 22 mL of anhydrous THF was added, followed by slow warming to RT overnight. After 16 h, sat. NH₄Cl_{(aq.)} was added to the reaction mixture and diluted with n-pentane and a little H₂O and. The phases were then separated, and the aqueous phase was extracted once with *n*-pentane before the combined organic phases were dried over MgSO₄ and the solvent was removed in vacuo.

Oxidation: The previously obtained α-bromoboronic acid ester was dissolved in 225 mL *t*-BuOH and mixed with 50 mL (472 mmol, 40 eq., *ρ* = 0.662 g/mL) 2-methyl-2-butene. A solution of 13.5 g (80 wt%, 120 mmol, 10 eq.) NaClO₂ and 16.2 g (120 mmol, 10 eq.) KH₂PO₄ in 114 mL water was then added dropwise. After 4 d, the yellow emulsion was concentrated in vacuo and acidified with 10 wt% aqueous citric acid solution to a pH of about 3 before extracting three times with Et₂O. The combined etheric phases were washed with sat. Na₂S₂O_{3(aq.)} solution, dried over MgSO₄ and the solvent was removed in vacuo. The residue was then purified by automated column chromatography (SiO₂, cHex/EtOAc 10:0 → 6:4).

Esterification:^{[8]} The previously isolated α-azidocarboxylic acid was dissolved in 106 mL DMAc and 48 mL (424 mmol, 36 eq., *p* = 1.210 g/mL) *t*-BuBr, 2.40 g (10.5 mmol, 0.9 eq.) BnNEt₃Cl and 37.9 g (274 mmol, 23 eq.) K₂CO₃ were added. The suspension was then stirred for 4.5 h at 55 °C. After cooling to RT, water was added to the mixture until a clear solution was obtained. This was extracted three times with Et₂O, the combined etheric phases were washed twice with water and once with sat. NaCl_{(aq.)} solution before drying over MgSO₄ and removing the solvent in vacuo. Automated column chromatography (SiO₂, cHex/EtOAc 10:0 → 8:2) afforded 2.83 g (5.83 mmol, 49 %) of the *α*-azido-*tert-*butyl ester **P32** as a colorless oil.
${\left[α\right]}_{D}^{20} = - 5.4$ (c = 1.0, CHCl₃)
**R_{f}** = 0.60 (*n*-Pentane/EtOAc 75:25) 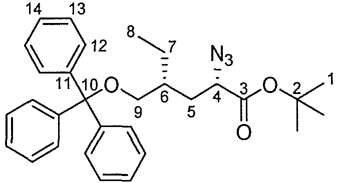
**HRMS** (ESI): Calculated for C₃₀H₃₅N₃NaO₃⁺ [M+Na]⁺: 508.2571, found: 508.2566.

### tert-Butyl (2S,4R)-2-Azido-4-(hydroxymethyl)hexanoate [P33]^{[9]}

2.77 g (5.70 mmol, 1.0 eq.) of the α-azido-*tert*-butyl ester **P32** were dissolved in 23 mL AcOH, mixed with 5.8 mL H₂O and heated to 55 °C. After 3.5 h, the reaction was cooled to RT and carefully neutralized (strong gas evolution) with sat. NaHCO_{3(aq.)} solution. The aqueous phase was extracted three times with Et₂O, and the combined organic phases were dried over MgSO₄. The solvent was then removed in vacuo and the residue was purified by automated column chromatography (cHex/EtOAc 1:0 → 6:4). 1.14 g (4.69 mmol, 82 %) of the alcohol **P33** was isolated as a pale-yellow liquid.
${\left[α\right]}_{D}^{20} = - 38.6$ (c = 1.0, CHCl₃) **R_{f}** = 0.30 (*n*-Pentane/Et₂O 1:1) 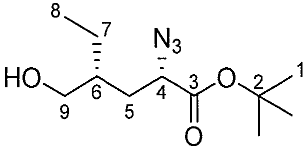
**HRMS** (CI): Calculated for C₁₁H₂₂N₃O₃⁺ [M+H]⁺: 244.1656, found: 244.1650.

### tert-Butyl (2S,4R)-2-Azido-4-((tosyloxy)methyl)hexanoate [P34]

To a solution of 1.13 g (4.65 mmol, 1.0 eq.) of the alcohol **P33** in 23 mL DCM, 1.95 mL (14.0 mmol, 3.0 eq., *ρ* = 0.726 g/mL) Et₃N followed by 1.79 g (9.37 mmol, 2.0 eq.) tosyl chloride were added at 0 °C and slowly warmed to RT after the addition was complete. After 3 d, the reaction mixture was washed with sat. NaHCO_{3(aq.)} solution and the aqueous phase was extracted three times with DCM before the combined organic phases were dried over MgSO₄. The solvent was then removed in vacuo and the residue was purified by column chromatography (*n*-Pentane/Et₂O 9:1 → 8:2). 1.74 g (4.37 mmol, 94 %) of the tosylate **P34** was isolated as a yellow oil.
${\left[α\right]}_{D}^{20} = - 28.8$ (c = 1.0, CHCl₃)
**R_{f}** = 0.64 (*n*-Pentane/EtOAc 1:1) 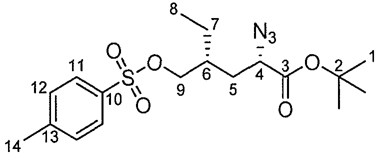
**HRMS** (CI): Calculated for C₁₈H₂₈N₃O₅S⁺ [M+H]⁺: 398.1744, found: 398.1748.

### tert-Butyl (2S,4R)-N-benzyloxycarbonyl-4-ethyl-prolinate [P35]

According to **GP2,** 922 mg (2.32 mmol, 1.0 eq.) of the tosylate **P34** were reacted in 12 mL MeOH with 99.1 mg Pd/C (10 wt%) under H₂ atmosphere. After 1.5 d, the resulting residue was dissolved in 12 mL MeOH and cooled to 0 °C. Subsequently, 970 µL (6.96 mmol, 3.0 eq., *ρ* = 0.726 g/mL) Et₃N and 361 µL (2.53 mmol, 1.1 eq., *p* = 1.195 g/mL) CbzCl were added dropwise to the solution and slowly warmed to RT after the addition was completed. After 3 h, the solvent was removed in vacuo and the residue was taken up in EtOAc. The organic phase was washed three times with 10 wt% aqueous citric acid solution and twice with water. It was then dried over MgSO₄, and the solvent was removed in vacuo. Lastly, the residue obtained was purified by automated column chromatography (cHex/EtOAc 100:0 → 75:25), resulting in the isolation of 702 mg (2.10 mmol, 91 %) of the proline derivative **P35** as a colorless oil.
${\left[α\right]}_{D}^{20} = - 28.0$ (c = 1.0, CHCl₃)
**R_{f}** = 0.65 (*n*-Pentane/EtOAc 1:1) 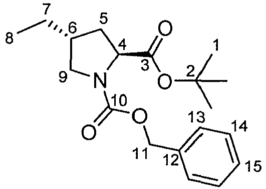
**HRMS** (CI): Calculated for C₁₉H₂₈NO₄⁺ [M+H]⁺: 334.2013, found: 334.2004.

### (2S,4R)-N-Benzyloxycarbonyl-4-ethyl-proline [P36]

1.12 g (3.37 mmol, 1.0 eq.) of the proline derivative **P35** were dissolved in a mixture of 17 mL CHCl₃ and 5.6 mL TFA and heated to 50 °C. After 6 h, the yellow solution was cooled to RT, the solvent was removed in vacuo and the residue was co-evaporated three times with toluene. Automated column chromatography (cHex/EtOAc/AcOH 100:0:0 → 50:50:2) afforded 841 mg (3.03 mmol, 90 %) of the carboxylic acid **P36** as a colorless oil.
${\left[α\right]}_{D}^{20} = - 37.7$ (c = 1.0, CHCl₃)
**R_{f}** = 0.43 (*n*-Pentane/EtOAc 1:1) 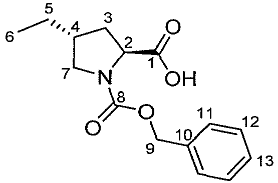
**HRMS** (ESI): Calculated for C₁₅H₂₀NO₄⁺ [M+H]⁺: 278.1387, found: 278.1384.

### Methyl (2S,4R)-2-[(Benzyloxycarbonyl)amino]-5-hydroxy-4-methylpentanoate [P51]^{[10]}

Under N₂ atmosphere, 2.56 g (6.26 mmol, 1.0 eq.) of methyl (2*S*,4*R*)-2-[((benzyloxy)-carbonyl)amino]-5-((*tert*-butyldimethylsilyl)oxy)-4-methylpentanoate^{[11]} was dissolved in 21 mL anhydrous MeOH and cooled to 0 °C before 66.8 µL (940 µmol, 0.15 eq., *ρ* = 1.104 g/mL) acetyl chloride was added. The solution was then slowly warmed to RT overnight and diluted with EtOAc after 18 h. It was then washed with sat. NaHCO_{3(aq.)} solution and water. Next, it was dried over MgSO₄, and the solvent was removed in vacuo. Automated column chromatography (SiO₂, cHex/EtOAc 10:0 → 4:6) of the residue afforded 1.59 g (5.38 mmol, 86 %) of the alcohol **P51** as a pale pink oil.
${\left[α\right]}_{D}^{20} = + 22.2$ (c = 0.5, CHCl₃)
**R_{f}** = 0.18 (*n*-Pentane/EtOAc 1:1) 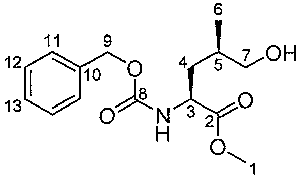
**HRMS** (ESI): Calculated for C₁₅H₂₂NO₅⁺ [M+H]⁺: 296.1492, found: 296.1479.

### Methyl (2S,4R)-2-[(Benzyloxycarbonyl)amino]-4-methyl-5-(tosyloxy)pentanoate [P52]

To a solution of 602 mg (2.04 mmol, 1.0 eq.) of the alcohol **P51** in 6.8 mL DCM was added 881 µL (6.32 mmol, 3.1 eq., *ρ* = 0.726 g/mL) Et₃N at 0 °C, followed by 777 mg (4.08 mmol, 2.0 eq.) tosyl chloride. The mixture was slowly warmed to RT, and after 21.5 h the now brown suspension was diluted with EtOAc and washed successively with 1 M HCl_{(aq.)}, water, sat. NaHCO_{3(aq.)} and sat. NaCl_{(aq.)} solution. The organic phase was then dried over MgSO₄, and the solvent was removed in vacuo. After automated column chromatography (SiO₂, cHex/EtOAc 10:0 → 6:4) of the residue, 797 mg (1.77 mmol, 87 %) of the tosylate **P52** could be isolated as a yellow oil.
${\left[α\right]}_{D}^{20} = + 10.0$ (c = 0.5, CHCl₃)
**R_{f}** = 0.51 (*n*-Pentane/EtOAc 1:1) 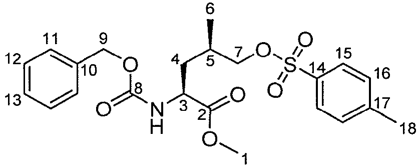
**HRMS** (ESI): Calculated for C₂₂H₂₈NO₇S⁺ [M+H]⁺: 450.1581, found: 450.1559.

### Methyl (2S,4R)-N-Benzyloxycarbonyl-4-ethyl-prolinate [P53]

To a solution of 1.70 g (3.78 mmol, 1.0 eq.) of tosylate **P52** in 13 mL anhydrous THF, 150 mg (60 wt%, 3.75 mmol, 1.0 eq.) NaH was added portionwise at 0 °C under N₂ atmosphere. After complete addition, the mixture was stirred at RT for 2 h before being carefully quenched with water. Subsequently, the mixture was extracted three times with Et₂O, the combined organic phases were dried over MgSO₄, and the solvent was removed in vacuo. Automated flash chromatography of the crude product afforded 877 mg (3.16 mmol, 84 %) of the proline derivative **P53** as a colorless oil. In addition, 131 mg (291 µmol, 8 %) of the tosylate **P52** could be recovered.
${\left[α\right]}_{D}^{20} = - 43.8$ (c = 0.5, CHCl₃)
**R_{f}** = 0.45 (*n*-Pentane/EtOAc 1:1) 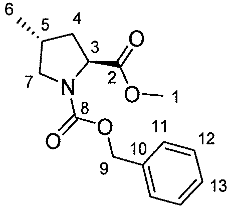
**HRMS** (ESI): Calculated for C₂₂H₂₈NO₇S⁺ [M+H]⁺: 278.1387, found: 278.1383.

### (2S,4R)-N-Benzyloxycarbonyl-4-methyl-proline [P54]

According to **GP3,** 420 mg (1.52 mmol, 1.0 eq.) of the proline derivative **P53** were reacted with 1.67 mL (1.0 M, 1.67 mmol, 1.1 eq.) LiOH_{(aq.)} in 5.2 mL 1,4-dioxane for 4 h. Automated column chromatography (C₁₈-SiO₂, H₂O/MeCN (+0.1 % HCOOH) 9:1 → 0:10) afforded 391 mg (1.49 mmol, 98 %) of carboxylic acid **P54** as a colorless oil.
${\left[α\right]}_{D}^{20} = - 40.8$ (c = 0.5, CHCl₃) 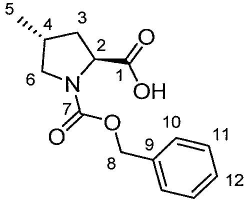
**HRMS** (ESI): Calculated for C₁₄H₁₈NO₄⁺ [M+H]⁺: 264.123, found: 264.1229.

### Synthesis of the other Amino Acid Building Blocks

### N-tert-Butyloxycarbonyl-N-methyl-d-leucine [M2]

According to **GP4,** 5.81 g (25.1 mmol, 1.0 eq.) Boc-d-Leu-OH, 4.60 mL (73.6 mmol, 2.9 eq., *ρ* = 2.270 g/mL) methyl iodide and 6.00 g (60 wt% in mineral oil, 151 mmol, 6.0 eq.) NaH were reacted in 80 mL anhydrous THF for 19 h. Contrary to **GP4,** Et₂O was used for extraction instead of EtOAc. A total of 5.77 g (23.5 mmol, 94 %) of Boc-*N*-Me-d-Leu-OH **(M2)** was isolated as a yellow oil.
${\left[α\right]}_{D}^{20} = + 28.5$ (c = 1.0, CHCl₃); Lit.: ${\left[α\right]}_{D}^{20} = + 33.5$ (c = 0.86, CHCl₃)^{[12]} 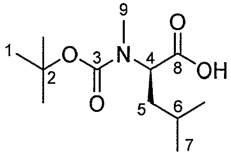
**HRMS** (ESI): Calculated for C₁₂H₂₄NO₄⁺ [M+H]⁺: 246.1700, found: 246.1698.

### Methyl N-tert-butyloxycarbonyl-N-methyl-d-leucinate [M3]

A solution of 5.73 g (23.4 mmol, 1.0 eq.) Boc-N-Me-d-Leu-OH (M2) in 24 mL DMF was treated with 9.69 g (70.1 mmol, 3.0 eq.) K₂CO₃ and 3.65 mL (58.4 mmol, 2.5 eq., *ρ* = 2.270 g/mL) methyl iodide at 0 °C and slowly warmed to RT after addition. After 19 h, the mixture was diluted with EtOAc and washed twice with sat. NaHCO_{3(aq.)} and once with sat. NaCl_{(aq.)} solution. The mixture was then dried over MgSO₄, and the solvent was removed in vacuo. Automated column chromatography (cHex/EtOAc 1:0 → 1:1) afforded 5.59 g (21.6 mmol, 92 %) of Boc-*N*-Me-d-Leu-OMe **(M3)** as a pale-yellow liquid.
${\left[α\right]}_{D}^{20} = + 38.6$ (c = 1.0, CHCl₃)
**R_{f}** = 0.50 (*n*-Pentane/EtOAc 8:2) 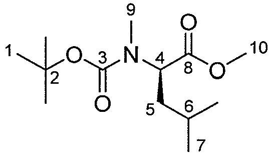
**HRMS** (ESI): Calculated for C₁₃H₂₆NO₄⁺ [M+H]⁺: 260.1856, found: 260.1852.

### N-tert-butyloxycarbonyl-trans-4-Cyclohexyl -I-proline [M9]

To a suspension of 1.91 g (9.69 mmol, 1.0 eq.) *trans*-I-CyPro-OH and 2.45 g (29.2 mmol, 3.0 eq.) NaHCO₃ in a mixture of 12 mL H₂O and 10 mL THF was slowly added a solution of 2.33 g (10.7 mmol, 1.1 eq.) Boc₂O in 2 mL THF at 0 °C. After the addition was complete, the reaction mixture was slowly warmed to RT and stirred for 14 h. The reaction mixture was then extracted twice with Et₂O, and the aqueous phase was acidified with 1 M HCl_{(aq.)} to a pH of 1, whereby a colorless solid precipitated. The suspension was extracted three times with Et₂O, the combined etheric phases were dried over MgSO₄, and the solvent was removed in vacuo. Drying in high vacuum afforded 2.69 g (9.06 mmol, 93 %) of the Boc-protected amino acid M9 as a colorless solid. ${\left[α\right]}_{D}^{20} = - 48.3$ -48.3 (c = 1.0, CHCl₃) Melting range: 123-125 °C

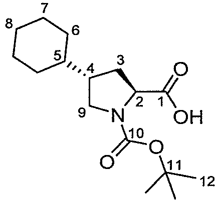

### N-Benzyloxycarbonyl-O-tert-butyldimethylsilyl-I-threonine [M13]^{[13]}

Under N₂ atmosphere, a solution of 5.56 g (81.7 mmol, 3.0 eq.) imidazole in 7 mL anhydrous DMF was added to a solution of 6.89 g (27.2 mmol, 1.0 eq.) Cbz-I-Thr-OH in 16 mL anhydrous DMF at 0 °C, followed by a solution of 12.3 g (81.6 mmol, 3.0 eq.) TBS-Cl in 22 mL anhydrous DMF, and warmed to RT after complete addition. After 27 h, the yellow solution was poured into approx. 300 mL ice water and extracted three times with Et₂O and dried over Na₂SO₄. After the solvent was removed in vacuo, the residue was dissolved in 54 mL THF, mixed with 54 mL 0.5 M KOH_{(aq.)} and stirred for 4 h at 0 °C. The mixture was then extracted once with Et₂O, whereby this organic phase was discarded. The aqueous phase was acidified with 1 M HCl_{(aq.)} solution to a pH of 2-3 and then extracted three times with Et₂O. Finally, the combined etheric phases were washed once with sat. NaCl_{(aq.)} solution and dried over Na₂SO₄. After removing the solvent in vacuo, 9.38 g (25.5 mmol, 94 %) of Cbz-O-TBS-I-Thr-OH (**M13**) was isolated as a colorless solid.
${\left[α\right]}_{D}^{20} = + 10.1$ (c = 1.0, CHCl₃); Lit.: ${\left[α\right]}_{D}^{23} = + 13.2$ (c = 1.0, CHCl₃)^{[14]}
**R_{f}** = 0.79 (DCM/MeOH 95:5)
**Melting range:** 157-159 °C (Lit.: 150.5-152.5 °C)^{[13]} 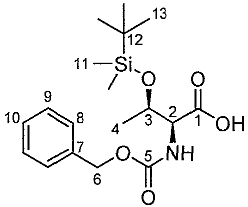
**HRMS** (ESI): Calculated for C₁₈H₂₉NNaO₅Si⁺ [M+Na]⁺: 390.1707, found: 390.1707.

### N-Benzyloxycarbonyl-N-methyl-O-tert-butyldimethylsilyl-I-threonine [M15]

According to **GP4,** 2.63 g (7.15 mmol, 1.0 eq.) of Cbz-O-TBS-I-Thr-OH **(M13),** 2.30 mL (36.8 mmol, 5.1 eq., *ρ* = 2.270 g/mL) methyl iodide and 1.44 g (60 wt% in mineral oil, 36.0 mmol, 5.0 eq.) NaH were reacted in 36 mL of anhydrous THF for 19 h. Contrary to **GP4,** the reaction mixture was acidified with 1 M HCl_{(aq.)} for workup and washed twice with sat. Na₂S₂O_{3(aq.)} solution. The residue obtained was purified by automated column chromatography (SiO₂, cHex/EtOAc/AcOH 100:0:0 → 50:50:1), whereby the isolated fraction was co-evaporated three times with toluene. A total of 2.20 g (5.78 mmol, 81 %) of *N*-methylated amino acid **M15** was isolated as a colorless, highly viscous oil.
${\left[α\right]}_{D}^{20} = + 13.4$ (c = 1.0, CHCl₃)
**R_{f}** = 0.07 (*n*-Pentane/EtOAc/AcOH 80:20:1) 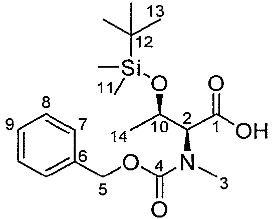
**HRMS** (Cl): Calculated for C₁₉H₃₂NO₅Si⁺ [M+H]⁺: 382.2044, found: 382.2059.

### N-Allyloxycarbonyl-I-valine [M14]^{[15]}

To a solution of 5.87 g (50.1 mmol, 1.0 eq.) I-Val-OH and 10.4 g (75.2 mmol, 1.5 eq.) K₂CO₃ in 110 mL water and 90 mL THF, a solution of 6.3 mL (59.1 mmol, 1.2 eq., *p* = 1.13 g/mL) allyl chloroformate in 20 mL THF was slowly added dropwise at 0 °C over a period of 15 min. After the addition was complete, the reaction was slowly warmed to RT and stirred for 3 d. The reaction mixture was concentrated in vacuo and extracted twice with Et₂O. The aqueous phase was acidified with 6 M HCl_{(aq.)} to a pH of 1, whereby a colorless solid precipitated. After the suspension was extracted three times with DCM, the combined DCM phases were dried over MgSO₄. Removal of the solvent in vacuo afforded 9.99 g (49.6 mmol, 99 %) of Alloc-I-Val-OH **(M14)** as a pale-yellow resin.
${\left[α\right]}_{D}^{20} = - 17.3$ (c = 1.0, CHCl₃); Lit.: ${\left[α\right]}_{D}^{20} = - 19.6$ (c = 0.7, CHCl₃)^{[16]} 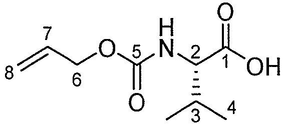
**HRMS** (ESI): Calculated for C₉H₁₆NO₄⁺ [M+H]⁺: 202.1074, found: 202.1073.

### N-Allyloxycarbonyl-N-methyl-I-valine [M16]

According to GP4, 4.82 g (24.0 mmol, 1.0 eq.) of Alloc-Val-OH (M14), 7.50 mL (120 mmol, 5.0 eq., p = 2.280 g/mL) methyl iodide and 2.88 g (60 wt% in mineral oil, 72.0 mmol, 3.0 eq.) NaH were reacted in 80 mL of anhydrous THF for 16.5 h. The crude product thus obtained was purified by automated column chromatography (SiO₂, cHex/EtOAc/AcOH 100:0:0 → 50:50:1) and the isolated fraction was co-evaporated three times with toluene. A total of 3.62 g (16.8 mmol, 70 %) of Alloc-*N*-Me-Val-OH **(M16)** was isolated as a yellow oil.
${\left[α\right]}_{D}^{20} = - 79.5$ (c = 1.0, CHCl₃) **R_{f}** = 0.16 (*n*-Pentane/EtOAc/AcOH 80:20:1) 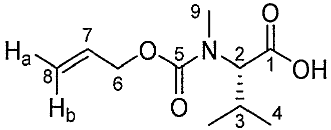
**HRMS** (CI): Calculated for C₁₀H₁₈NO₄⁺ [M+H]⁺: 216.1230, found: 216.1234.

### (2S,4R)-N-Benzyloxycarbonyl-4-methoxy-I-proline [M92]

According to **GP4,** 193 mg (728 µmol, 1.0 eq.) of Cbz-I-Hyp-OH was reacted with 0.23 mL (3.69 mmol, 5.1 eq., *ρ* = 2.280 g/mL) methyl iodide and 87.3 mg (60 wt% in mineral oil, 2.18 mmol, 3.0 eq.) NaH in 3.6 mL anhydrous THF for 23 h. Contrary to **GP4,** the aqueous work-up was carried out as follows: After adding H₂O to the reaction mixture, it was acidified with 1 M HCl_{(aq.)} to a pH of about 1. The aqueous phase was extracted three times with Et₂O and the combined etheric phases were washed once with sat. Na₂S₂O_{3(aq.)}- followed by sat. NaCl_{(aq.)} solution. It was then dried over MgSO₄, the solvent was removed in vacuo and the residue was purified by automated column chromatography (SiO₂, cHex/EtOAc/AcOH 100:0:0 → 50:50:1). The isolated fraction was co-evaporated three times with toluene. A total of 203 mg (727 µmol, 100 %) of the proline derivative **M92** was isolated as a colorless, viscous oil.
${\left[α\right]}_{D}^{20} = - 59.8$ (c = 1.0, CHCl₃)
**R_{f}** = 0.38 (*n*-Pentane/EtOAc/AcOH 50:50:1) 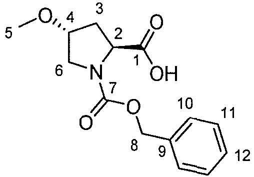
**HRMS** (ESI): Calculated for C₁₄H₁₈NO₅⁺ [M+H]⁺: 280.1179, found: 280.1185.

### (2S,4R)-N-Benzyloxycarbonyl-4-ethoxy-I-proline [M93]

According to **GP4,** 116 mg (436 µmol, 1.0 eq.) of Cbz-L-Hyp-OH were reacted with 0.17 mL (2.10 mmol, 4.8 eq., *ρ* = 1.930 g/mL) of ethyl iodide and 53.7 mg (60 wt% in mineral oil, 1.34 mmol, 3.0 eq.) of NaH in 1.5 mL of anhydrous THF for 24 h. Contrary to **GP4,** the aqueous work-up was carried out as follows: After adding H₂O to the reaction mixture, it was acidified with 1 M HCl_{(aq.)} to a pH of about 1. The aqueous phase was extracted three times with Et₂O, and the combined etheric phases were washed once with sat. Na₂S₂O_{3(aq.)}- followed by sat. NaCl_{(aq.)} solution. It was then dried over MgSO₄, the solvent was removed in vacuo and the residue was purified by automated column chromatography (SiO₂, cHex/EtOAc/AcOH 100:0:0 → 50:50:1; then C₁₈-SiO₂, H₂O/MeCN (+0.1 % HCOOH), 9:1 → 0:10). A total of 73.3 mg (250 µmol, 57 %) of the proline derivative **M93** was isolated as a colorless, viscous oil.
${\left[α\right]}_{D}^{20} = - 61.4$ (c = 1.0, CHCl₃)
**R_{f}** = 0.46 (*n*-Pentane/EtOAc/AcOH 50:50:1) 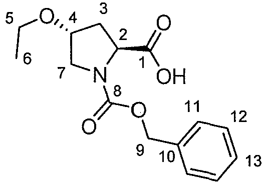
**HRMS** (ESI): Calculated for C₁₅H₂₀NO₅⁺ [M+H]⁺: 294.1336, found: 294.1332.

### N_{α}-tert-Butyloxycarbonyl-N_{ε}-azido-I-lysine [M53]^{[17]}

Triflylazide: A solution of 1.28 g (19.7 mmol, 1.6 eq.) NaN₃ in 3.1 mL H₂O was mixed with 3.1 mL toluene and cooled to 0 °C before 2.1 mL (12.3 mmol, 1.0 eq., p = 1.670 g/mL) Tf₂O was added. The two-phase mixture was first stirred vigorously for 30 min at 0 °C and then for 2 h at 10 °C. Sat. NaHCO_{3(aq.)} solution was then added until no more gas evolution was observed. The phases were then separated, and the reaction vessel was rinsed with 1.0 mL toluene when the mixture was transferred. Next, the aqueous phase was extracted twice with 2.7 mL toluene, whereby the combined organic phases correspond to an approx. 1.3 M solution of TfN₃.

Diazotransfer: To a solution of 412 mg (1.67 mmol, 1.0 eq.) Boc-I-Lys-OH in 2.0 mL water were added sequentially 561 mg (6.68 mmol, 4.0 eq.) NaHCO₃, 18.0 mg (72.1 µmol, 4 mol%) CuSO₄·5H₂O, 4.0 mL (1.3 M in toluene, 5.20 mmol, 3.1 eq.) TfN₃, and 15 mL MeOH. After the blue suspension was stirred vigorously for 17 h, the solvent was removed by rotary evaporation at RT(!). The resulting residue was purified by automated column chromatography (SiO₂, cHex/EtOAc/AcOH 100:0:0 → 50:50:1; then C₁₈-SiO₂, H₂O/MeCN, 9:1 → 0:10). A total of 361 mg (1.33 mmol, 79 %) of the lysine derivative **M53** was obtained as a colorless, highly viscous oil.
${\left[α\right]}_{D}^{20} = - 27.4$ (c = 0.5, CHCl₃); Lit.: ${\left[α\right]}_{D} = - 1.1$ (c = 1.0, MeOH)^{[18]}
**R_{f}** = 0.65 (*n*-Pentane/EtOAc/AcOH 50:50:1) 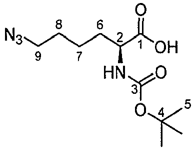
**HRMS** (ESI): Calculated for C₁₁H₂₁N₄O₄⁺ [M+H]⁺: 273.1557, found: 273.1552.

### Synthesis of the Peptide Fragments

### Synthesis of the Tetrapeptide Fragments

### Boc-I-Pro-N-Me-d-Leu-OMe [M5]^{[19]}

According to **GP5,** 4.71 g (18.2 mmol, 1.0 eq.) of Boc-*N*-Me-d-Leu-OMe **(M3)** was reacted with 12.7 mL (179 mmol, 10 eq., *ρ* = 1.104 g/mL) acetyl chloride in 45 mL MeOH. After 2 h, 3.61 g (18.2 mmol, 100 %) of *N*-Me-d-Leu-OMe·HCl was isolated as a colorless solid.

Under N₂ atmosphere, 3.09 g (14.4 mmol, 1.4 eq.) of Boc-I-Pro-OH was dissolved in 51 mL 1,4-dioxane and treated with 3.93 mL (22.5 mmol, 2.2 eq., *ρ* = 0.742 g/mL) DIPEA, followed by 152 µL (1.02 mmol, 0.1 eq., *ρ* = 0.910 g/mL) BnNMe₂. The solution was heated to 60 °C and treated dropwise with 14.3 mL (1.0 M in toluene, 14.3 mmol, 1.4 eq.) IPCF solution. After stirring at 60 °C for 15 min, a solution of 2.00 g (10.2 mmol, 1.0 eq.) *N*-Me-d-Leu-OMe·HCl, 163 µL (2.05 mmol, 0.2 eq., *ρ* = 1.030 g/mL) NMI, and 256 µL (4.0 M in 1,4-dioxane, 1.02 mmol, 0.1 eq.) HCl, dissolved in a mixture of 26 mL 1,4-dioxane and 42 mL MeCN, was added (rinsed twice with a total of 26 mL 1,4-dioxane). Stirring was continued at 60 °C for 17 h, and then the mixture was cooled to RT.

After concentration in vacuo, the residue was diluted with EtOAc. The organic phase was washed with 1 M KHSO_{4(aq.)}, water, sat. NaHCO_{3(aq.)} and sat. NaCl_{(aq.)} solution. Then it was dried over MgSO₄, the solvent was removed in vacuo, and the residue was purified by automated column chromatography (cHex/EtOAc 10:0 → 4:6). A total of 3.02 g (8.48 mmol, 84 %) of dipeptide **M5** was isolated as a colorless solid.
${\left[α\right]}_{D}^{20} = + 12.4$ (c = 1.0, CHCl₃)
**Melting range:** 119-120 °C
**R_{f}** = 0.30 (*n*-Pentane/EtOAc 1:1) 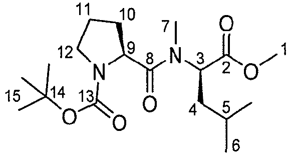
**HRMS** (CI): Calculated for C₁₈H₃₂N₂O₅⁺ [M]⁺: 356.2311, found: 356.2306.

### Boc-N-Me-I-Val-I-Pro-N-Me-d-Leu-OMe [M6]

According to **GP5,** 1.52 g (4.26 mmol, 1.0 eq.) of dipeptide **M5** was reacted with 2.30 mL (31.7 mmol, 7.5 eq., *p* = 1.104 g/mL) acetyl chloride in 11 mL MeOH for 1.5 h. Contrary to **GP5, M5** was added only after acetyl chloride. The resulting peptide hydrochloride was reacted according to **GP6** with 985 mg (4.26 mmol, 1.0 eq.) Boc-*N*-Me-I-Val-OH, 1.51 g (4.70 mmol, 1.1 eq.) TBTU, and 1.64 mL (9.39 mmol, 2.2 eq., *ρ* = 0.742 g/mL) DIPEA in 29 mL anhydrous MeCN for 17 h. Afterwards, the mixture was concentrated in vacuo, the residue was taken up in EtOAc, and the following workup was performed accordingly. Instead of 1 M KHSO_{4(aq.)}, 1 M HCl_{(aq.)} was used in the first washing step. Automated column chromatography (SiO₂, DCM/MeOH 100:0 → 97:3) of the crude product yielded 1.39 g (2.96 mmol, 70 %) of tripeptide **M6** as a colorless resin.
${\left[α\right]}_{D}^{20} = - 76.0$ (c = 1.0, CHCl₃)
**R_{f}** = 0.47 (DCM/MeOH 95:5) 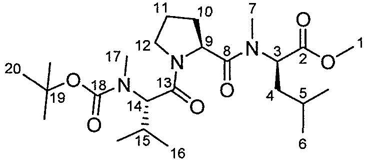
**HRMS** (Cl): Calculated for C₂₄H₄₄N₃O₆⁺ [M+H]⁺: 470.3225, found: 470.3214.

### Boc-I-HoLeu-N-Me-I-Val-I-Pro-N-Me-d-Leu-OMe [M7]

According to **GP5,** 99.9 mg (213 µmol, 1.0 eq.) of tripeptide **M6** was reacted with 151 µL (2.13 mmol, 10 eq., *p =* 1.104 g/mL) acetyl chloride in 0.71 mL MeOH for 3 h. The resulting peptide hydrochloride was reacted according to **GP6** with 70.0 mg (285 µmol, 1.3 eq.) Boc-I-HoLeu-OH, 29.5 mg (217 µmol, 1.0 eq.) HOAt, 162 mg (426 µmol, 2.0 eq.) HATU, and 148 µL (852 µmol, 4.0 eq., *ρ* = 0.742 g/mL) DIPEA in 2.1 mL anhydrous DMF for 16 h. Automated column chromatography (C₁₈-SiO₂, H₂O/MeCN 9:1 → 0:10) of the crude product yielded 109 mg (183 µmol, 86 %) of the tetrapeptide **M7** as a colorless, amorphous solid.
${\left[α\right]}_{D}^{20} = - 85.9$ (c = 1.0, CHCl₃)
**R_{f}** = 0.25 (*n*-Pentane/EtOAc 1:1) 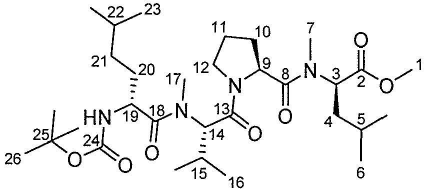
**HRMS** (CI): Calculated for C₃₁H₅₇N₄O₇⁺ [M]⁺: 597.4222, found: 597.4234.

### Boc-I-HoLeu-N-Me-I-Val-I-Pro-N-Me-d-Leu-OH [M48]

According to **GP3,** 102 mg (170 µmol, 1.0 eq.) of tetrapeptide **M7** was reacted with 188 µL (1.0 M, 188 µmol, 1.1 eq.) LiOH_{(aq.)} in 0.85 mL 1,4-dioxane for 3 h. The residue obtained after workup was lyophilized and used without further purification. A total of 94.4 mg (162 µmol, 95 %) of carboxylic acid **M48** was obtained as a colorless lyophilizate.
${\left[α\right]}_{D}^{20} = - 75.8$ (c = 1.0, CHCl₃) 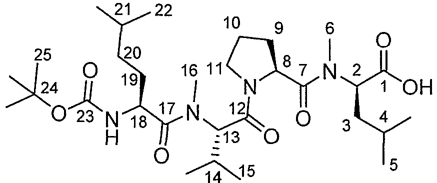
**HRMS** (ESI): Calculated for C₃₀H₅₅N₄O₇⁺ [M+H]⁺: 583.4065, found: 583.4074.

### Boc-I-CyPro-N-Me-d-Leu-OMe [M10]^{[19]}

According to **GP5,** 1.61 g (6.21 mmol, 1.0 eq.) of Boc-*N*-Me-d-Leu-OMe **(M3)** was reacted with 4.41 mL (62.1 mmol, 10 eq., *ρ* = 1.104 g/mL) acetyl chloride and 2.51 mL (62.1 mmol, 10 eq., *ρ* = 0.792 g/mL) MeOH in 16 mL 1,4-dioxane for 2.5 h.

Under N₂ atmosphere, 2.01 g (6.75 mmol, 1.1 eq.) of Boc-I-CyPro-OH was dissolved in 44 mL of 1,4-dioxane and treated with 2.38 mL (13.7 mmol, 2.2 eq., *ρ* = 0.742 g/mL) DIPEA, followed by 92.2 µL (621 µmol, 0.1 eq., *ρ* = 0.910 g/mL) BnNMe₂. The solution was heated to 60 °C and treated dropwise with 6.8 mL (1.0 M in toluene, 6.80 mmol, 1.1 eq.) IPCF solution. After stirring at 60 °C for 10 min, a solution of the previously obtained *N*-Me-d-Leu-OMe·HCl, 99.0 µL (1.24 mmol, 0.2 eq., *ρ* = 1.030 g/mL) NMI, and 160 µL (4.0 M in 1,4-dioxane, 640 µmol, 0.1 eq.) HCl, dissolved in 22 mL 1,4-dioxane and 37 mL MeCN, was added (rinsed with a total 44 mL of 1,4-dioxane). Stirring was continued at 60 °C for 17 h, and then the mixture was cooled to RT.

After concentration in vacuo, the residue was taken up in DCM. The organic phase was washed with 1 M KHSO_{4(aq.),} H₂O, sat. NaHCO_{3(aq.)} and sat. NaCl_{(aq.)} solution. Then it was dried over MgSO₄, the solvent was removed in vacuo, and the residue was purified by automated column chromatography (cHex/EtOAc 10:0 → 1:1). A total of 2.19 g (5.00 mmol, 81 %) of dipeptide **M10** was isolated as a colorless solid. ${\left[α\right]}_{D}^{20} = + 22.4$ = +22.4 (c = 1.0, CHCl₃)
**Melting point:** 149 °C
**R_{f}** = 0.59 (*n*-Pentane/EtOAc 1:1) 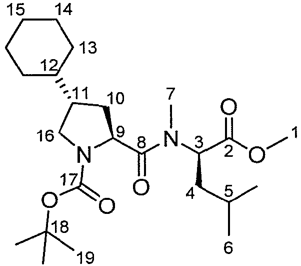
**HRMS** (Cl): Calculated for C₂₄H₄₃N₂O₅⁺ [M+H]⁺: 439.3166, found: 439.3173.

### Boc-N-Me-I-Val-I-CyPro-N-Me-d-Leu-OMe [M11]

According to **GP5,** 543 mg (1.24 mmol, 1.0 eq.) of dipeptide **M10** was treated with 3.0 mL (4.0 M in 1,4-dioxane, 12.0 mmol, 10 eq.) HCl for 3 h. The resulting peptide hydrochloride was reacted according to **GP6** with 328 mg (1.42 mmol, 1.1 eq.) of Boc-*N*-Me-I-Val-OH, 441 mg (1.38 mmol, 1.1 eq.) TBTU, and 544 µL (3.13 mmol, 2.5 eq., p = 0.742 g/mL) DIPEA in 4.2 mL anhydrous MeCN for 19 h. Before dilution with EtOAc for aqueous workup, the reaction mixture was concentrated in vacuo, and 1 M HCl_{(aq.)} was used instead of 1 M KHSO_{4(aq.)} in the first extraction step. Automated column chromatography (SiO₂, cHex/EtOAc 10:0 → 5:5) yielded 602 mg (1.09 mmol, 87 %) of tripeptide **M11** as a pale-yellow resin.
${\left[α\right]}_{D}^{20} = - 43.9$ (c = 1.0, CHCl₃)
**R_{f}** = 0.42 (n-Pentane/EtOAc 1:1) 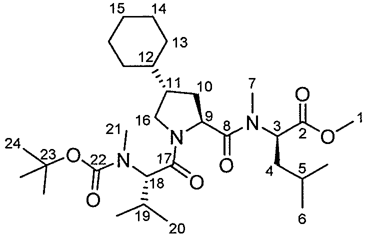
**HRMS** (ESI): Calculated for C₃₀H₅₄N₃O₆⁺ [M+H]⁺: 552.4007, found: 552.3996.

### Boc-I-HoLeu-N-Me-I-Val-I-CyPro-N-Me-d-Leu-OMe [M12]

According to **GP5,** 105 mg (176 µmol, 1.0 eq.) of tripeptide **M11** was treated with 1.0 mL (4.0 M in 1,4-dioxane, 4.00 mmol, 23 eq.) HCl for 1 h. The resulting peptide hydrochloride was reacted according to **GP6** with 55.5 mg (226 µmol, 1.3 eq.) Boc-I-HoLeu-OH, 24.7 mg (181 µmol, 1.0 eq.) HOAt, 133 mg (349 µmol, 2.0 eq.) HATU, and 122 µL (703 µmol, 4.0 eq., *p* = 0.742 g/mL) DIPEA in 1.8 mL anhydrous DMF for 17 h. Automated column chromatography (SiO₂, cHex/EtOAc 10:0 → 5:5) of the crude product, followed by lyophilization, yielded 104 mg (153 µmol, 87 %) of the tetrapeptide **M12** as a colorless lyophilizate.
${\left[α\right]}_{D}^{20} = - 45.6$ (c = 1.0, CHCl₃)
**R_{f}** = 0.50 (n-Pentane/EtOAc 1:1) 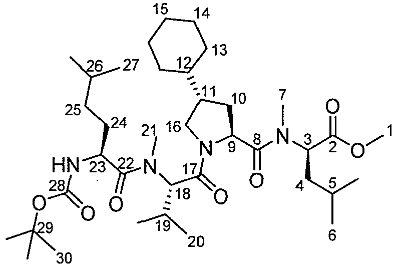
**HRMS** (ESI): Calculated for C₃₇H₆₇N₄O₇⁺ [M+H]⁺: 679.5004, found: 679.5003.

### Boc-l-HoLeu-N-Me-l-Val-l-CyPro-N-Me-d-Leu-OH [M49]

According to GP3, 594 mg (874 µmol, 1.0 eq.) of tetrapeptide **M12** and 1.09 mL (1.0 M, 1.09 mmol, 1.2 eq.) LiOH_{(aq.)} were reacted in 4.4 mL 1,4-dioxane for 3 h. After automated column chromatography (C₁₈-SiO₂, H₂O/MeCN 9:1 → 0:10) and lyophilization, 495 mg (745 µmol, 85 %) of the carboxylic acid **M49** were isolated as a colorless lyophilizate.
${\left[α\right]}_{D}^{20} = - 66.6$ (c = 0.5, CHCl₃) R_{f} = 0.46 (n-Pentane/EtOAc/AcOH 50:50:1) 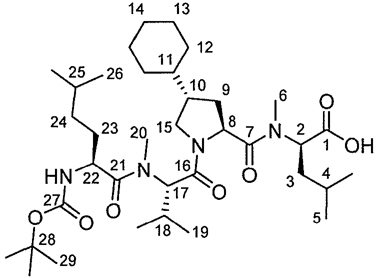
**HRMS** (ESI): Calculated for C₃₆H₆₅N₄O₇⁺ [M+H]⁺: 665.4848, found: 665.4828.

### Boc-I-Leu-N-Me-I-Val-I-CyPro-N-Me-d-Leu-OMe [M64]

According to **GP5,** 234 mg (424 µmol, 1.0 eq.) of tripeptide **M11** was treated with 1.1 mL (4.0 M in 1,4-dioxane, 4.00 mmol, 10 eq.) HCl for 3.5 h. The resulting peptide hydrochloride was reacted according to **GP6** with 140 mg (560 µmol, 1.3 eq.) Boc-I-Leu-OH, 210 mg (552 µmol, 1.3 eq.) HATU, and 185 µL (1.06 mmol, 2.5 eq., p = 0.742 g/mL) DIPEA for 16.5 h. Automated column chromatography (C₁₈-SiO₂, H₂O/MeCN 9:1 → 0:10; then SiO₂, cHex/EtOAc 10:0 → 5:5) of the crude product, followed by lyophilization, yielded 201 mg (302 µmol, 71 %) of the tetrapeptide **M64** as a colorless lyophilizate.
${\left[α\right]}_{D}^{20} = - 69.6$ (c = 0.5, CHCl₃) 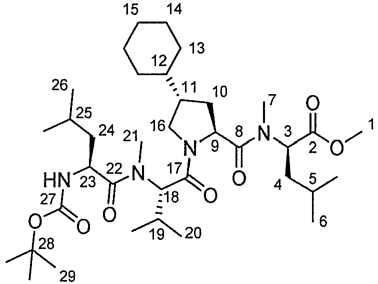
**HRMS** (ESI): Calculated for C₃₆H₆₄N₄NaO₇⁺ [M+Na]⁺: 687.4667, found: 687.4673.

### Boc-I-Leu-N-Me-I-Val-I-CyPro-N-Me-d-Leu-OH [M65]

According to GP3, 176 mg (264 µmol, 1.0 eq.) of tetrapeptide **M64** was reacted with 290 µL (1.0 M, 290 µmol, 1.1 eq.) LiOH_{(aq.)} in 0.9 mL 1,4-dioxane. After 5 h, additional 26.4 µL (1.0 M, 26.4 µmol, 0.1 eq.) of LiOH_{(aq.)} was added. The reaction mixture was then worked up after 1 h and the crude product was purified by automated column chromatography (C₁₈-SiO₂, H₂O/MeCN 9:1 → 0:10). After lyophilization, a total of 153 mg (235 µmol, 89%) of carboxylic acid **M65** was isolated as a colorless lyophilizate.
${\left[α\right]}_{D}^{20} = - 65.4$ (c = 0.5, CHCl₃) 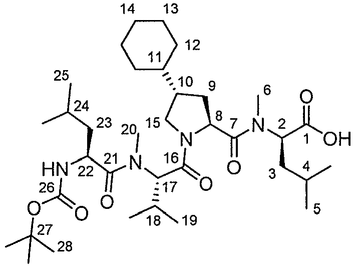
**HRMS** (ESI): Calculated for C₃₅H₆₃N₄O₇⁺ [M+H]⁺: 651.4691, found: 651.4676.

### Boc-I-MePro-N-Me-d-Leu-OMe [M66]^{[19]}

According to **GP5,** 4.71 g (862 µmol, 1.0 eq.) of Boc-*N*-Me-d-Leu-OMe **(M3)** was reacted with 45 mL MeOH and 12.7 mL (179 mmol, 10 eq., *ρ* = 1.104 g/mL) acetyl chloride. After a reaction time of 2 h, 3.61 g (18.2 mmol, 100 %) of *N*-Me-d-Leu-OMe·HCl was isolated as a colorless solid.

Under N₂ atmosphere, 227 mg (862 µmol, 1.1 eq.) of Cbz-I-MePro-OH **(P54)** was dissolved in 6 mL 1,4-dioxane and treated with 294 µL (1.69 mmol, 2.2 eq., ρ = 0.742 g/mL) DIPEA, followed by 11.4 µL (76.7 µmol, 0.1 eq., *ρ* = 0.910 g/mL) BnNMe₂. The solution was heated to 60 °C and treated dropwise with 0.86 mL (1.0 M in toluene, 860 µmol, 1.1 eq.) IPCF solution. After stirring at 60 °C for 10 min, a solution consisting of 152 mg (778 µmol, 1.0 eq.) of the previously obtained *N*-Me-d-Leu-OMₑ·HCl, 12.4 µL (156 µmol, 0.2 eq., *ρ* = 1.030 g/mL) NMI, and 19.4 µL (4.0 M in 1,4-dioxane, 77.8 µmol, 0.1 eq.) HCl, dissolved in 3 mL 1,4-dioxane and 5 mL MeCN, was added (rinsed twice with a total of 6 mL of 1,4-dioxane). Stirring was continued at 60 °C for 14 h, and then the mixture was cooled to RT. After concentration in vacuo, the residue was taken up in EtOAc. The organic phase was washed with 1 M KHSO_{4(aq.),} water, sat. NaHCO_{3(aq.)} and sat. NaCk_{(aq.)} solution. Then it was dried over MgSO₄, the solvent was removed in vacuo, and the residue was purified by automated column chromatography (C₁₈-SiO₂, H₂O/MeCN 9:1 → 0:10). After lyophilization, a total of 247 mg (611 µmol, 79%) of dipeptide **M66** was isolated as a colorless lyophilizate.
${\left[α\right]}_{D}^{20} = + 32.0$ (c = 0.5, CHCl₃) 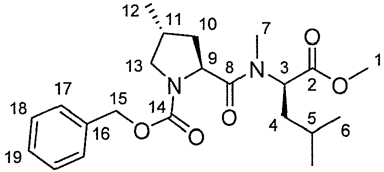
**HRMS** (ESI): Calculated for C₂₂H₃₂N₂NaO₅⁺ [M+Na]⁺: 427.2203, found: 427.2203.

### Boc-N-Me-I-Val-I-MePro-N-Me-d-Leu-OMe [M67]

According to **GP2,** 224 mg (555 µmol, 1.0 eq.) of dipeptide **M66** was reacted with 22.3 mg (10 wt%) Pd/C for 3.5 h. After addition of Pd/C, 0.14 mL (4.0 M in 1,4-dioxane, 560 µmol, 1.0 eq.) HCl was added before stirring under H₂ atmosphere. The resulting peptide hydrochloride was reacted according to **GP6** with 157 mg (678 µmol, 1.2 eq.) of Boc-*N-*Me-I-Val-OH, 214 mg (668 µmol, 1.2 eq.) TBTU, and 251 µL (1.44 mmol, 2.6 eq., *p* = 0.742 g/mL) DIPEA in 2.8 mL anhydrous MeCN for 16 h. Before dilution with EtOAc for aqueous workup, the reaction mixture was concentrated in vacuo. Automated column chromatography (SiO₂, cHex/EtOAc 10:0 → 5:5) of the crude product yielded 219 mg (453 µmol, 82 %) of tripeptide **M67** as a colorless resin.
${\left[α\right]}_{D}^{20} = - 74.3$ (c = 0.5, CHCl₃) 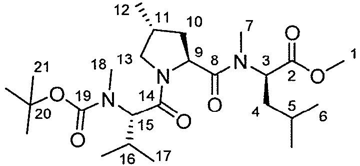
**HRMS** (ESI): Calculated for C₂₅H₄₆N₃O₆⁺ [M+H]⁺: 484.3381, found: 484.3395.

### Boc-I-HoLeu-N-Me-I-Val-I-MePro-N-Me-d-Leu-OMe [M72]

According to **GP5,** 215 mg (445 µmol, 1.0 eq.) of tripeptide **M67** was reacted with 317 µL (4.45 mmol, 10 eq., *ρ* = 1.104 g/mL) acetyl chloride in 2.0 mL MeOH for 6 h. The resulting peptide hydrochloride was reacted according to **GP6** with 146 mg (595 µmol, 1.3 eq.) Boc-I-HoLeu-OH, 220 mg (577 µmol, 1.3 eq.) HATU, and 194 µL (1.11 mmol, 2.5 eq., *p* = 0.742 g/mL) DIPEA in 2.2 mL anhydrous DMF for 16.5 h. Automated column chromatography (SiO₂, cHex/EtOAc 10:0 → 5:5) of the crude product, followed by lyophilization, yielded 216 mg (353 µmol, 79 %) of tetrapeptide **M72** as a pale-yellow lyophilizate.
${\left[α\right]}_{D}^{20} = - 88.4$ (c = 0.5, CHCl₃) 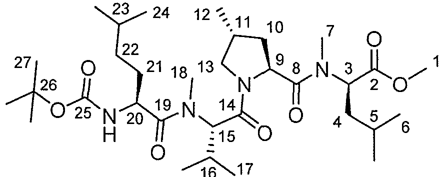
**HRMS** (ESI): Calculated for C₃₂H₅₈N₄NaO₇⁺ [M+Na]⁺: 633.4198, found: 633.4202.

### Boc-I-HoLeu-N-Me-I-Val-I-MePro-N-Me-d-Leu-OH [M73]

According to **GP3,** 211 mg (345 µmol, 1.0 eq.) of tetrapeptide **M72** was treated with 422 µL (1.0 M, 422 µmol, 1.2 eq.) LiOH_{(aq.)} in 2.3 mL 1,4-dioxane. After 5 h, an additional 86.2 µL (1.0 M, 86.2 µmol, 0.25 eq.) of LiOH_{(aq.)} was added. The reaction mixture was then worked up after 1 h and the crude product was purified by automated column chromatography (C₁₈-SiO₂, H₂O/MeCN 9:1 → 0:10). After lyophilization, a total of 194 mg (325 µmol, 94 %) of carboxylic acid **M73** was isolated as a colorless lyophilizate.
${\left[α\right]}_{D}^{20} = - 72.8$ (c = 0.5, CHCl₃) 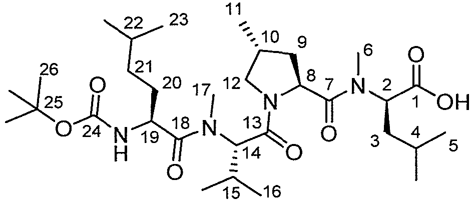
**HRMS** (ESI): Calculated for C₃₁H₅₇N₄O₇⁺ [M+H]⁺: 597.4222, found: 597.4234.

### Boc-1-Lys(N₃)-I-CyPro-d-Leu-OMe [M54]

According to **GP5,** 205 mg (466 µmol, 1.0 eq.) of dipeptide **M10** was treated with 1.2 mL (4.0 M in 1,4-dioxane, 4.80 mmol, 10 eq.) of HCl for 2 h. Subsequently, the resulting peptide hydrochloride was reacted according to **GP6** with 156 mg (574 µmol, 1.2 eq.) Boc-I-Lys(N₃)-OH **(M53),** 188 mg (585 µmol, 1.2 eq.) TBTU, and 203 µL (1.17 mmol, 2.5 eq., *ρ* = 0.742 g/mL) DIPEA in 1.6 mL anhydrous DMF for 22 h. After automated column chromatography (C₁₈-SiO₂, H₂O/MeCN 9:1 → 0:10) and lyophilization, 242 mg (408 µmol, 88 %) of tripeptide **M54** was isolated as a colorless lyophilizate. ${\left[α\right]}_{D}^{20} = - 28.8$ = -28.8 (c = 0.5, CHCl₃) 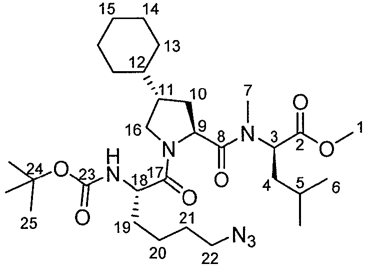 **HRMS** (ESI): Calculated for C₃₀H₅₂N₆NaO₆⁺ [M+Na]⁺: 615.3841, found: 615.3836.

### Boc-N_{α}-Me-I-Lys(N₃)-I-CyPro-d-Leu-OMe [M55]

In a round-bottomed flask wrapped with aluminium foil, under an Ar atmosphere, 223 mg (377 µmol, 1.0 eq.) of tripeptide **M54** was dissolved in 1.3 mL anhydrous DMF and treated with 350 mg (1.51 mmol, 4.0 eq.) Ag₂O, followed by 0.38 mL (6.08 mmol, 16 eq., *p* = 2.270 g/mL) methyl iodide. After 3 d, the black suspension was filtered through Celite and rinsed with EtOAc. The filtrate was then washed three times with H₂O, dried over MgSO₄, and concentrated in vacuo. Finally, the residue was purified by automated column chromatography (cHex/EtOAc 10:0 → 5:5). After lyophilization, 222 mg (366 µmol, 97 %) of the N-methylated tripeptide **M55** was isolated as a colorless lyophilizate.
${\left[α\right]}_{D}^{20} = - 39.2$ (c = 0.5, CHCl₃) 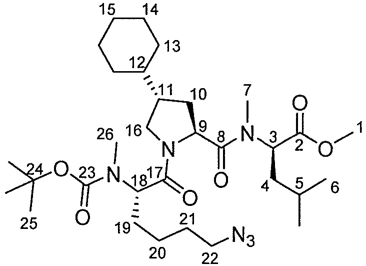
**HRMS** (ESI): Calculated for C₃₁H₅₅N₆O₆⁺ [M+H]⁺: 607.4178, found: 607.4169.

### Boc-I-HoLeu-N_{α}-Me-I-Lys(N₃)-I-CyPro-d-Leu-OMe [M56]

According to **GP5,** 176 mg (290 µmol, 1.0 eq.) of tripeptide **M55** was reacted with 0.73 mL (4.0 M in 1,4-dioxane, 2.90 mmol, 10 eq.) HCl for 2 h. Subsequently, the resulting peptide hydrochloride was reacted according to GP6 with 85.3 mg (348 µmol, 1.2 eq.) Boc-I-HoLeu-OH, 135 mg (354 µmol, 1.2 eq.) HATU, and 131 µL (753 µmol, 2.6 eq., *p* = 0.742 g/mL) DIPEA in 2.0 mL of anhydrous DMF for 17 h. After automated column chromatography (SiO₂, cHex/EtOAc 10:0 → 4:6), 190 mg (258 µmol, 89 %) of tetrapeptide **M56** was isolated as a colorless resin.
${\left[α\right]}_{D}^{20} = - 33.6$ (c = 0.5, CHCl₃) 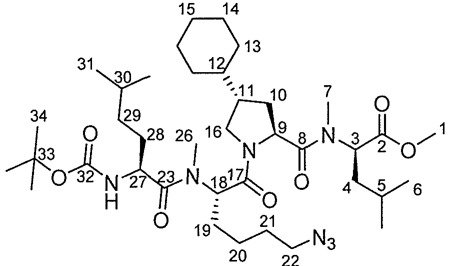
**HRMS** (ESI): Calculated for C₃₈H₆₇N₇O₇⁺ [M+H]⁺: 734.5175, found: 734.5202.

### Boc-I-HoLeu-N_{α}-Me-I-Lys(N₃)-I-CyPro-d-Leu-OH [M116]

According to **GP3,** 185 mg (252 µmol, 1.0 eq.) of the methyl ester **M56** was reacted with 315 µL (1.0 M, 315 µmol, 1.25 eq.) LiOH(aq.) in 2.4 mL 1,4-dioxane for 18.5 h. Automated column chromatography (C₁₈-SiO₂, H₂O/MeCN 9:1 → 0:10) and lyophilization yielded 153 mg (213 µmol, 84 %) of the carboxylic acid **M116** as a colorless lyophilizate.
${\left[α\right]}_{D}^{20} = - 28.8$ (c = 0.5, CHCl₃) 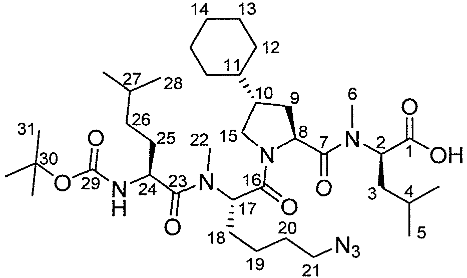
**HRMS** (ESI): Calculated for C₃₇H₆₅N₇NaO₇⁺ [M+Na]⁺: 742.4838, found: 742.4836.

### Synthesis of the Hexapeptide Fragments

### Cbz-I-Leu-I-MePro-Ot-Bu [M35]

According to GP2, 1.01 g (3.17 mmol, 1.0 eq.) of Cbz-I-MePro-O*t*-Bu (P31) was hydrogenated with 100 mg (10 wt%) Pd/C in 13 mL MeOH for 3 h. The resulting free amine was coupled according to **GP6** with 1.16 g (4.35 mmol, 1.4 eq.) Cbz-I-Leu-OH, 682 mg (4.45 mmol, 1.4 eq.) HOBt, 850 mg (4.44 mmol, 1.4 eq.) EDC, and 871 µL (7.92 mmol, 2.5 eq., *ρ* = 0.920 g/mL) NMM for 14.5 h. Automated column chromatography (SiO₂, cHex/EtOAc 10:0 → 7:3) of the crude product yielded 1.24 g (2.87 mmol, 90 %) of dipeptide **M35** as a colorless resin.
${\left[α\right]}_{D}^{20} = - 48.7$ (c = 1.0, CHCl₃)
**R_{f}** = 0.57 (n-Pentane/EtOAc 1:1) 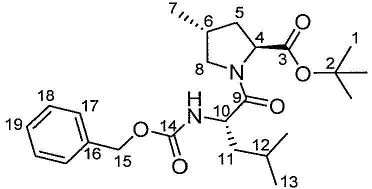
**HRMS** (ESI): Calculated for C₂₄H₃₇N₂O₅⁺ [M+H]⁺: 433.2700, found: 433.2697.

### Cbz-O-TBS-N-Me-I-Thr-I-Leu-I-MePro-Ot-Bu [M37]

According to **GP2,** 1.12 g (2.58 mmol, 1.0 Äq.) of the dipeptide **M35** was hydrogenated with 117 mg (10 wt%) Pd/C in 9.2 mL MeOH for 3 h. After addition of Pd/C, 650 µL (4.0 M in 1,4-dioxane, 2.60 mmol, 1.0 eq.) of HCl was added before stirring under H₂ atmosphere.

Then, under an Ar atmosphere, 988 mg (2.59 mmol, 1.0 eq.) of Cbz-O-TBS-*N*-Me-I-Thr-OH **(M15)** was dissolved in 14 mL of anhydrous THF and cooled to -20 °C before 625 µL (5.68 mmol, 2.2 eq., *p* = 0.920 g/mL) NMM was added. Subsequently, 340 µL (2.59 mmol, 1.0 eq., *p =* 1.040 g/mL) IBCF was added dropwise, and the resulting colorless suspension was stirred for 15 min at -20 °C. A solution of the previously obtained free amine in 8 mL DCM was then added dropwise, followed by two rinses with 1.5 mL of DCM each, and the mixture was slowly warmed to RT.

After 1.5 d, the reaction mixture was concentrated in vacuo and the residue was taken up in EtOAc before washing with 1 M KHSO_{4(aq.)}, H₂O, sat. NaHCO_{3(aq.)} and sat. NaCk_{(aq.)} solution. The organic phase was then dried over MgSO₄ and concentrated in vacuo. Automated column chromatography of the residue (SiO₂, cHex/EtOAc 100:0 → 65:35) yielded 1.58 g (2.39 mmol, 92 %) of the tripeptide **M37** as a colorless foam.
${\left[α\right]}_{D}^{20} = - 42.7$ (c = 1.0, CHCl₃)
**R_{f}** = 0.57 (n-Pentane/EtOAc 1:1) 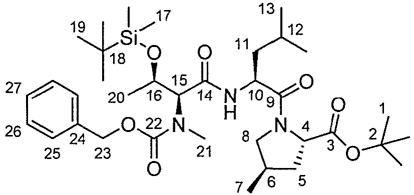
**HRMS** (CI): Calculated for C₃₁ H₅₀N₃O₇Si⁺ [M-C₄H₉]⁺: 604.3413, found: 604.3438.

### Cbz-N-Me-I-Thr-1-Leu-I-MePro-Ot-Bu [M43]^{[10]}

To a solution of 100 mg (152 µmol, 1.0 eq.) of tripeptide **M37** in 1.5 mL of anhydrous MeOH, 4.80 µL (67.5 µmol, 0.45 eq., *ρ* = 1.104 g/mL) acetyl chloride was added under an Ar atmosphere at 0 °C. The colorless solution was slowly warmed to RT and after 17 h diluted with EtOAc. Subsequently, it was washed with saturated NaHCO_{3(aq.)} solution and water before the organic phase was dried over MgSO₄ and concentrated in vacuo. After automated column chromatography (C₁₈-SiO₂, H₂O/MeCN 9:1 → 0:10) and subsequent lyophilization of the residue, 76.0 mg (139 µmol, 92 %) of the alcohol **M43** was isolated as a colorless lyophilizate.
${\left[α\right]}_{D}^{20} = - 108.4$ (c = 0.5, CHCl₃)
**R_{f}** = 0.12 (n-Pentane/EtOAc 1:1) 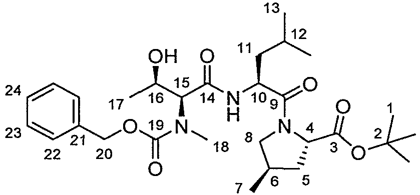
**HRMS** (ESI): Calculated for C₂₉H₄₆N₃O₇⁺ [M+H]⁺: 548.3330, found: 548.3308.

### Cbz-O-(EtPro-Cbz)-N-Me-I-Thr-I-Leu-I-MePro-Ot-Bu [M46]

According to **GP7,** 127 mg (231 µmol, 1.0 eq.) of the tripeptide **M43,** 88.4 mg (319 µmol, 1.4 eq.) Cbz-I-EtPro-OH **(P36),** 43.6 mg (294 µmol, 1.3 eq.) PPY, and 112 mg (326 µmol, 1.4 eq) MNBA were reacted in 1.2 mL anhydrous THF for 15.5 h. After automated column chromatography (SiO₂, cHex/EtOAc 10:0 → 4:6) and lyophilization, 179 mg (222 µmol, 96 %) of the depsipeptide **M46** was isolated as a colorless lyophilizate.
${\left[α\right]}_{D}^{20} = - 79.4$ (c = 0.5, CHCl₃) 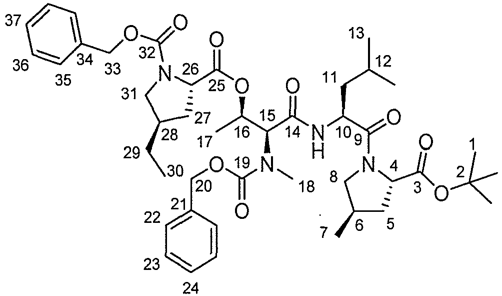
**HRMS** (ESI): Calculated for C₄₄H₆₃N₄O₁₀⁺ [M+H]⁺: 807.4539, found: 807.4513.

### Alloc-N-Me-I-Val-I-EtPro-N-Me-I-Thr-I-Leu-I-MePro-Ot-Bu [M40]

According to **GP2,** 1.38 g (1.71 mmol, 1.0 eq.) of the depsipeptide **M46** was hydrogenated with 132 mg (10 wt%) Pd/C in 10 mL MeOH for 6 h. The resulting amine was then coupled according to **GP6** with 917 mg (4.26 mmol, 2.5 eq.) Alloc-N-Me-I-Val-OH **(M16),** 2.22 g (4.26 mmol, 2.5 eq.) PyAOP, and 658 µL (5.98 mmol, 3.5 eq., *ρ* = 0.920 g/mL) NMM in 5.8 mL anhydrous DMF for 15 h. Automated column chromatography (SiO₂, cHex/EtOAc 1:0 → 0:1) yielded 1.10 g (1.50 mmol, 88 %) of the pentapeptide **M40** as a colorless foam.
${\left[α\right]}_{D}^{20} = - 136.6$ (c = 0.5, CHCl₃)
**R_{f}** = 0.04 (n-Pentane/EtOAc 1:1) 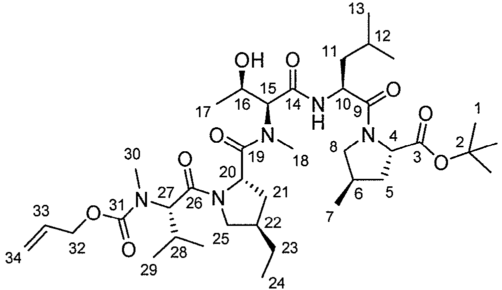
**HRMS** (Cl): Calculated for C₃₈H₆₆N₅O₉⁺ [M+H]⁺: 736.4855, found: 736.4831.

### Alloc-N-Me-I-Val-I-EtPro-O-(Gly-Fmoc)-N-Me-I-Thr-I-Leu-I-MePro-Ot-Bu [M41]

According to **GP7,** 95.2 mg (129 µmol, 1.0 eq.) of alcohol **M40,** 78.3 mg (263 µmol, 2.0 eq.) Fmoc-Gly-OH, 21.6 mg (145 µmol, 1.1 eq.) PPY, and 89.6 mg (260 µmol, 2.0 eq) of MNBA were reacted in 1.3 mL anhydrous THF for 15 h. Automated column chromatography (C₁₈-SiO₂; H₂O/MeCN 9:1 → 0:10) and lyophilization yielded 126 mg (124 µmol, 96 %) of the depsipeptide **M41** as a colorless lyophilizate.
${\left[α\right]}_{D}^{20} = - 114.2$ (c = 0.5, CHCl₃) **R_{f}** = 0.04 (n-Pentane/EtOAc 1:1) 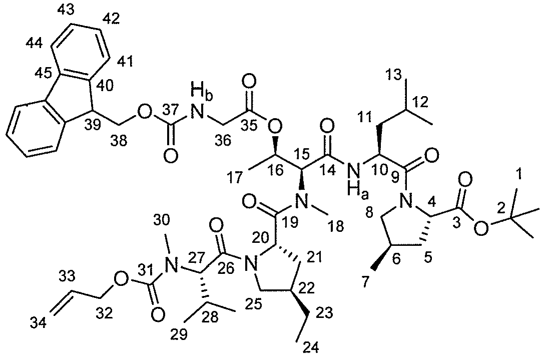
**HRMS** (ESI): Calculated for C₅₅H₇₉N₆O₁₂⁺ [M+H]⁺: 1015.5750, found: 1015.5733.

### Cbz-O-(MePro-Cbz)-N-Me-I-Thr-I-Leu-I-MePro-Ot-Bu [M68]

According to **GP7,** 187 mg (342 µmol, 1.0 eq) of tripeptide **M43,** 126 mg (479 µmol, 1.4 eq) Cbz-I-MePro-OH **(P54),** 58.4 mg (394 µmol, 1.2 eq) PPY, and 166 mg (483 µmol, 1.4 eq) MNBA were reacted for 20 h. After automated column chromatography (SiO₂, cHex/EtOAc 10:0 → 4:6) and lyophilization, 253 mg (319 µmol, 93 %) of depsipeptide **M68** was isolated as a colorless lyophilizate.
${\left[α\right]}_{D}^{20} = - 73.0$ (c = 0.5, CHCl₃) 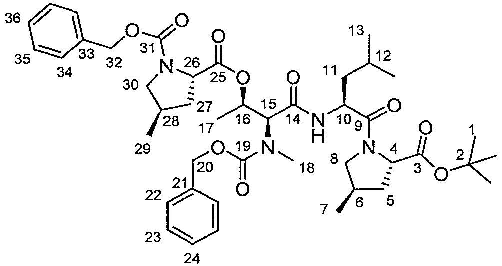
**HRMS** (ESI): Calculated for C₄₃H₆₁N₄O₁₀⁺ [M+H]⁺: 793.4382, found: 793.4407.

### Alloc-N-Me-I-Val-I-MePro-N-Me-I-Thr-I-Leu-I-MePro-Ot-Bu [M74]

According to **GP2,** 232 mg (292 µmol, 1.0 eq.) of depsipeptide **M68** was hydrogenated with 23.5 mg (10 wt%) Pd/C in 1.5 mL MeOH for 4 h. The resulting amine was then coupled according to **GP6** with 155 mg (721 µmol, 2.5 eq.) Alloc-*N*-Me-I-Val-OH **(M16),** 373 mg (716 µmol, 2.5 eq.) PyAOP, and 113 µL (1.02 mmol, 3.5 eq., *ρ* = 0.920 g/mL) NMM in 1.0 mL anhydrous DMF for 16 h. Automated column chromatography (C₁₈-SiO₂, H₂O/MeCN 1:9 → 0:10; then SiO₂, cHex/EtOAc 1:0 → 0:1) and subsequent lyophilization yielded 138 mg (190 µmol, 65 %) of pentapeptide **M74** as a colorless lyophilizate.
${\left[α\right]}_{D}^{20} = - 157.2$ (c = 0.5, CHCl₃) 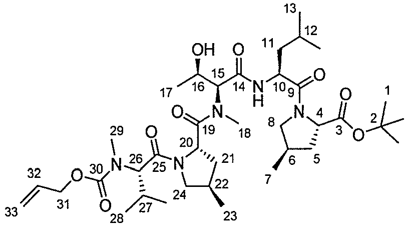
**HRMS** (ESI): Calculated for C₃₇H₆₄N₅O₉⁺ [M+H]⁺: 722.4699, found: 722.4704.

### Alloc-N-Me-I-Val-I-MePro-O-(Gly-Fmoc)-N-Me-I-Thr-I-Leu-I-MePro-Ot-Bu [M75]

According to **GP7,** 115 mg (159 µmol, 1.0 eq.) of alcohol **M74,** 95.6 mg (322 µmol, 2.0 eq.) Fmoc-Gly-OH, 27.1 mg (183 µmol, 1.2 eq.) PPY, and 110 mg (321 µmol, 2.0 eq.) MNBA were reacted in 1.6 mL anhydrous THF for 17 h. Automated column chromatography (C₁₈-SiO₂, H₂O/MeCN 9:1 → 0:10) and lyophilization yielded 155 mg (155 µmol, 97 %) of the depsipeptide **M75** as a colorless lyophilizate.
${\left[α\right]}_{D}^{20} = - 102.8$ (c = 0.5, CHCl₃) 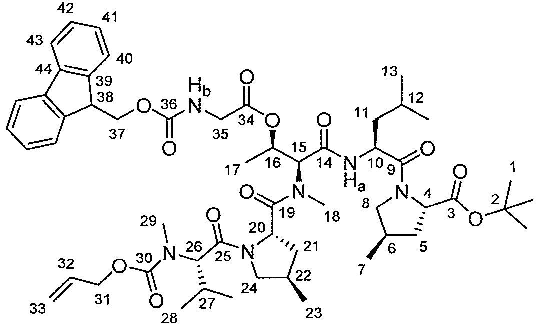
**HRMS** (ESI): Calculated for C₅₄H₇₇N₆O₁₂⁺ [M+H]⁺: 1001.5594, found: 1001.5608.

### Cbz-O-(MeOPro-Cbz)-N-Me-I-Thr-I-Leu-I-MePro-Ot-Bu [M94]

According to GP7, 160 mg (292 µmol, 1.0 eq.) of tripeptide **M43,** 116 mg (415 µmol, 1.4 eq.) Cbz-I-MeOPro-OH (M92), 60.3 mg (407 µmol, 1.4 eq.) PPY, and 145 mg (421 µmol, 1.4 eq.) MNBA were reacted in 1.9 mL anhydrous THF for 19 h. After automated column chromatography (C₁₈-SiO₂, H₂O/MeCN 9:1 → 0:10) and lyophilization, 217 mg (269 µmol, 92 %) of depsipeptide **M94** was isolated as a colorless lyophilizate.
${\left[α\right]}_{D}^{20} = - 98.2$ (c = 0.5, CHCl₃) 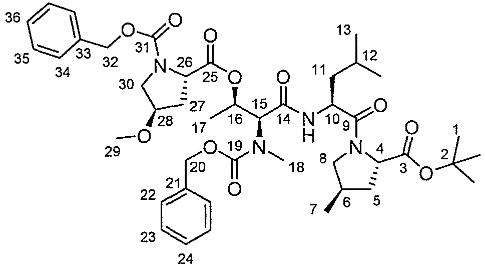
**HRMS** (ESI): Calculated for C₄₃H₆₁N₄O₁₁⁺ [M+H]⁺: 809.4331, found: 809.4338.

### Cbz-O-(Cbz-EtOPro)-N-Me-I-Thr-I-Leu-I-MePro-Ot-Bu [M95]

According to **GP7,** 171 mg (312 µmol, 1.0 eq.) of tripeptide **M43,** 125 mg (427 µmol, 1.4 eq.) Cbz-I-EtOPro-OH **(M93),** 60.3 mg (407 µmol, 1.4 eq.) PPY, and 145 mg (433 µmol, 1.4 eq) MNBA were reacted for 19 h in 1.4 mL anhydrous THF. After automated column chromatography (C₁₈-SiO₂, H₂O/MeCN 9:1 → 0:10) and lyophilization, 246 mg (299 µmol, 96 %) of depsipeptide **M95** was isolated as a colorless lyophilizate.
${\left[α\right]}_{D}^{20} = - 86.8$ (c = 0.5, CHCl₃) 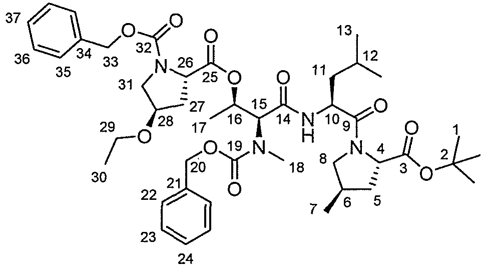
**HRMS** (ESI): Calculated for C₄₄H₆₂N₄NaO₁₁⁺ [M+Na]⁺: 845.4307, found: 845.4311.

### Alloc-N-Me-I-Val-I-MeOPro-N-Me-1-Thr-I-Leu-I-MePro-Ot-Bu [M96]

According to GP2, 201 mg (249 µmol, 1.0 eq.) of depsipeptide **M94** was hydrogenated with 22.2 mg (10 wt%) Pd/C in 2.5 mL MeOH for 5 h. The resulting amine was then coupled according to **GP6** with 135 mg (625 µmol, 2.5 eq.) Alloc-*N*-Me-I-Val-OH **(M16),** 324 mg (621 µmol, 2.5 eq.) PyAOP, and 95.8 µL (871 µmol, 3.5 eq., *ρ* = 0.920 g/mL) NMM in 1.2 mL anhydrous DMF for 17 h. Automated column chromatography (C₁₈-SiO₂, H₂O/MeCN 1:9 → 9:1) and subsequent lyophilization yielded 141 mg (190 µmol, 76 %) of pentapeptide **M96** as a colorless lyophilizate.
${\left[α\right]}_{D}^{20} = - 138.6$ (c = 0.5, CHCl₃) 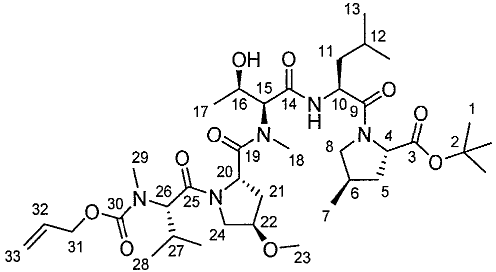
**HRMS** (ESI): Calculated for C₃₇H₆₃N₅NaO₁₀⁺ [M+Na]⁺: 760.4467, found: 760.4454.

### Alloc-N-Me-I-Val-I-EtOPro-N-Me-I-Thr-I-Leu-I-MePro-Ot-Bu [M97]

According to **GP2,** 229 mg (278 µmol, 1.0 eq.) of depsipeptide **M95** was hydrogenated with 23.2 mg (10 wt%) Pd/C in 2.8 mL MeOH for 5 h. The resulting amine was then coupled according to **GP6** with 148 mg (688 µmol, 2.5 eq.) Alloc-*N*-Me-I-Val-OH **(M16),** 360 mg (691 µmol, 2.5 eq.) PyAOP, and 107 µL (972 µmol, 3.5 eq., p = 0.920 g/mL) NMM in 1.4 mL anhydrous DMF for 20.5 h. Automated column chromatography (C₁₈-SiO₂, H₂O/MeCN 1:9 → 9:1) and subsequent lyophilization yielded 160 mg (213 µmol, 77 %) of pentapeptide **M96** as a colorless lyophilizate.
${\left[α\right]}_{D}^{20} = - 140.2$ (c = 0.5, CHCl₃) 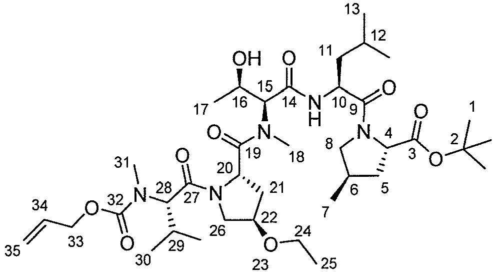
**HRMS** (ESI): Calculated for C₃₈H₆₆N₅O₁₀⁺ [M+H]⁺: 752.4804, found: 752.4806.

### Alloc-N-Me-I-Val-I-MeOPro-O-(Gly-Fmoc)-N-Me-I-Thr-I-Leu-I-MePro-Ot-Bu [M98]

According to **GP7,** 122 mg (165 µmol, 1.0 eq.) of alcohol **M96,** 99.2 mg (334 µmol, 2.0 eq.) Fmoc-Gly-OH, 32.2 mg (218 µmol, 1.3 eq.) PPY, and 114 mg (330 µmol, 2.0 eq.) MNBA were reacted in 1.6 mL anhydrous THF for 18 h. Automated column chromatography (C₁₈-SiO₂, H₂O/MeCN 9:1 → 0:10) and lyophilization yielded 155 mg (153 µmol, 92 %) of depsipeptide **M98** as a colorless lyophilizate.
${\left[α\right]}_{D}^{20} = - 100.8$ (c = 0.5, CHCl₃) 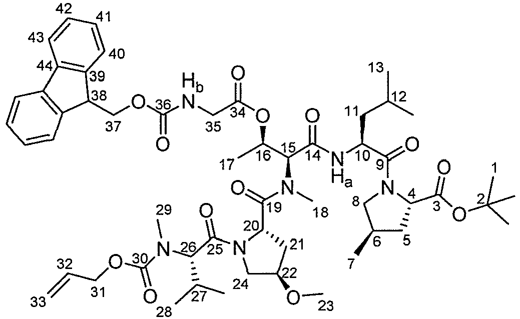
**HRMS** (ESI): Calculated for C₅₄H₇₇N₆O₁₃⁺ [M+H]⁺: 1017.5543, found: 1017.5547.

### Alloc-N-Me-I-Val-I-EtOPro-O-(Gly-Fmoc)-N-Me-I-Thr-I-Leu-I-MePro-Ot-Bu [M99]

According to **GP7,** 139 mg (184 µmol, 1.0 eq.) of alcohol **M97,** 109 mg (366 µmol, 2.0 eq.) Fmoc-Gly-OH, 36.8 mg (248 µmol, 1.4 eq.) PPY, and 127 mg (369 µmol, 2.0 eq.) MNBA were reacted in 1.8 mL anhydrous THF for 18 h. Automated column chromatography (C₁₈-SiO₂, H₂O/MeCN 9:1 → 0:10) and lyophilization yielded 157 mg (152 µmol, 83 %) of depsipeptide **M99** as a colorless lyophilizate.
${\left[α\right]}_{D}^{20} = - 107.8$ (c = 0.5, CHCl₃) 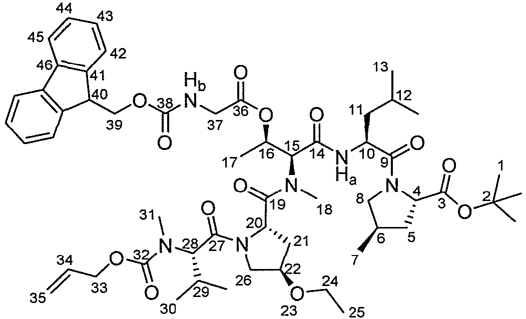
**HRMS** (ESI): Calculated for C₅₅H₇₉NₑO₁₃⁺ [M+H]⁺: 1031.5700, found: 1031.5707.

### Fragment Couplings and macrocyclizations

### Alloc-N-Me-I-Val-I-EtPro-O-(Gly-N-Me-d-Leu-I-Pro-N-Me-I-Val-I-HoLeu-Boc)-N-Me-I-Thr-I-Leu-I-MePro-Ot-Bu [M42]

According to **GP8,** 426 mg (420 µmol, 1.0 eq.) of hexapeptide **M41** was treated with 630 µL (4.20 mmol, 10 eq., *ρ* = 0.976 g/mL) tren in 4.2 mL DCM for 50 min. The resulting free amine was then coupled according to **GP6** with 294 mg (505 µmol, 1.2 eq.) of carboxylic acid **M48,** 218 mg (509 µmol, 1.2 eq.) COMU, and 115 µL (1.05 mmol, 2.5 eq., *p* = 0.920 g/mL) NMM in 4.2 mL anhydrous DMF for 25 h. After automated column chromatography (C₁₈-SiO₂, H₂O/MeCN 9:1 → 0:10), 461 mg (340 µmol, 81 %) of decapeptide **M42** was isolated as a colorless foam.
${\left[α\right]}_{D}^{20} = - 100.0$ (c = 0.5, CHCl₃) 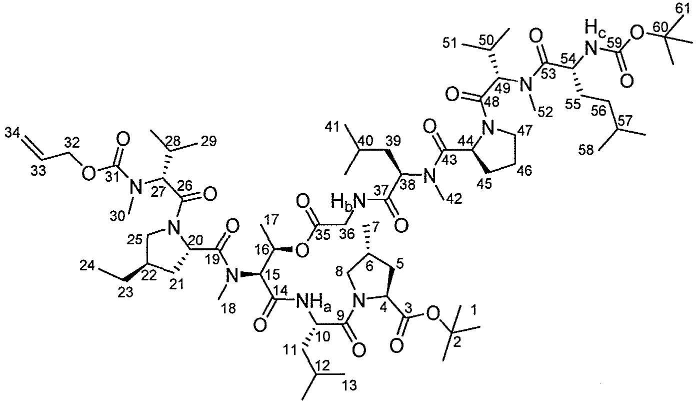
**HRMS** (ESI): Calculated for C₇₀H₁₂₀N₁₀NaO₁₀⁺ [M+Na]⁺: 1379.8776, found: 1379.8768.

### Alloc-N-Me-I-Val-I-EtPro-N-Me-cyclo-O-(Gly-N-Me-d-Leu-I-Pro-N-Me-I-Val-I-HoLeu-I-MePro-I-Leu)-I-Thr [M44]

According to **GP9,** 4.9 mg (3.61 µmol, 1.0 eq.) of decapeptide **M42** was treated with 0.5 mL of a 1:1 mixture of DCM/TFA for 3 h. Deviating from **GP9,** macrocyclization was performed with 19.6 mg (37.6 µmol, 10 eq.) PyAOP and 4.40 µL (40.0 µmol, 11 eq., *p* = 0.920 g/mL) NMM, instead of FDPP and DIPEA, in a total of 3.6 mL of anhydrous DMF (addition time = 2.5 h, stirring time = 15 h). After automated column chromatography (C₁₈-SiO₂, H₂O/MeCN 9:1 → 0:10) and lyophilization, 2.9 mg (2.45 µmol, 68 %) of the cyclic peptide **M44** was isolated as a colorless lyophilizate.
${\left[α\right]}_{D}^{20} = - 66.4$ (c = 0.5, CHCl₃) 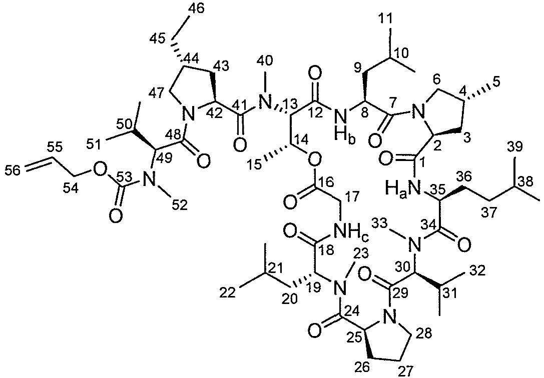
**HRMS** (ESI): Calculated for C₆₁H₁₀₂N₁₀NaO₁₃⁺ [M+Na]⁺: 1205.7520, found: 1205.7508.

### Alloc-N-Me-I-Val-I-EtPro-O-(Gly-N-Me-d-Leu-I-CyPro-N-Me-I-Val-I-HoLeu-Boc)-N-Me-I-Thr-I-Leu-I-MePro-Ot-Bu [M50]

According to **GP8,** 426 mg (420 µmol, 1.0 eq.) of the hexapeptide **M41** was treated with 630 µL (4.20 mmol, 10 eq., *ρ* = 0.976 g/mL) of tren in 4.2 mL of DCM for 1.5 h. The resulting free amine was then coupled according to **GP6** with 336 mg (505 µmol, 1.2 eq.) of carboxylic acid **M49,** 216 mg (505 µmol, 1.2 eq.) COMU, and 115 µL (1.05 mmol, 2.5 eq., *ρ* = 0.920 g/mL) NMM in 4.7 mL anhydrous DMF for 17.5 h. After automated column chromatography (C₁₈-SiO₂, H₂O/MeCN 9:1 → 0:10), 495 mg (343 µmol, 82 %) of decapeptide **M50** was isolated as a colorless resin.
${\left[α\right]}_{D}^{20} = - 96.2$ (c = 0.5, CHCl₃) 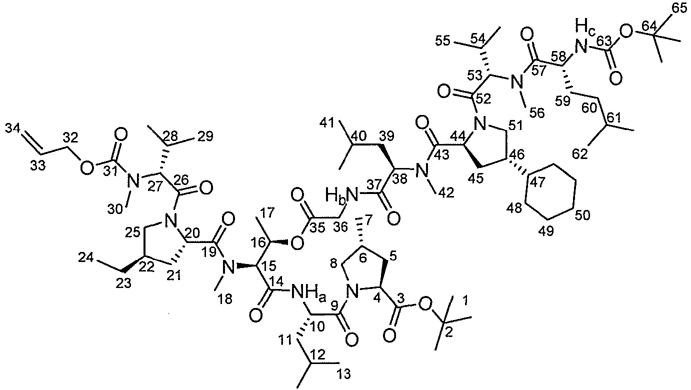
**HRMS** (ESI): Calculated for C₇₆H₁₃₀N₁₀NaO₁₆⁺ [M+Na]⁺: 1461.9558, found: 1461.9543.

### Alloc-N-Me-I-Val-I-EtPro-N-Me-cyclo-O-(Gly-N-Me-d-Leu-I-CyPro-N-Me-I-Val-I-HoLeu-I-MePro-I-Leu)-1-Thr [M51]

According to **GP9,** 55.8 mg (38.8 µmol, 1.0 eq.) of decapeptide **M50** was treated with 2.0 mL of a 1:1 mixture of DCM/TFA for 3.5 h. The resulting fully deprotected peptide was then treated with 77.0 mg (200 µmol, 5.1 eq.) FDPP and 74.3 µL (427 µmol, 11 eq., *p* = 0.742 g/mL) DIPEA in a total of 38.5 mL anhydrous DMF (addition time = 20 min, stirring time = 40 min). Automated column chromatography (C₁₈-SiO₂, H₂O/MeCN 9:1 → 0:10) of the crude product, followed by lyophilization, yielded 30.4 mg (24.0 µmol, 62 %) of the macrocycle **M51** as a colorless lyophilizate.
${\left[α\right]}_{D}^{20} = - 89.4$ (c = 0.5, CHCl₃) 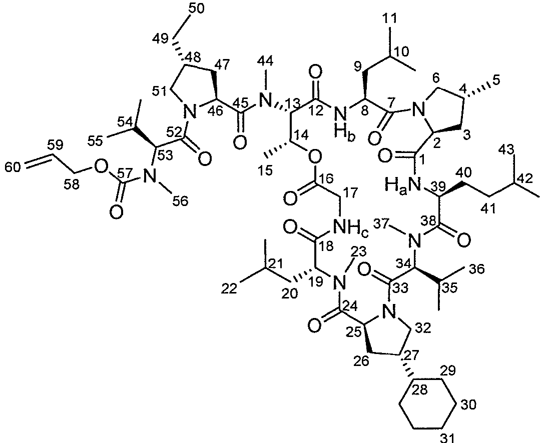
**HRMS** (ESI): Calculated for C₆₇H₁₁₂N₁₀O₁₃⁺ [M+H]⁺: 1265.8483, found: 1265.8475.

### Alloc-N-Me-I-Val-I-EtPro-O-(Gly-N-Me-d-Leu-I-CyPro-N-Me-I-Val-I-Leu-Boc)-N-Me-I-Thr-I-Leu-I-MePro-Ot-Bu [M70]

According to **GP8,** 52.8 mg (52.0 µmol, 1.0 eq.) of hexapeptide **M41** was treated with 78.0 µL (520 µmol, 10 eq., *ρ* = 0.976 g/mL) tren in 0.5 mL DCM for 3 h. The resulting free amine was then coupled according to **GP6** with 37.4 mg (57.5 µmol, 1.1 eq.) of carboxylic acid **M65,** 25.3 mg (59.1 µmol, 1.1 eq.) COMU, and 12.0 µL (109 µmol, 2.5 eq., *p* = 0.920 g/mL) NMM in 1 mL of a 1:1 mixture of anhydrous DCM and anhydrous DMF for 20 h. After automated column chromatography (C₁₈-SiO₂, H₂O/MeCN 9:1 → 0:10) and lyophilization, 59.4 mg (41.7 µmol, 80 %) of the decapeptide **M70** was isolated as a colorless lyophilizate.
${\left[α\right]}_{D}^{20} = - 84.6$ (c = 0.5, CHCl₃) 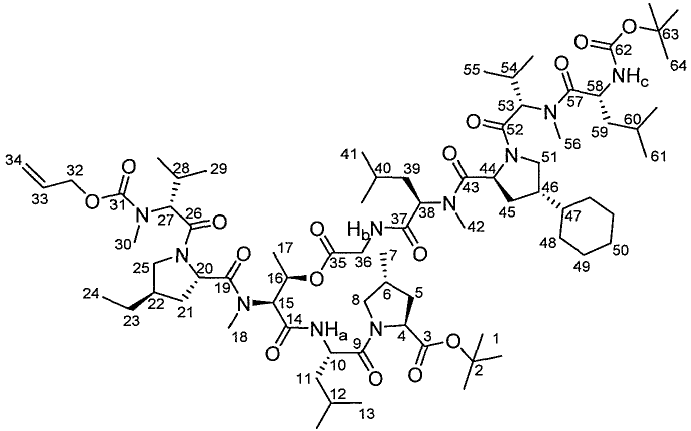
**HRMS** (ESI): Calculated for C₇₅H₁₂₈N₁₀NaO₁₆⁺ [M+Na]⁺: 1447.9402, found: 1447.9404.

### Alloc-N-Me-I-Val-I-EtPro-N-Me-cyclo-O-(Gly-N-Me-d-Leu-I-CyPro-N-Me-I-Val-I-Leu-I-MePro-I-Leu)-I-Thr [M76]

According to **GP9,** 35.4 mg (24.8 µmol, 1.0 eq.) of the decapeptide **M70** was treated with 2.0 mL of a 1:1 mixture of DCM/TFA for 2 h. The resulting fully deprotected peptide was then treated with 49.9 mg (130 µmol, 5.2 eq.) FDPP and 47.4 µL (272 µmol, 11 eq., *p* = 0.742 g/mL) DIPEA in a total of 25 mL anhydrous DMF (addition time = 60 min, stirring time = 30 min). Automated column chromatography (C₁₈-SiO₂, H₂O/MeCN 9:1 → 0:10) and purification by preparative HPLC (H₂O/MeCN 5:5 → 0:10) of the crude product, followed by lyophilization, yielded 14.9 mg (11.9 µmol, 48 %) of the macrocycle **M76** as a colorless lyophilizate. ${\left[α\right]}_{D}^{20} = - 62.2$ (c = 0.5, CHCl₃) 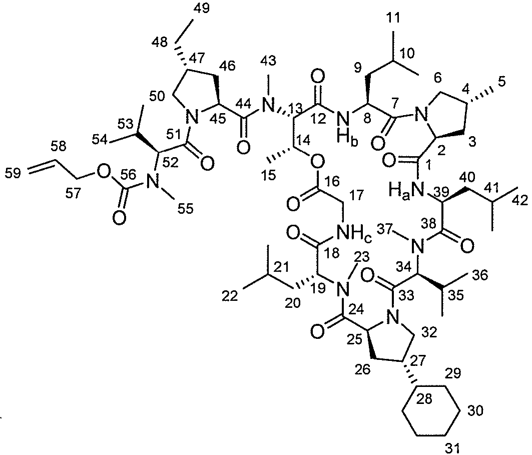 **HRMS** (ESI): Calculated for C₆₆H₁₁₀N₁₀O₁₃⁺ [M+H]⁺: 1251.8327, found: 1251.8329.

### Alloc-N-Me-I-Val-I-MePro-O-(Gly-N-Me-d-Leu-I-CyPro-N-Me-I-Val-I-HoLeu-Boc)-N-Me-I-Thr-I-Leu-I-MePro-Ot-Bu [M77]

According to **GP8,** 44.9 mg (44.8 µmol, 1.0 eq.) of hexapeptide **M75** was treated with 67.2 µL (448 µmol, 10 eq., *ρ* = 0.976 g/mL) of tren in 1.0 mL of DCM for 2.5 h. The resulting free amine was then coupled according to **GP6** with 42.3 mg (63.6 µmol, 1.4 eq.) of carboxylic acid **M49,** 27.5 mg (63.6 µmol, 1.4 eq.) COMU, and 12.8 µL (116 µmol, 2.6 eq., *ρ* = 0.920 g/mL) NMM in 1 mL of a 1:1 mixture of anhydrous DCM and anhydrous DMF for 20 h. After automated column chromatography (C₁₈-SiO₂, H₂O/MeCN 9:1 → 0:10) and lyophilization, 51.0 mg (35.8 µmol, 80 %) of decapeptide **M77** was isolated as a colorless lyophilizate.
${\left[α\right]}_{D}^{20} = - 88.6$ (c = 0.5, CHCl₃) 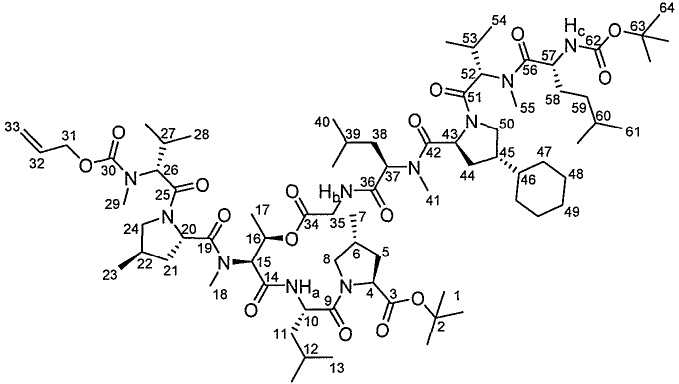
**HRMS** (ESI): Calculated for C₇₅H₁₂₈N₁₀NaO₁₆⁺ [M+Na]⁺: 1447.9402, found: 1447.9457.

### Alloc-N-Me-I-Val-I-MePro-N-Me-cyclo-O-(Gly-N-Me-d-Leu-I-CyPro-N-Me-I-Val-I-Leu-I-MePro-I-Leu)-I-Thr [M79]

According to **GP9,** 43.6 mg (30.6 µmol, 1.0 eq.) of decapeptide **M77** was treated with 2.0 mL of a 1:1 mixture of DCM/TFA for 2 h. The resulting fully deprotected peptide was then treated with 58.8 mg (153 µmol, 5.0 eq.) FDPP and 53.3 µL (306 µmol, 10 eq., *p* = 0.742 g/mL) DIPEA in a total of 30 mL anhydrous DMF (addition time = 60 min, stirring time = 60 min). Automated column chromatography (C₁₈-SiO₂, H₂O/MeCN 9:1 → 0:10) of the crude product, followed by preparative HPLC (H₂O/MeCN 5:5 → 0:10) and lyophilization, yielded 17.1 mg (13.7 µmol, 45 %) of the macrocycle **M79** as a colorless lyophilizate.
${\left[α\right]}_{D}^{20} = - 63.6$ (c = 0.5, CHCl₃) 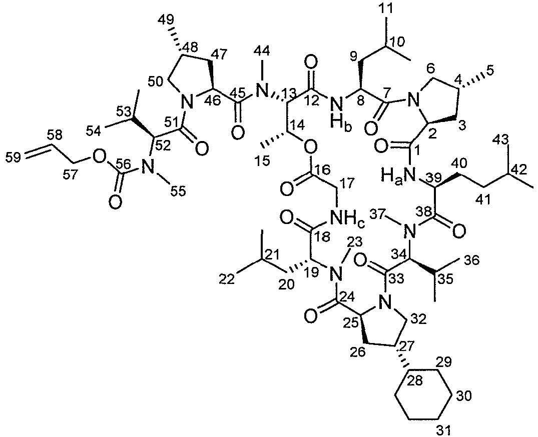
**HRMS** (ESI): Calculated for C₆₆H₁₁₁N₁₀O₁₃⁺ [M+H]⁺: 1251.8327, found: 1251.8334.

### Alloc-N-Me-I-Val-I-MePro-O-(Gly-N-Me-d-Leu-I-MePro-N-Me-I-Val-I-HoLeu-Boc)-N-Me-I-Thr-I-Leu-I-MePro-Ot-Bu [M78]

According to **GP8,** 47.6 mg (47.5 µmol, 1.0 eq.) of hexapeptide **M75** was treated with 71.2 µL (475 µmol, 10 eq., p = 0.976 g/mL) tren in 1.0 mL DCM for 3 h. The resulting free amine was then coupled according to **GP6** with 39.7 mg (66.5 µmol, 1.4 eq.) of carboxylic acid **M73,** 26.6 mg (62.1 µmol, 1.3 eq.) COMU, and 13.6 µL (124 µmol, 2.6 eq., *p* = 0.920 g/mL) NMM in 1.0 mL of a 1:1 mixture of anhydrous DCM and anhydrous DMF for 15.5 h. After automated column chromatography (C₁₈-SiO₂, H₂O/MeCN 9:1 → 0:10) and lyophilization, 50.9 mg (37.5 µmol, 79 %) of the decapeptide **M78** was isolated as a colorless lyophilizate.
${\left[α\right]}_{D}^{20} = - 99.2$ (c = 0.5, CHCl₃) 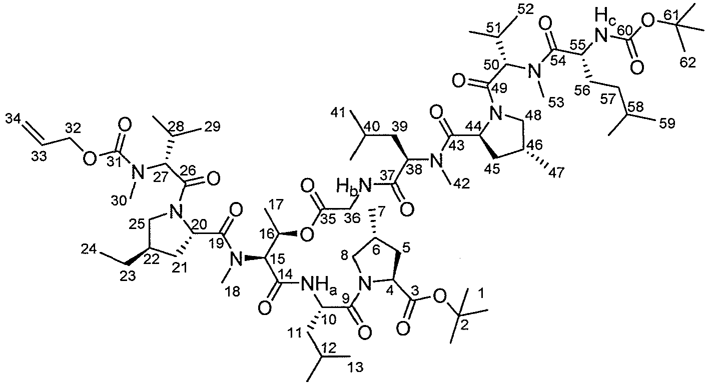
**HRMS** (ESI): Calculated for C₇₀H₁₂₁N₁₀O₁₆⁺ [M+H]⁺: 1357.8957, found: 1357.8957.

### Alloc-N-Me-I-Val-I-MePro-N-Me-cyclo-O-(Gly-N-Me-d-Leu-I-MePro-N-Me-I-Val-I-Leu-I-MePro-I-Leu)-I-Thr [M80]

According to **GP9,** 44.2 mg (32.6 µmol, 1.0 eq.) of decapeptide **M78** was treated with 2.0 mL of a 1:1 mixture of DCM/TFA for 2 h. The resulting fully deprotected peptide was then treated with 63.6 mg (166 µmol, 5.1 eq.) FDPP and 56.8 µL (326 µmol, 10 eq., *p* = 0.742 g/mL) DIPEA in a total of 32.5 mL anhydrous DMF (addition time = 60 min, stirring time = 30 min). Automated column chromatography (C₁₈-SiO₂, H₂O/MeCN 9:1 → 0:10) of the crude product, followed by lyophilization, yielded 17.2 mg (14.5 µmol, 45 %) of the macrocycle **M80** as a colorless lyophilizate.
${\left[α\right]}_{D}^{20} = - 66.8$ (c = 0.5, CHCl₃) 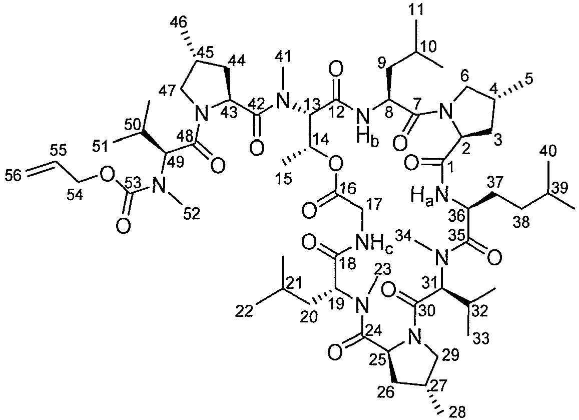
**HRMS** (ESI): Calculated for C₆₁H₁₀₂N₁₀O₁₃⁺ [M+H]⁺: 1183.7701, found: 1183.7690.

### Alloc-N-Me-I-Val-I-EtPro-O-(Gly-N-Me-d-Leu-I-CyPro-N_{α}-Me-I-Lys(N₃)-I-HoLeu-Boc)-N-Me-I-Thr-I-Leu-I-MePro-Ot-Bu [M63]

According to GP8, 103 mg (101 µmol, 1.0 eq.) of hexapeptide **M41** was treated with 151 µL (1.01 mmol, 10 eq., p = 0.976 g/mL) tren in 1.0 mL DCM for 1.5 h. The resulting free amine was then coupled according to **GP6** with 82.2 mg (114 µmol, 1.1 eq.) of carboxylic acid **M116,** 50.2 mg (117 µmol, 1.2 eq.) COMU, and 27.8 µL (253 µmol, 2.5 eq., p = 0.920 g/mL) NMM in 1.0 mL of anhydrous DCM for 18.5 h. After automated column chromatography (C₁₈-SiO₂, H₂O/MeCN 9:1 → 0:10) and lyophilization, 130 mg (87.2 µmol, 86 %) of the decapeptide **M63** was isolated as a colorless lyophilizate.
${\left[α\right]}_{D}^{20} = - 61.8$ (c = 0.5, CHCl₃) 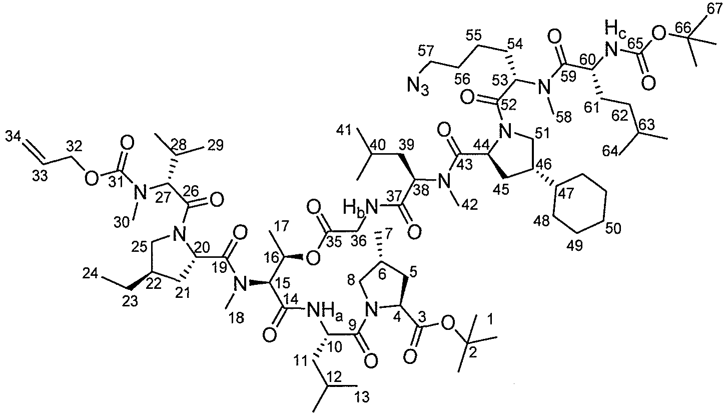
**HRMS** (ESI): Calculated for C₇₇H₁₃₁N₁₃NaO₁₆⁺ [M+Na]⁺: 1516.9729, found: 1516.9798.

### Alloc-N-Me-I-Val-I-EtPro-N-Me-cyclo-O-(Gly-N-Me-d-Leu-I-CyPro-N_{α}-Me-I-Lys(N₃)-I-HoLeu-I-MePro-I-Leu)-I-Thr [M69]

According to **GP9,** 38.9 mg (26.0 µmol, 1.0 eq.) of decapeptide **M63** was treated with 1.0 mL of a 1:1 mixture of DCM/TFA for 2 h. The resulting fully deprotected peptide was then treated with 102 mg (265 µmol, 10 eq.) FDPP and 95.2 µL (547 µmol, 21 eq., *p* = 0.742 g/mL) DIPEA in a total of 25 mL anhydrous DMF (addition time = 30 min, stirring time = 40 min). Automated column chromatography (C₁₈-SiO₂, H₂O/MeCN 9:1 → 0:10) of the crude product, followed by lyophilization, yielded 22.8 mg (17.3 µmol, 66%) of macrocycle **M69** as a colorless lyophilizate.
${\left[α\right]}_{D}^{20} = - 58.7$ (c = 0.3, CHCl₃) 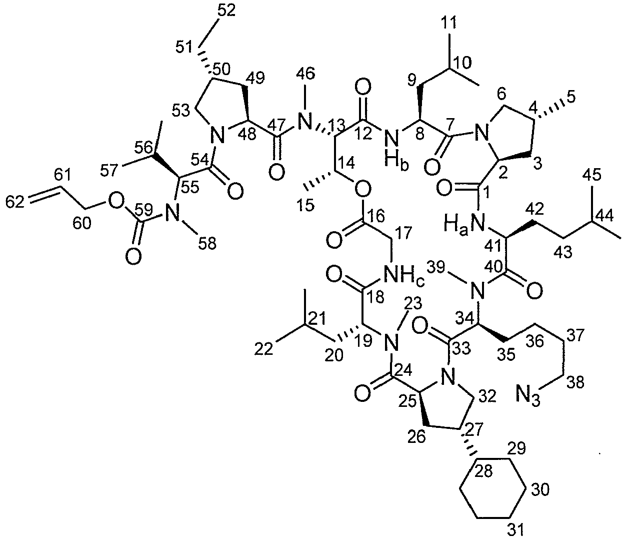
HRMS (ESI): Calculated for C₆₈H₁₁₄N₁₃O₁₃⁺ [M+H]⁺: 1320.8654, found: 1320.8668.

**Acetyl-*N*-Me-I-Val-I-EtPro-*N*-Me-cyclo-O-(Gly-*N*-Me-d-Leu-I-CyPro-N_{α}-Me-I-Lys(N₃)-I-HoLeu-I-MePro-I-Leu)-I-Thr [M106]**

According to **GP10,** 50.0 mg (37.9 µmol, 1.0 eq.) of the Alloc-protected amine **M69** was treated with 31.5 mg (201 µmol, 5.3 eq.) DMBA and 2.4 mg (2.08 µmol, 5 mol%) Pd(PPh₃)₄ in 2.5 mL anhydrous DCM for 3 h.

The resulting free amine was dissolved under Ar atmosphere in 2.5 mL anhydrous DCM and cooled to 0 °C before 49.5 µL (284 µmol, 7.5 eq., *p* = 0.742 g/mL) DIPEA was added. Subsequently, the solution was treated with 13.4 µL (190 µmol, 5.0 eq., *p* = 1.104 g/mL) acetyl chloride and then slowly warmed to RT.

After 15.5 h, the mixture was diluted with EtOAc and washed with 1 M HCI_{(aq.)}, H₂O, sat. NaHCO_{3(aq.)}, and sat. NaCk_{(aq.)} solution. The organic phase was dried over MgSO₄ and concentrated in vacuo. Automated column chromatography (C₁₈-SiO₂, H₂O/MeCN 9:1 → 0:10) of the crude product, followed by lyophilization, yielded 38.4 mg (30.0 µmol, 79 %) of cyclic peptide **M106** as a colorless lyophilizate
${\left[α\right]}_{D}^{20} = - 65.4$ (c = 0.5, CHCl₃) 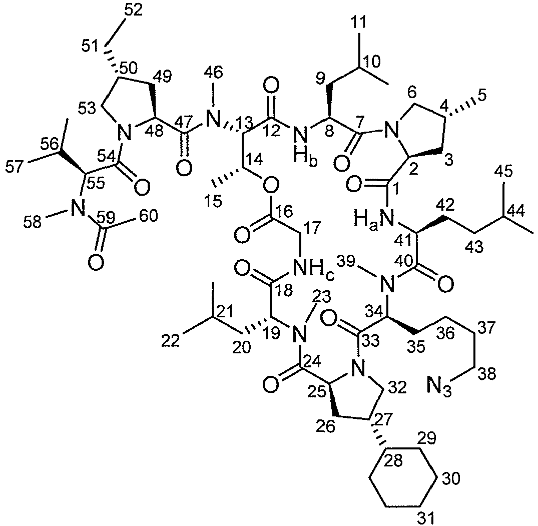
**HRMS** (ESI): Calculated for C₆₆H₁₁₁N₁₃NaO₁₂⁺ [M+Na]⁺: 1300.8367, found: 1300.8364.

### Alloc-N-Me-I-Val-I-MeOPro-O-(Gly-N-Me-d-Leu-I-CyPro-N_{α}-Me-I-Lys(N₃)-I-HoLeu-Boc)-N-Me-I-Thr-I-Leu-I-MePro-Ot-Bu [M100]

According to **GP8,** 63.5 mg (62.4 µmol, 1.0 eq.) of hexapeptide **M98** was treated with 93.5 µL (624 µmol, 10 eq., ρ = 0.976 g/mL) tren in 1.0 mL DCM for 50 min. The resulting free amine was then coupled according to **GP6** with 48.5 mg (72.9 µmol, 1.2 eq.) of carboxylic acid **M49,** 34.1 mg (79.6 µmol, 1.3 eq.) COMU, and 17.2 µL (156 µmol, 2.5 eq., *ρ* = 0.920 g/mL) NMM in a mixture of 1.0 mL anhydrous DCM and 0.5 mL anhydrous DMF for 15 h. After automated column chromatography of the crude product (C₁₈-SiO₂, H₂O/MeCN 9:1 → 0:10) and lyophilization, 75.8 mg (52.6 µmol, 84 %) of decapeptide **M100** was isolated as a colorless lyophilizate.
${\left[α\right]}_{D}^{20} = - 66.6$ (c = 0.5, CHCl₃) 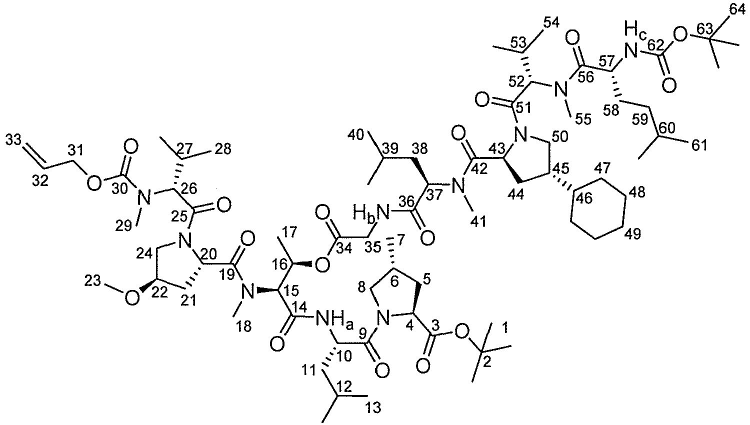
**HRMS** (ESI): Calculated for C₇₅H₁₂₉N₁₀O₁₇⁺ [M+H]⁺: 1441.9532, found: 1441.9537.

**Alloc-*N*-Me-I-Val-I-MeOPro-*N*-Me-cyclo-O-(Gly-*N*-Me-d-Leu-I-Pro-N-Me-I-Val-I-HoLeu-I-MePro-I-Leu)-I-Thr [M102]**

According to GP9, 57.2 mg (39.7 µmol, 1.0 eq.) of decapeptide **M100** was treated with 2.0 mL of a 1:1 mixture of DCM/TFA for 2.5 h. The resulting fully deprotected peptide was then treated with 79.5 mg (207 µmol, 5.2 eq.) FDPP and 76.1 µL (437 µmol, 11 eq., *p* = 0.742 g/mL) DIPEA in a total of 40 mL of anhydrous DMF (addition time = 60 min, stirring time = 15 min). Automated column chromatography (C₁₈-SiO₂, H₂O/MeCN (+0.1 % HCOOH) 9:1 → 0:10) of the crude product, followed by lyophilization, yielded 27.0 mg (21.3 µmol, 54 %) of the macrocycle **M102** as a colorless lyophilizate.
${\left[α\right]}_{D}^{20} = - 53.4$ (c = 0.5, CHCl₃) 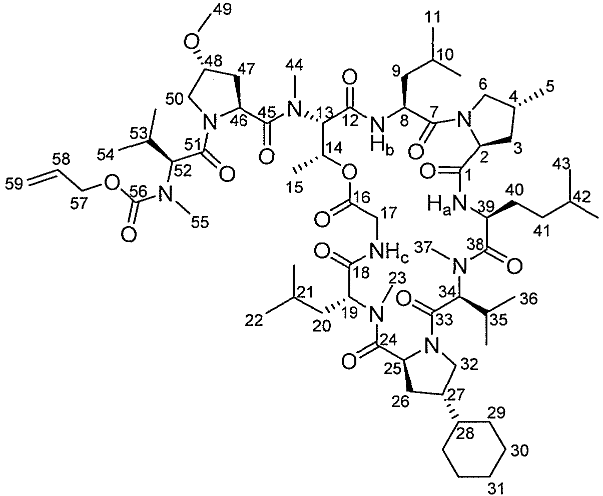
**HRMS** (ESI): Calculated for C₆₆H₁₁₁N₁₀O₁₄⁺ [M+H]⁺: 1267.8276, found: 1267.8280.

### Alloc-N-Me-I-Val-i-EtOPro-O-(Gly-N-Me-d-Leu-I-CyPro-N-Me-I-Val-I-HoLeu-Boc)-N-Me-I-Thr-I-Leu-I-MePro-Ot-Bu [M101]

According to **GP8,** 62.8 mg (60.9 µmol, 1.0 eq.) of hexapeptide **M99** was treated with 95.0 µL (609 µmol, 10 eq., *ρ* = 0.976 g/mL) tren in 1.0 mL of DCM for 50 min. The resulting free amine was then coupled according to **GP6** with 48.0 mg (72.2 µmol, 1.2 eq.) of carboxylic acid **M49,** 30.8 mg (71.9 µmol, 1.2 eq.) COMU, and 16.7 µL (152 µmol, 2.5 eq., *ρ* = 0.920 g/mL) NMM in a mixture of 1.0 mL of anhydrous DCM and 0.5 mL of anhydrous DMF for 25 h. After automated column chromatography (C₁₈-SiO₂, H₂O/MeCN 9:1 → 0:10) of the crude product and lyophilization, 76.6 mg (52.6 µmol, 86 %) of decapeptide **M101** was isolated as a colorless lyophilizate.
${\left[α\right]}_{D}^{20} = - 83.0$ (c = 0.5, CHCl₃) 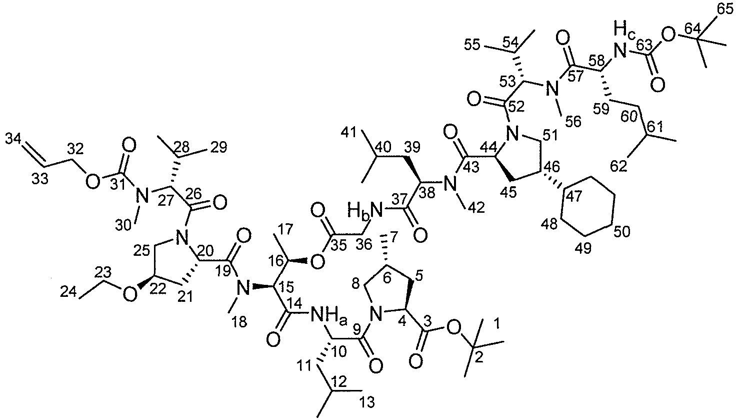
**HRMS** (ESI): Calculated for C₇₆H₁₃₁N₁₀O₁₇⁺ [M+H]⁺: 1455.9688, found: 1455.9705.

### Alloc-N-Me-I-Val-I-EtOPro-N-Me-cyclo-O-(Gly-N-Me-d-Leu-I-Pro-N-Me-I-Val-I-HoLeu-I-MePro-I-Leu)-I-Thr [M103]

According to GP9, 58.3 mg (40.0 µmol, 1.0 eq.) of decapeptide **M101** was treated with 2.0 mL of a 1:1 mixture of DCM/TFA for 3 h. The resulting fully deprotected peptide was then treated with 79.0 mg (207 µmol, 5.1 eq.) FDPP and 76.6 µL (440 µmol, 11 eq., *p* = 0.742 g/mL) DIPEA in a total of 40 mL of anhydrous DMF (addition time = 60 min, stirring time = 15 min). Automated column chromatography (C₁₈-SiO₂, H₂O/MeCN (+0.1 % HCOOH) 9:1 → 0:10) of the crude product, followed by lyophilization, yielded 23.6 mg (18.4 µmol, 46 %) of macrocycle **M103** as a colorless lyophilizate.
${\left[α\right]}_{D}^{20} = - 55.0$ (c = 0.5, CHCl₃) 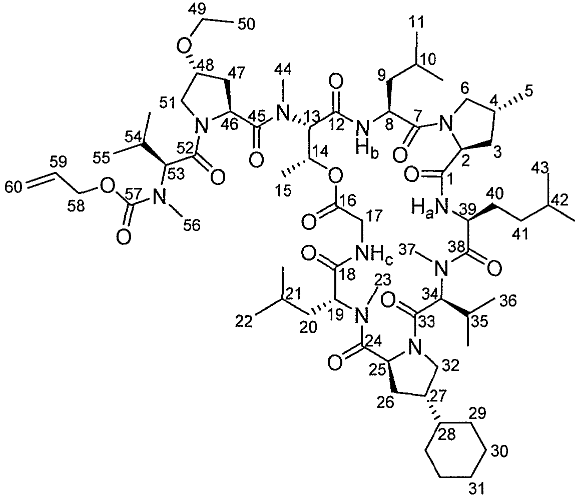
**HRMS** (ESI): Calculated for C₆₇H₁₁₃N₁₀O₁₄⁺ [M+H]⁺: 1281.8432, found: 1281.8435.

### Synthesis of Mycoplanecin A and its Derivatives

### Mycoplanecin A [M45]

According to **GP10,** 208 mg (176 µmol, 1.0 eq.) of the Alloc-protected amine **M44** was treated with 134 mg (860 µmol, 4.9 eq.) DMBA and 10.9 mg (9.41 µmol, 5 mol%) Pd(PPh₃)₄ in 2.3 mL anhydrous DCM for 2.5 h.

According to **GP11,** to prepare the acid chloride solution, 112 mg (1.09 mmol, 6.2 eq.) of 2-oxobutyric acid was treated with 94.0 µL (1.07 mmol, 6.1 eq., p = 1.450 g/mL) of oxalyl chloride and one drop of anhydrous DMF in 1.0 mL anhydrous DCM for 2.5 h. This solution was then treated with a solution of the previously obtained free amine and 306 µL (1.76 mmol, 10 eq., p = 0.742 g/mL) DIPEA in 1.0 mL anhydrous DCM for 19 h. Automated column chromatography (C₁₈-SiO₂, H₂O/MeCN 9:1 → 0:10) of the crude product followed by preparative HPLC purification (H₂O/MeCN 3:7 → 0:10) and lyophilization yielded 145 mg (176 µmol, 70 %) of Mycoplanecin A **(M45)** as a colorless lyophilizate.
${\left[α\right]}_{D}^{20} = - 57.5$ (c = 0.4, CHCl₃); Lit.: ${\left[α\right]}_{D}^{25} = - 66$ (c = 0.4, CHCl₃)^{[20]} 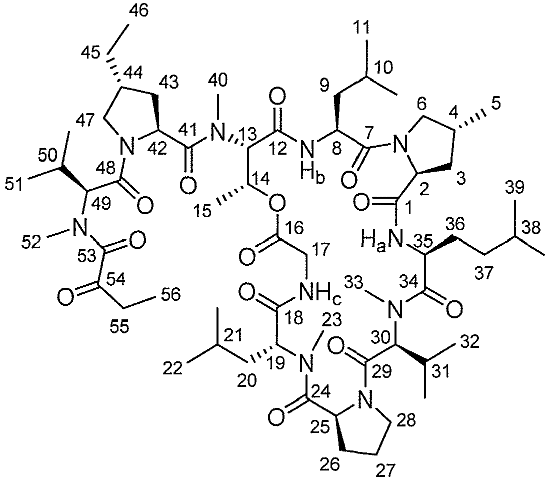
**HRMS** (ESI): Calculated for C₆₁H₁₀₂N₁₀NaO₁₃⁺ [M+Na]⁺: 1205.7520, found: 1205.7511.

### Cyclohexylmycoplanecin A [M52]

According to **GP10,** 9.8 mg (7.74 µmol, 1.0 eq.) of the Alloc-protected amine **M51** was treated with 30.0 mg (192 µmol, 25 eq.) DMBA and 0.5 mg (0.433 µmol, 6 mol%) Pd(PPh₃)₄ in 0.5 mL anhydrous DCM for 4 h.

According to **GP11,** to prepare the acid chloride solution, 15.8 mg (155 µmol, 20 eq.) of 2-oxobutyric acid was treated with 13.0 µL (1.57 mmol, 20 eq., *ρ* = 1.450 g/mL) oxalyl chloride and one drop of anhydrous DMF in 0.5 mL anhydrous DCM for 2.5 h. This solution was then added to a solution of the previously obtained free amine and 40.0 µL (230 µmol, 30 eq., *ρ* = 0.742 g/mL) DIPEA in 0.5 mL anhydrous DCM and stirred for 16 h. Automated column chromatography (C₁₈-SiO₂, H₂O/MeCN 9:1 → 0:10) of the crude product followed by preparative HPLC purification (H₂O/MeCN 9:1 → 0:10) and lyophilization yielded 6.9 mg (5.45 µmol, 70 %) of Cyclohexylmycoplanecin A **(M52)** as a colorless lyophilizate.
${\left[α\right]}_{D}^{20} = - 50.0$ (c = 0.3, CHCl₃) 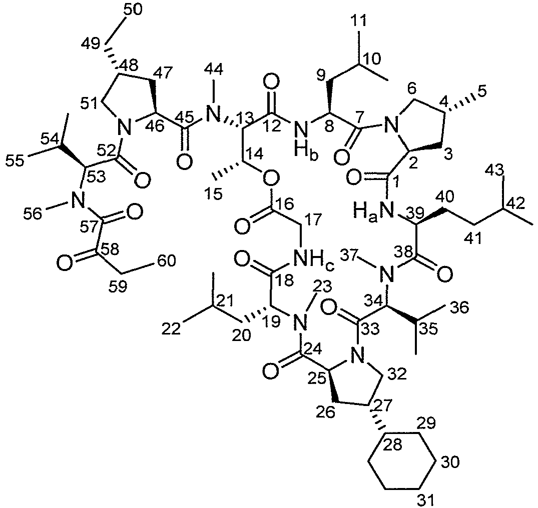
**HRMS** (ESI): Calculated for C₆₇H₁₁₂N₁₀NaO₁₃⁺ [M+Na]⁺: 1287.8303, found: 1287.8298.

### Acetyl-cyclohexylmycoplanecin A [M57]

According to **GP10,** 237 mg (187 µmol, 1.0 eq.) of the Alloc-protected amine **M51** was treated with 149 mg (954 µmol, 5.1 eq.) DMBA and 10.9 mg (9.41 µmol, 5 mol%) Pd(PPh₃)₄ in 2.5 mL anhydrous DCM for 3 h.

The resulting free amine was dissolved under an Ar atmosphere in 2.5 mL anhydrous DCM and cooled to 0 °C before 244 µL (1.40 mmol, 7.5 eq., *ρ* = 0.742 g/mL) DIPEA was added. Subsequently, 66.4 µL (934 µmol, 5.0 eq., *ρ* = 1.104 g/mL) acetyl chloride was added dropwise, and the reaction mixture was slowly warmed to RT.

After 19 h, the reaction mixture was diluted with EtOAc and washed with 1 M HCI_{(aq.)}, H₂O, sat. NaHCO_{3(aq.)} and sat. NaCk_{(aq.)} solution. The organic phase was dried over MgSO₄ and concentrated in vacuo. Automated column chromatography (C₁₈-SiO₂, H₂O/MeCN 9:1 → 0:10) of the crude product followed by preparative HPLC purification (H₂O/MeCN 3:7 → 0:10) and lyophilization yielded 169 mg (138 µmol, 74 %) of the Mycoplanecin derivative **M57** as a colorless lyophilizate.
${\left[α\right]}_{D}^{20} = - 85.6$ (c = 0.25, CHCl₃) 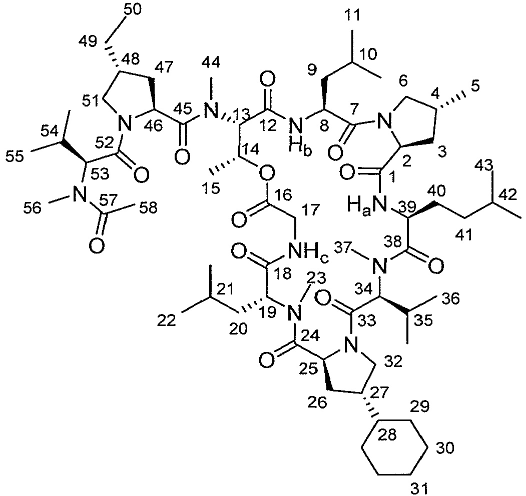
**HRMS** (ESI): Calculated for C₆₅H₁₁₁N₁₀O₁₂⁺ [M+H]⁺: 1223.8377, found: 1223.8362.

### Butyryl-cyclohexylmycoplanecin A [M58]

According to **GP10,** 14.0 mg (11.1 µmol, 1.0 eq.) of the Alloc-protected amine **M51** was treated with 17.2 mg (111 µmol, 10 eq.) DMBA and 0.3 mg (0.260 µmol, 5 mol%) Pd(PPh₃)₄ in 0.5 mL anhydrous DCM for 2.5 h.

The resulting free amine was dissolved under N₂ atmosphere in 0.5 mL anhydrous DCM and cooled to 0 °C before 58.0 µL (333 µmol, 30 eq., *ρ* = 0.742 g/mL) DIPEA was added. Subsequently, 23.2 µL (223 µmol, 20 eq, p = 1.025 g/mL) of butyryl chloride was added dropwise, and the reaction mixture was slowly warmed to RT.

After 17 h, the reaction mixture was diluted with EtOAc and washed with 1 M HCI_{(aq.)}, H₂O, sat. NaHCO_{3(aq.)} and sat. NaCk_{(aq.)} solution. The organic phase was dried over MgSO₄ and concentrated in vacuo. Automated column chromatography (C₁₈-SiO₂, H₂O/MeCN 9:1 → 0:10) of the crude product followed by preparative HPLC purification (H₂O/MeCN 2:8 → 0:10) and lyophilization yielded 9.2 mg (7.35 µmol, 66 %) of the Mycoplanecin derivative **M58** as a colorless lyophilizate.
${\left[α\right]}_{D}^{20} = - 64.3$ (c = 0.3, CHCl₃) 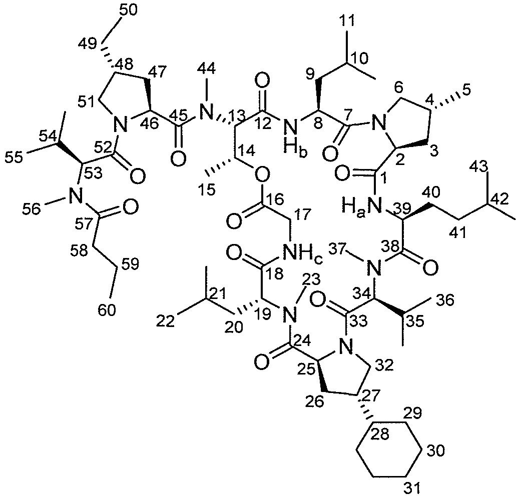
**HRMS** (ESI): Calculated for C₆₇H₁₁₅N₁₀O₁₂⁺ [M+H]⁺: 1251.8690, found: 1251.8656.

### Caproyl-cyclohexylmycoplanecin A [M59]

According to **GP10,** 10.1 mg (7.98 µmol, 1.0 eq.) of the Alloc-protected amine **M51** was treated with 9.9 mg (63.4 µmol, 8 eq.) DMBA and 0.7 mg (0.594 µmol, 7 mol%) Pd(PPh₃)₄ in 0.5 mL anhydrous DCM for 3.5 h.

According to **GP11,** to prepare the acid chloride solution, 24.6 mg (143 µmol, 18 eq.) of capric acid was treated with 12.2 µL (139 µmol, 18 eq., *p* = 1.450 g/mL) oxalyl chloride and one drop of anhydrous DMF in 0.5 mL of anhydrous DCM for 2.5 h. This solution was then treated with a solution of the previously obtained free amine and 41.8 µL (240 µmol, 30 eq, *ρ* = 0.742 g/mL) DIPEA in 0.5 mL anhydrous DCM for 16 h. Automated column chromatography (C₁₈-SiO₂, H₂O/MeCN 9:1 → 0:10) of the crude product followed by preparative HPLC purification (H₂O/MeCN (+0.1 % HCOOH) 2:8 → 0:10) and lyophilization yielded 8.1 mg (6.06 µmol, 76 %) of the Mycoplanecin derivative **M59** as a colorless lyophilizate.
${\left[α\right]}_{D}^{20} = - 64.8$ (c = 0.25, CHCl₃) 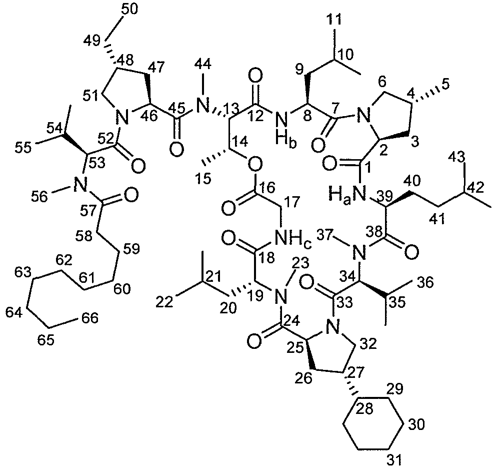
**HRMS** (ESI): Calculated for C₇₃H₁₂₆N₁₀NaO₁₂⁺ [M+Na]⁺: 1357.9407, found: 1357.9449.

### p-Fluorbenzoyl-cyclohexylmycoplanecin A [M60]

According to **GP10,** 12.5 mg (9.88 µmol, 1.0 eq.) of the Alloc-protected amine **M51** was treated with 15.7 mg (101 µmol, 10 eq.) DMBA and 0.3 mg (0.260 µmol, 3 mol%) Pd(PPh₃)₄ in 0.5 mL anhydrous DCM for 3 h.

According to **GP11,** to prepare the acid chloride solution, 27.7 mg (198 µmol, 20 eq.) of p-fluorobenzoic acid was treated with 17.3 µL (198 µmol, 18 eq., *ρ* = 1.450 g/mL) oxalyl chloride and one drop of anhydrous DMF in 0.5 mL anhydrous DCM for 2.5 h. This solution was then treated with a solution of the previously obtained free amine and 51.6 µL (296 µmol, 30 eq., *ρ* = 0.742 g/mL) DIPEA in 0.5 mL anhydrous DCM for 17 h. Automated column chromatography (C₁₈-SiO₂, H₂O/MeCN 9:1 → 0:10) of the crude product followed by preparative HPLC purification (H₂O/MeCN 4:6 → 0:10) and lyophilization yielded 7.6 mg (5.83 µmol, 59 %) of Mycoplanecin derivative **M60** as a colorless lyophilizate.
${\left[α\right]}_{D}^{20} = - 44.8$ (c = 0.25, CHCl₃) 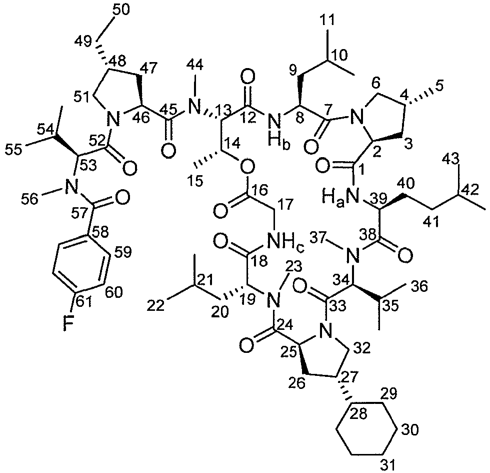
**HRMS** (ESI): Calculated for C₇₀H₁₁₁FN₁₀NaO₁₂⁺ [M+Na]⁺: 1325.8259, found: 1325.8248.

### Cyclohexanoyl-cyclohexylmycoplanecin A [M61]

According to **GP10,** 12.5 mg (9.88 µmol, 1.0 eq.) of the Alloc-protected amine **M51** was treated with 8.2 mg (52.5 µmol, 5.3 eq) DMBA and 0.3 mg (0.260 µmol, 3 mol%) Pd(PPh₃)₄ in 0.5 mL anhydrous DCM for 3 h.

According to **GP11,** to prepare the acid chloride solution, 25.7 mg (200 µmol, 20 eq.) cyclohexanecarboxylic acid was treated with 17.3 µL (198 µmol, 20 eq., *ρ* = 1.450 g/mL) oxalyl chloride and one drop of anhydrous DMF in 0.5 mL anhydrous DCM for 3.5 h. This solution was then treated with a solution of the previously obtained free amine and 68.8 µL (395 µmol, 40 eq., *ρ* = 0.742 g/mL) DIPEA in 0.5 mL of anhydrous DCM for 19 h. Automated column chromatography (C₁₈-SiO₂, H₂O/MeCN 9:1 → 0:10) of the crude product followed by preparative HPLC purification (H₂O/MeCN 4:6 → 0:10) and lyophilization yielded 6.4 mg (4.95 µmol, 50 %) of the Mycoplanecin derivative **M61** as a colorless lyophilizate.
${\left[α\right]}_{D}^{20} = - 56.4$ (c = 0.25, CHCl₃) 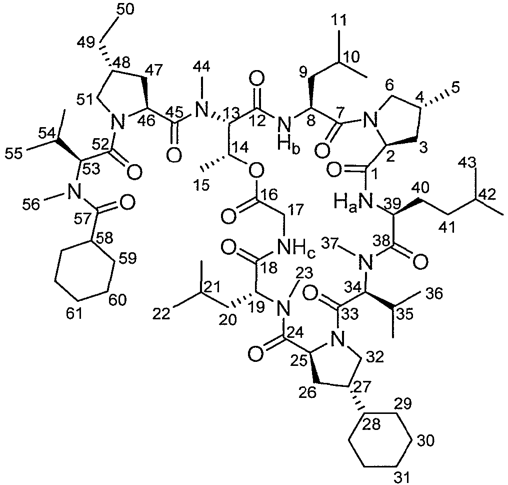
**HRMS** (ESI): Calculated for C₇₀H₁₁₈N₁₀NaO₁₂⁺ [M+Na]⁺: 1313.8823, found: 1313.8766.

### ω-Cyclohexylbutyryl-cyclohexylmycoplanecin A [M62]

According to **GP10,** 10.2 mg (8.06 µmol, 1.0 eq.) of Alloc-protected amine **M51** was treated with 8.2 mg (52.5 µmol, 6.5 eq.) DMBA and 0.5 mg (0.433 µmol, 5 mol%) Pd(PPh₃)₄ in 0.5 mL anhydrous DCM for 2.5 h.

According to **GP11,** to prepare the acid chloride solution, 27.0 mg (159 µmol, 20 eq.) 4-cyclohexylbutyric acid was treated with 13.8 µL (157 µmol, 20 eq., *ρ* = 1.450 g/mL) of oxalyl chloride and one drop of anhydrous DMF in 0.5 mL anhydrous DCM for 4 h. This solution was then treated with a solution of the previously obtained free amine and 42.2 µL (242 µmol, 30 eq., *p* = 0.742 g/mL) DIPEA in 0.5 mL anhydrous DCM for 17 h. Automated column chromatography (C₁₈-SiO₂, H₂O/MeCN 9:1 → 0:10) of the crude product followed by preparative HPLC purification (H₂O/MeCN (+0.1 % HCOOH) 4:6 → 0:10) and lyophilization yielded 7.7 mg (5.77 µmol, 72 %) of Mycoplanecin derivative **M62** as a colorless lyophilizate.
${\left[α\right]}_{D}^{20} = - 57.3$ (c = 0.15, CHCl₃) 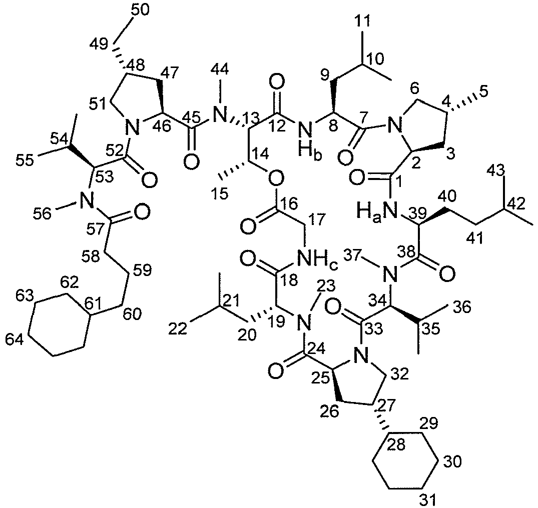
**HRMS** (ESI): Calculated for C₇₃H₁₂₄N₁₀NaO₁₂⁺ [M+Na]⁺: 1355.9292, found: 1355.9352.

### Di-I-Leucyl-cyclohexylmycoplanecin A [M81]

According to **GP10,** 10.7 mg (8.55 µmol, 1.0 eq.) of the Alloc-protected amine **M76** was treated with 8.7 mg (55.7 µmol, 6.5 eq.) DMBA and 0.5 mg (0.433 µmol, 5 mol%) Pd(PPh₃)₄ in 0.5 mL anhydrous DCM for 3 h.

According to **GP11,** a stock solution of the acid chloride was prepared from 33.1 mg (324 µmol, 1.0 eq.) 2-oxobutyric acid, 28.4 µL (324 µmol, 1.0 eq., *p* = 1.450 g/mL) oxalyl chloride, and one drop of anhydrous DMF in 2.5 mL anhydrous DCM. After 3 h, 0.66 mL (0.13 M, 85.5 µmol, 10 eq. relative to the free amine) of the freshly prepared acid chloride solution was treated with a solution of the previously obtained free amine and 29.8 µL (171 µmol, 20 eq., *ρ* = 0.742 g/mL) of DIPEA in 0.5 mL of anhydrous DCM for 20 h. Automated column chromatography (C₁₈-SiO₂, H₂O/MeCN 9:1 → 0:10) of the crude product followed by preparative HPLC purification (H₂O/MeCN 5:5 → 0:10) and lyophilization yielded 5.1 mg (4.07 µmol, 48 %) of Mycoplanecin derivative **M81** as a colorless lyophilizate. (EP745)
${\left[α\right]}_{D}^{20} = - 61.0$ (c = 0.3, CHCl₃) 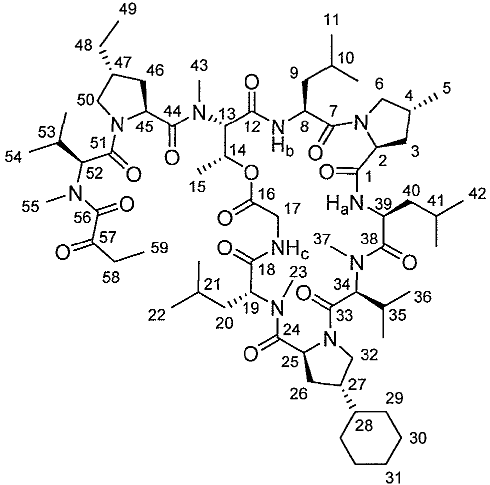
**HRMS** (ESI): Calculated for C₆₆H₁₁₀N₁₀NaO₁₃⁺ [M+Na]⁺: 1273.8146, found: 1273.8119.

**Di-I-Methylprolyl-cyclohexylmycoplanecin A [M83]**

According to **GP10,** 7.6 mg (6.07 µmol, 1.0 eq.) of Alloc-protected amine **M79** was treated with 5.3 mg (33.9 µmol, 5.6 eq.) DMBA and 0.4 mg (0.346 µmol, 6 mol%) Pd(PPh₃)₄ in 0.5 mL anhydrous DCM for 2.5 h.

According to **GP11,** a stock solution of the acid chloride was prepared from 33.1 mg (324 µmol, 1.0 eq.) 2-oxobutyric acid, 28.4 µL (324 µmol, 1.0 eq., p = 1.450 g/mL) oxalyl chloride, and one drop of anhydrous DMF in 2.5 mL anhydrous DCM. After 3 h, 0.47 mL (0.13 M, 60.1 µmol, 10 eq. relative to the free amine) of the freshly prepared acid chloride solution was treated with a solution of the previously obtained free amine and 21.0 µL (121 µmol, 20 eq., p = 0.742 g/mL) DIPEA in 0.5 mL anhydrous DCM for 19 h. Automated column chromatography (C₁₈-SiO₂, H₂O/MeCN 9:1 → 0:10) of the crude product followed by preparative HPLC purification (H₂O/MeCN 5:5 → 0:10) and lyophilization yielded 4.4 mg (3.51 µmol, 58 %) of Mycoplanecin derivative **M83** as a colorless lyophilizate.
${\left[α\right]}_{D}^{20} = - 61.3$ (c = 0.3, CHCl₃) 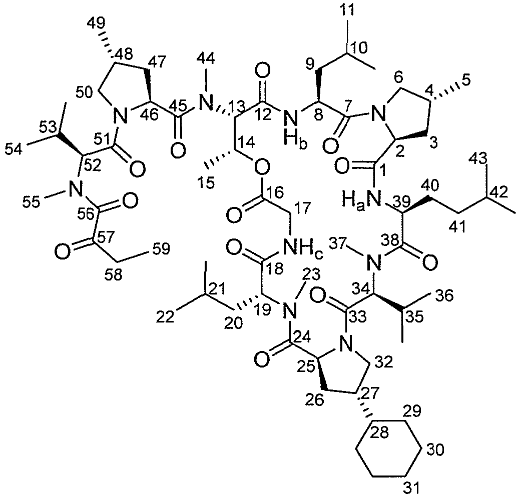
**HRMS** (ESI): Calculated for C₆₆H₁₁₁N₁₀O₁₃⁺ [M+H]⁺: 1251.8345, found: 1251.8327.

### Di-I-Methylprolyl-Methylmycoplanecin A [M82]

According to **GP10,** 7.3 mg (6.16 µmol, 1.0 eq.) of Alloc-protected amine **M80** were reacted with 6.0 mg (38.4 µmol, 5.6 eq.) DMBA and 0.4 mg (0.346 µmol, 6 mol%) Pd(PPh₃)₄ in 0.5 mL anhydrous DCM for 2 h.

According to **GP11,** a stock solution of the acid chloride was prepared from 33.1 mg (324 µmol, 1.0 eq.) 2-oxobutyric acid, 28.4 µL (324 mmol, 1.0 eq., p = 1.450 g/mL) oxalyl chloride, and one drop of anhydrous DMF in 2.5 mL anhydrous DCM. After 3 h, 0.48 mL (0.13 M, 61.6 µmol, 10 eq. based on the free amine) of the freshly prepared acid chloride solution was reacted with a solution of the previously obtained free amine and 22.0 µL (126 µmol, 20 eq., p = 0.742 g/mL) DIPEA in 0.5 mL anhydrous DCM for 21.5 h. Automated column chromatography (C₁₈-SiO₂, H₂O/MeCN 9:1 → 0:10) of the crude product followed by purification via preparative HPLC (H₂O/MeCN 5:5 → 0:10) yielded, after lyophilization, 3.5 mg (2.96 µmol, 48 %) of mycoplanecin derivative **M82** as a colorless lyophilizate.
${\left[α\right]}_{D}^{20} = - 64.3$ (c = 0.3, CHCl₃) 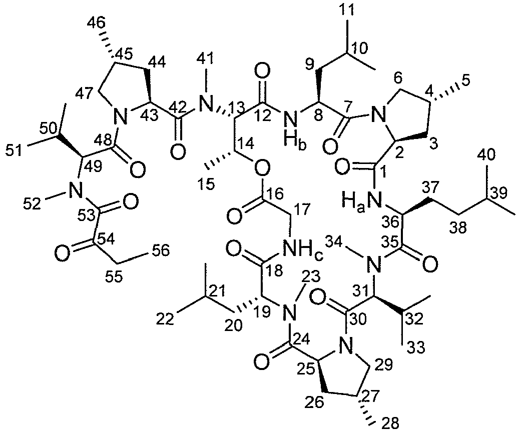
**HRMS** (ESI): Calculated for C₆₁H₁₀₃N₁₀O₁₃⁺ [M+H]⁺: 1183.7701, found: 1183.7722.

### ω-Cyclohexylbutyryl-I-methoxyprolyl-cyclohexylmycoplanecin A [M104]

According to **GP10,** 9.4 mg (7.42 µmol, 1.0 eq.) of the Alloc-protected amine **M102** were reacted with 6.6 mg (42.2 µmol, 5.7 eq.) DMBA and 0.4 mg (0.346 µmol, 5 mol%) Pd(PPh₃)₄ in 0.5 mL anhydrous DCM for 2 h.

According to **GP11,** to prepare the acid chloride solution, 24.3 mg (143 µmol, 19 eq.) 4-cyclo-hexylbutyric acid were reacted with 12.3 µL (141 µmol, 19 eq., p = 1.450 g/mL) oxalyl chloride and one drop of anhydrous DMF in 0.5 mL of anhydrous DCM for 3 h. This solution was then reacted with a solution of the previously obtained free amine and 38.7 µL (222 µmol, 30 eq., p = 0.742 g/mL) DIPEA in 0.5 mL anhydrous DCM for 17 h. Automated column chromatography (C₁₈-SiO₂, H₂O/MeCN (+0.1 % HCOOH) 9:1 → 0:10) of the crude product followed by purification via preparative HPLC (H₂O/MeCN 5:5 → 0:10) yielded, after lyophilization, 8.2 mg (6.14 µmol, 83 %) of the mycoplanecin derivative **M104** as a colorless lyophilizate.
${\left[α\right]}_{D}^{20} = - 68.0$ (c = 0.5, CHCl₃) 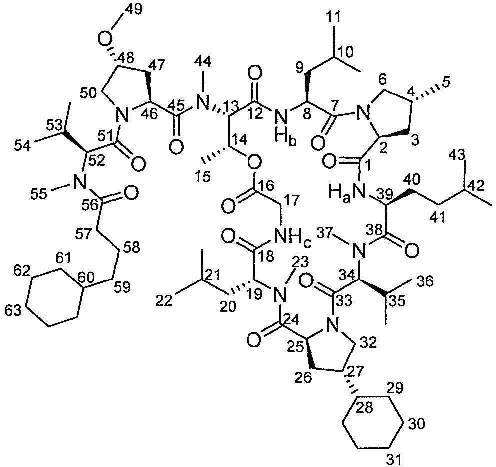
**HRMS** (ESI): Calculated for C₇₂H₁₂₂N₁₀NaO₁₃⁺ [M+Na]⁺: 1357.9085, found: 1357.9094.

### ω-Cyclohexylbutyryl-I-ethoxyprolyl-cyclohexylmycoplanecin A [M105]

According to **GP10,** 10.4 mg (8.11 µmol, 1.0 eq.) of the Alloc-protected amine **M103** was treated with 6.9 mg (44.2 µmol, 5.4 eq.) DMBA and 0.3 mg (0.260 µmol, 3 mol%) Pd(PPh₃)₄ in 0.5 mL anhydrous DCM for 2 h.

According to **GP11,** to prepare the acid chloride solution, 27.4 mg (161 µmol, 20 eq.) 4-cyclo-hexylbutyric acid were reacted with 14.0 µL (160 µmol, 20 eq., p = 1.450 g/mL) oxalyl chloride and one drop of anhydrous DMF in 0.5 mL of anhydrous DCM for 3 h. This solution was then reacted with a solution of the previously obtained free amine and 42.4 µL (243 µmol, 30 eq., p = 0.742 g/mL) DIPEA in 0.5 mL anhydrous DCM for 18 h. Automated column chromatography (C₁₈-SiO₂, H₂O/MeCN 9:1 → 0:10) of the crude product followed by purification via preparative HPLC (H₂O/MeCN 2:8 → 0:10) yielded, after lyophilization, 9.3 mg (6.89 µmol, 85 %) of the mycoplanecin derivative **M105** as a colorless lyophilizate.
${\left[α\right]}_{D}^{20} = - 57.6$ (c = 0.5, CHCl₃) 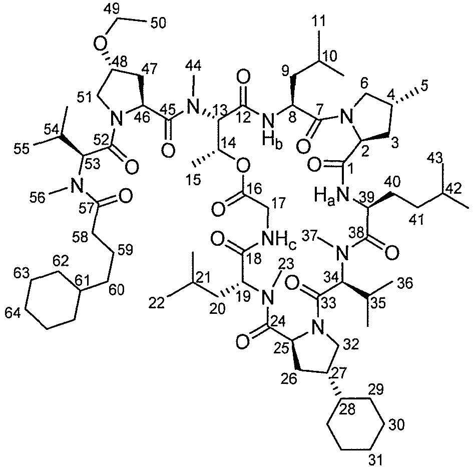
**HRMS** (ESI): Calculated for C₇₃H₁₂₄N₁₀NaO₁₃⁺ [M+Na]⁺: 1371.9258, found: 1371.9242.

### Acetyl-I-methoxyprolyl-cyclohexylmycoplanecin A [M114]

According to **GP10,** 13.0 mg (10.3 µmol, 1.0 eq.) of the Alloc-protected amine **M102** were reacted with 8.8 mg (56.4 µmol, 5.5 eq.) DMBA and 0.3 mg (0.260 µmol, 3 mol%) Pd(PPh₃)₄ in 0.6 mL anhydrous DCM for 1.5 h.

The resulting free amine was dissolved under Ar atmosphere in 0.5 mL anhydrous DCM and cooled to 0 °C before 13.5 µL (77.3 µmol, 7.5 eq., p = 0.742 g/mL) DIPEA were added. Subsequently, the solution was treated with 3.67 µL (51.5 µmol, 5.0 eq., *ρ* = 1.104 g/mL) acetyl chloride and then slowly warmed to RT. After 17 h, it was diluted with EtOAc and washed with 1 M HCl_{(aq.)}, followed by H₂O, sat. NaHCO_{3(aq.)}, and sat. NaCl_{(aq.)} solution, the organic phase was dried over MgSO₄ and the solvent was removed in vacuo. Automated column chromatography (C₁₈-SiO₂, H₂O/MeCN (+0.1 % HCOOH) 9:1 → 0:10) of the crude product and subsequent purification via preparative HPLC (H₂O/MeCN (+0.1 % HCOOH) 3:7 → 0:10) yielded, after lyophilization, 10.1 mg (8.24 µmol, 80 %) of the mycoplanecin derivative **M114** as a colorless lyophilizate.
${\left[α\right]}_{D}^{20} = - 58.5$ (c = 1.0, CHCl₃) 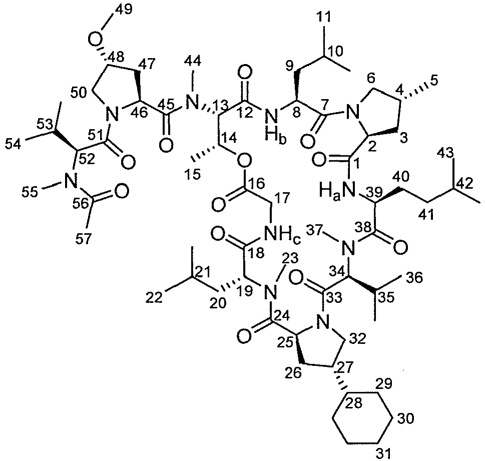
**HRMS** (ESI): Calculated for C₆₅H₁₀₉N₉NaO₁₃⁺ [M+Na]⁺: 1247.8071, found: 1247.8024.

### Acetyl-I-ethoxyprolyl-cyclohexylmycoplanecin A [M115]

According to **GP10,** 14.8 mg (11.5 µmol, 1.0 eq.) of the Alloc-protected amine **M103** were reacted with 9.0 mg (57.7 µmol, 5.0 eq.) DMBA and 0.7 mg (0.606 µmol, 5 mol%) Pd(PPh₃)₄ in 0.6 mL anhydrous DCM for 3 h.

The resulting free amine was dissolved under Ar atmosphere in 0.6 mL anhydrous DCM and cooled to 0 °C before 15.0 µL (86.3 µmol, 7.5 eq., p = 0.742 g/mL) DIPEA were added. Subsequently, the solution was treated with 4.12 µL (57.9 µmol, 5.0 eq., *ρ =* 1.104 g/mL) acetyl chloride and then slowly warmed to RT. After 18 h, it was diluted with EtOAc and washed with 1 M HCl_{(aq.)}, followed by H₂O, sat. NaHCO_{3(aq.)}, and sat. NaCl_{(aq.)} solution, the organic phase was dried over MgSO₄ and the solvent was removed in vacuo. Automated column chromatography (C₁₈-SiO₂, H₂O/MeCN (+0.1 % HCOOH) 9:1 → 0:10) of the crude product and subsequent purification via preparative HPLC (H₂O/MeCN (+0.1 % HCOOH) 3:7 → 0:10) yielded, after lyophilization, 4.9 mg (3.95 µmol, 34 %) of the mycoplanecin derivative **M115** as a colorless lyophilizate.
${\left[α\right]}_{D}^{20} = - 51.0$ (c = 0.5, CHCl₃) 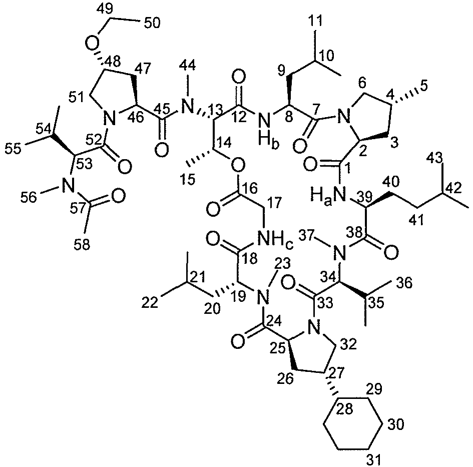
**HRMS** (ESI): Calculated for C₆₆H₁₁₁N₉NaO₁₃⁺ [M+Na]⁺: 1261.8228, found: 1261.8198.

### Acetyl-ω-[4-(morpholinomethyl)-1H-1,2,3-triazol-1-yl]-I-lysyl-cyclohexylmycoplanecin A [M107]

4.9 mg (3.83 µmol, 1.0 eq.) of azide **M106** were dissolved in 0.6 mL of a 1:1 mixture of THF/H₂O and treated with 2.30 µL (2.30 µmol, 0.6 eq., 1.0 M in H₂O) of a freshly prepared sodium ascorbate solution, 1.90 µL (1.0 M, 1.90 µmol, 0.5 eq.) of CuSO_{4(aq.)}, and 0.57 µL (4.63 µmol, 1.2 eq., p = 1.002 g/mL) of N-propargylmorpholine at RT.

After 15.5 h, the solvent was removed in vacuo and the residue was purified by automated column chromatography (C₁₈-SiO₂, H₂O/MeCN (+0.1 % HCOOH) 9:1 → 0:10), followed by preparative HPLC (H₂O/MeCN (+0.1 % HCOOH) 9:1 → 0:10). After lyophilization of the isolated fraction, 3.5 mg (2.49 µmol, 65 %) of the mycoplanecin derivative **M107** were obtained as a colorless lyophilizate.
${\left[α\right]}_{D}^{20} = - 44.0$ (c = 0.25, CHCl₃) 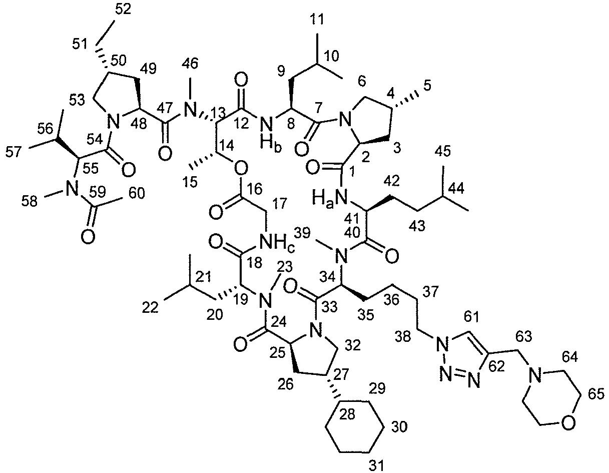
**HRMS** (ESI): Calculated for C₇₃H₁₂₃N₁₄O₁₃⁺ [M+H]⁺: 1403.9389, found: 1403.9385.

### Acetyl-ω-[4-ethinylestradiolyl-1H-1,2,3-triazol-1-yl]-I-lysyl-cyclohexylmycoplanecin A [M108]

5.3 mg (4.14 µmol, 1.0 eq.) of azide **M106** were dissolved in 0.6 mL of a 1:1 mixture of THF/H₂O and treated with 2.50 µL (1.0 M in H₂O, 2.50 µmol, 0.6 eq.) of a freshly prepared sodium ascorbate solution, 2.10 µL (1.0 M, 2.10 µmol, 0.5 eq.) CuSO_{4(aq.)}, and 1.7 mg (5.74 µmol, 1.4 eq.) ethinylestradiol at RT.

After 16 h, the solvent was removed in vacuo and the residue was purified by automated column chromatography (C₁₈-SiO₂, H₂O/MeCN (+0.1 % HCOOH) 9:1 → 0:10), followed by preparative HPLC (H₂O/MeCN (+0.1 % HCOOH) 7:3 → 0:10). After lyophilization of the isolated fraction, 5.3 mg (3.36 µmol, 81%) of the mycoplanecin derivative **M108** were obtained as a colorless lyophilizate.
${\left[α\right]}_{D}^{20} = - 31.6$ (c = 0.25, CHCl₃) 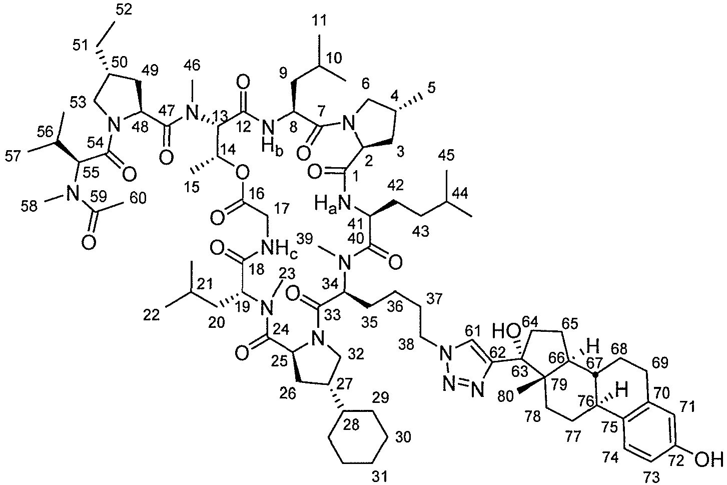
**HRMS** (ESI): Calculated for C₈₆H₁₃₆N₁₃O₁₄⁺ [M+H]⁺: 1575.0324, found: 1575.0326.

### Acetyl-ω-[4-cyclopropyl-1H-1,2,3-triazol-1-yl]-I-lysyl-cyclohexylmycoplanecin A [M109]

5.0 mg (3.91 µmol, 1.0 eq.) of azide **M106** and 0.50 µL (5.92 µmol, 1.5 eq., *ρ* = 0.783 g/mL) cyclopropylacetylene were dissolved in 0.6 mL of a 1:1 mixture of THF/H₂O and treated with 2.30 µL (1.0 M in H₂O, 2.30 µmol, 0.6 eq.) of a freshly prepared sodium ascorbate solution and 2.00 µL (1.0 M, 2.00 µmol, 0.5 eq.) of CuSO_{4(aq.)} at RT. After 17 h, an additional 2.30 µL (1.0 M in H₂O, 2.30 µmol, 0.6 eq.) of a freshly prepared sodium ascorbate solution and 1.00 µL (11.8 µmol, 3.0 eq., *ρ* = 0.783 g/mL) of cyclopropylacetylene were added.

After 15.5 h, the solvent was removed in vacuo and the residue was purified by automated column chromatography (C₁₈-SiO₂, H₂O/MeCN (+0.1 % HCOOH) 9:1 → 0:10), followed by preparative HPLC (H₂O/MeCN (+0.1 % HCOOH) 4:6 → 0:10). After lyophilization of the isolated fraction, 3.5 mg (2.60 µmol, 67 %) of the mycoplanecin derivative **M109** were obtained as a colorless lyophilizate.
${\left[α\right]}_{D}^{20} = - 63.5$ (c = 0.4, CHCl₃) 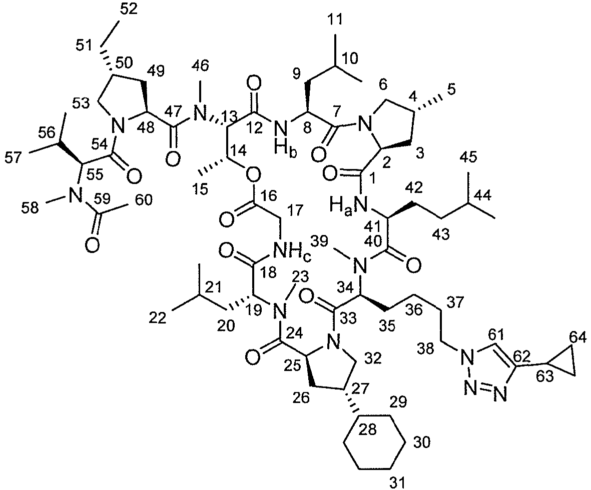
**HRMS** (ESI): Calculated for C₇₁H₁₁₇N₁₃O₁₂⁺ [M+H]⁺: 1344.9017, found: 1344.9015.

### 1-O-Propargyl-2,3,4,6-tetra-O-acetyl-β-d-glucopyranoside [M110]^{[21]}

To a solution of 203 mg (521 µmol, 1.0 eq.) β-d-glucose pentaacetate in 5.0 mL anhydrous DCM, 99.0 µL (781 µmol, 1.5 eq., *ρ =* 1.120 g/mL) BF₃·OEt₂ followed by 45.1 µL (781 µmol, 1.5 eq., *ρ* = 0.972 g/mL) propargyl alcohol were added under N₂ atmosphere at RT. The slightly brownish solution was stirred for 22 h and then treated with 110 mg (796 µmol, 1.5 eq.) of K₂CO₃ and stirred for an additional 17.5 h.

After that, it was diluted with EtOAc, washed three times with H₂O and once with sat. NaCl_{(aq.)} solution, before the organic phase was dried over MgSO₄ and the solvent was removed in vacuo. Finally, the resulting residue was purified by automated column chromatography (C₁₈-SiO₂, H₂O/MeCN 9:1 → 0:10) and the isolated fraction was lyophilized. In total, 149 mg (385 µmol, 74 %) of the glucose derivative **M110** were isolated as a colorless lyophilizate.
${\left[α\right]}_{D}^{20} = - 43.7$ (c = 1.0, CHCl₃); Lit.: ${\left[α\right]}_{D}^{25} = - 42.6$ (c = 1.0, CHCl₃)^{[22]}
R_{f} = 0.38 (*n*-Pentane/EtOAc 1:1) 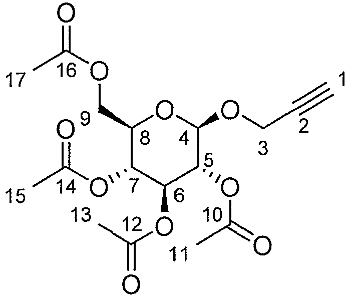
**HRMS** (ESI): Calculated for C₁₇H₂₂NaO₁₀⁺ [M+Na]⁺: 409.1105, found: 409.1103.

### Acetyl-ω-[4-tetraacetyl-methyl-α-d-glucopyranosidyl-1H-1,2,3-triazol-1-yl]-I-lysyl-cyclohexylmycoplanecin A [M111]

5.0 mg (3.91 µmol, 1.0 eq.) of azide **M106** and 2.0 mg (5.18 µmol, 1.3 eq.) of glucose derivative **M110** were dissolved in 0.6 mL of a 1:1 mixture of THF/H₂O and treated with 2.30 µL (1.0 M in H₂O, 2.30 µmol, 0.6 eq.) of a freshly prepared sodium ascorbate solution and 2.00 µL (1.0 M, 2.00 µmol, 0.5 eq.) CuSO_{4(aq.)} at RT.

After 20.5 h, the solvent was removed in vacuo and the residue was purified by automated column chromatography (C₁₈-SiO₂, H₂O/MeCN 9:1 → 0:10), followed by preparative HPLC (H₂O/MeCN 4:6 → 0:10). After lyophilization of the isolated fraction, 3.9 mg (2.34 µmol, 60 %) of the mycoplanecin derivative **M111** were obtained as a colorless lyophilizate.
${\left[α\right]}_{D}^{20} = - 45.6$ (c = 0.25, CHCl₃) 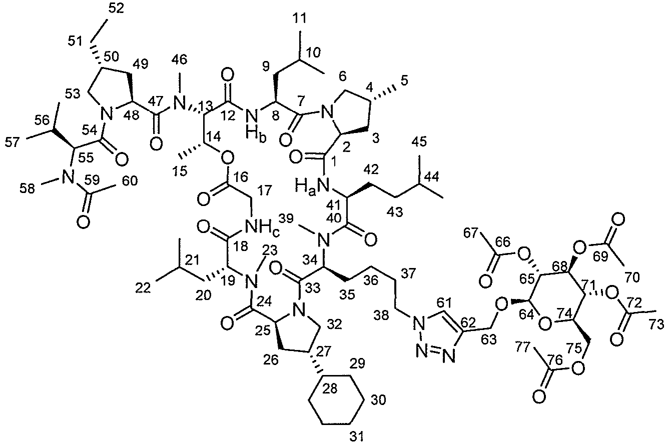
**HRMS** (ESI): Calculated for C₈₃H₁₃₄N₁₃O₂₂⁺ [M+H]⁺: 1664.9761, found: 1664.9758.

### Propargyl-β-d-glucopyranoside [M112]^{[23]}

To a suspension of 72.3 mg (187 µmol, 1.0 eq.) of peracetylated sugar **M110** in 0.62 mL of MeOH, 3.1 mg (57.4 µmol, 0.3 eq.) of NaOMe was added. After 23 h, the mixture was neutralized with Amberlite IR120 and filtered, rinsing with MeOH. Finally, the solvent was removed in vacuo, yielding 40.9 mg (187 µmol, 100 %) of the glucose derivative **M112** as a colorless oil.
${\left[α\right]}_{D}^{20} = - 80.1$ (c = 1.0, MeOH); Lit.: ${\left[α\right]}_{D}^{26} = - 91.9$ (c = 0.66, MeOH)
**R_{f}** = 0.03 (n-Pentane/EtOAc 1:1) 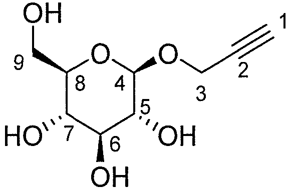
**HRMS** (ESI): Calculated for C₉H₁₄NaO₆⁺ [M+Na]⁺: 241.0683, found: 241.0685.

### Acetyl-ω-[4-methyl-β-d-glucopyranosidyl-1H-1,2,3-triazol-1-yl]-I-lysyl-cyclohexylmycoplanecin A [M113]

8.5 mg (6.65 µmol, 1.0 eq.) of azide **M106** and 2.1 mg (9.62 µmol, 1.5 eq.) of the glucopyranoside **M112** were dissolved in 0.6 mL of a 1:1 mixture of THF/H₂O and treated with 4.00 µL (1.0 M in H₂O, 4.00 µmol, 0.6 eq.) of a freshly prepared sodium ascorbate solution and 3.30 µL (1.0 M, 3.30 µmol, 0.5 eq.) of CuSO_{4(aq.)} at RT.

After 20.5 h, the solvent was removed in vacuo and the residue was purified by automated column chromatography (C₁₈-SiO₂, H₂O/MeCN 9:1 → 0:10), followed by preparative HPLC (H₂O/MeCN 9:1 → 0:10). After lyophilization of the isolated fraction, 4.2 mg (2.81 µmol, 42 %) of the mycoplanecin derivative **M113** were obtained as a colorless lyophilizate.
${\left[α\right]}_{D}^{20} = - 46.4$ (c = 0.25, CHCl₃) 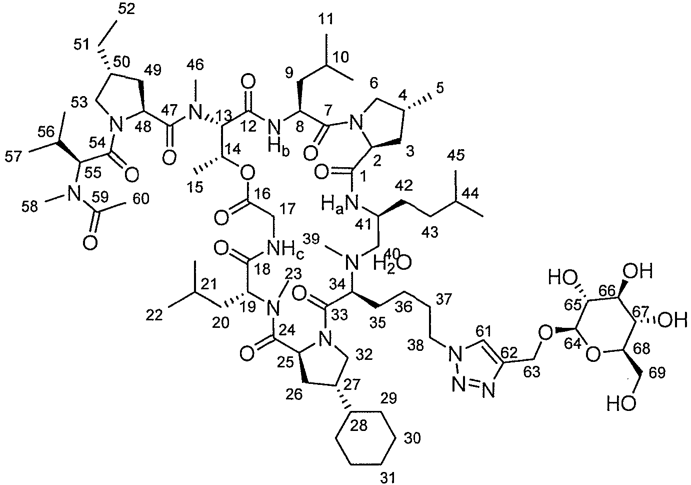
**HRMS** (ESI): Calculated for C₇₅H₁₂₆N₁₃O₁₈⁺ [M+H]⁺: 1496.9338, found: 1496.9333.

### Literature

[1] Z. M. Wang, K. B. Sharpless, J. Org. Chem. 1994, 59, 8302-8303.
[2] W. C. Hiscox, D. S. Matteson, J. Org. Chem. 1996, 61, 8315-8316.
[3] S. T. Cheung, N. L. Benoiton, Can. J. Chem. 1977, 55, 906-910.
[4] L. A. Carpino, D. Sadat-Aalaee, M. Beyermann, J. Org. Chem. 1990, 55, 1673-1675.
[5] U. Kazmaier, A. Horn, E. Papadopoulos, T. Kinsinger, J. Greve, E. Bickel, S. Pachoula, Z. Für Anorg. Allg. Chem. 2024, e202400113.
[6] O. C. Ho, R. Soundararajan, J. Lu, D. S. Matteson, Z. Wang, X. Chen, M. Wei, R. D. Willett, Organometallics 1995, 14, 2855-2860.
[7] A. Horn, U. Kazmaier, Org. Lett. 2022, 24, 7072-7076.
[8] F. Brackmann, H. Schill, A. De Meijere, Chem. - Eur. J. 2005, 11, 6593-6600.
[9] G. Franck, K. Brödner, G. Helmchen, Org. Lett. 2010, 12, 3886-3889.
[10] A. T. Khan, E. Mondal, Synlett 2003, 694-698.
[11] P. Barbie, U. Kazmaier, Org. Lett. 2016, 18, 204-207.
[12] R. M. Wenger, Helv Chim Acta 1983, 66, 2672-2702.
[13] N. Kurokawa, Y. Ohfune, Tetrahedron 1993, 49, 6195-6222.
[14] N. Kurokawa, Y. Ohfune, J. Am. Chem. Soc. 1986, 108, 6043-6045.
[15] F. Gille, A. Kirschning, Beilstein J. Org. Chem. 2016, 12, 564-570.
[16] P. M. E. Hawkins, W. Tran, G. Nagalingam, C. Y. Cheung, A. M. Giltrap, G. M. Cook, W. J. Britton, R. J. Payne, Chem. - Eur. J. 2020, 26, 15200-15205.
[17] A. Titz, Z. Radic, O. Schwardt, B. Ernst, Tetrahedron Lett. 2006, 47, 2383-2385.
[18] A. Le Chevalier Isaad, F. Barbetti, P. Rovero, A. M. D'Ursi, M. Chelli, M. Chorev, A. M. Papini, Eur. J. Org. Chem. 2008, 2008, 5308-5314.
[19] Y. Otake, Y. Shibata, Y. Hayashi, S. Kawauchi, H. Nakamura, S. Fuse, Angew. Chem. 2020, 132, 13025-13030.
[20] M. Nakajima, A. Torikata, Y. Ichikawa, T. Katayama, A. Shiraishi, T. Haneishi, A. Mamoru, J. Antibiot. (Tokyo) 1983, 36, 961-966.
[21] O. Rasheed, A. Lawrence, P. Quayle, P. Bailey, Synlett 2015, 27, 905-911.
[22] W. Ma, J. Bi, C. Zhao, Y. Gao, G. Zhang, Carbohydr. Res. 2020, 491, 107977.
[23] K. Jiang, D. Fan, Y. Belabassi, G. Akkaraju, J.-L. Montchamp, J. L. Coffer, ACS Appl. Mater. Interfaces 2009, 1, 266-269.

### Biological evaluation

### 1. Minimal Inhibitory concentration (MIC) determination

To determine the inhibitory activity of the mycoplanecins, the minimum inhibitory concentration (MIC) assay was conducted against *Mycobacterium tuberculosis* (*Mtb*) strains H37Ra (in-house) and H37Rv (Evotec ID, Lyon, France). *Mtb* cultures were grown to mid-log phase (optical density at 600 nm [OD600] of 0.6) in Middlebrook 7H9 complete medium (BD Difco; Becton Dickinson) supplemented with 10% Middlebrook Oleic Albumin Dextrose Catalase (OADC, BD), 0.4% glycerol, and 0.05% Tween 80 (Merck) at °C and 5% CO2. Single cells were prepared by thoroughly suspending the culture using a 26-gauge syringe needle in Middlebrook 7H9 medium. The cultures were harvested and centrifuged at 3,700 × g for 10 minutes. The resulting pellet was reconstituted in phosphate buffered saline (PBS) and stored at -80°C in 1.5 mL aliquots prior to testing. The number of colony forming units (CFU) was determined by culturing on Middlebrook 7H11 complete medium (BD Difco; Becton Dickinson) supplemented with 10% OADC.

Compounds were tested in 2-fold serial dilutions starting from 128 µM and 1.28 µM as the highest concentrations. MIC values were validated using a Resazurin microtiter assay (REMA) by adding 45 µL of 0.01% Resazurin (Cayman) solution to each well, followed by 20 hours of incubation without agitation. Fluorescence was measured with a microplate reader (Tecan Infinite M200Pro) at an excitation wavelength of 570 nm and an emission wavelength of 590 nm.

### 2. Cytotoxicity evaluation

HepG2 cells (human hepatoblastoma cell line; ACC 180, DSMZ) and CHO-K1 cells (Chinese hamster (*Cricetulus griseus*) ACC 110) were handeled under conditions recommended by the depositor. The cell lines were cultured in Dulbecco's Modified Eagle Medium (DMEM) supplemented with 10% fetal bovine serum (FBS). To determine the anti-proliferative activity of test compounds, cells were seeded at 6 × 10³ cells per well in 96-well plates containing 120 µL of complete medium. After 2 hours of equilibration, compounds were added in serial dilutions in 60 µL of complete medium. Compounds, the solvent control, and doxorubicin (reference compound) were tested in duplicate across two independent experiments.

Following a 5-day incubation period, 20 µL of 5 mg/mL MTT (thiazolyl blue tetrazolium bromide) in PBS was added to each well, and cells were incubated for an additional 2 hours at 37 °C. The medium was then discarded, and cells were washed with 100 µL of PBS before adding 100 µL of 2-propanol/10 N HCl (250:1) to dissolve formazan granules. Absorbance at 570 nm was measured using a microplate reader (Tecan Infinite M200Pro). Cell viability was expressed as a percentage relative to the solvent control. IC₅₀ values were determined by sigmoidal curve fitting using GraphPad PRISM 8 (GraphPad Software, San Diego, CA, USA).

### 3. Vesicle Based Efflux (ABC) transporter screening inhibition

The assay was conducted at Cyprotex (United Kingdom) to evaluate the ability of mycoplanecis to inhibit the active uptake of a known probe substrate into inside-out vesicles that express a single ATP-binding cassette (ABC) efflux transporter, specifically BSEP (Bile Salt Export Pump). The below described protocol is adopted and summarized from the Proposal for ADMET services provided by Cyprotex.

The vesicle inhibition assay was performed in a 96-well plate using a rapid filtration system (vacuum manifold). A reaction mixture containing a specified amount of vesicles per well and radiolabeled probe substrate in assay buffer (10 mM HEPES-Tris pH 7.4, 100 mM KNO₃, 12.5 mM Mg(NO₃)₂, and 50 mM sucrose) was pre-incubated at 37 °C for 5 minutes at 300 rpm with solvent (DMSO), mycoplanecins, or positive control inhibitor. Uptake was initiated by adding 5 mM ATP or AMP (solvent control 0 µM only) and incubating at 37 °C for a set time with gentle shaking (300 rpm). The assay was terminated by transferring the mixture to a 96-well filter plate pre-treated with 100 µM unlabeled taurocholic acid and containing cold washing buffer (10 mM HEPES-Tris pH 7.4, 100 mM KNO₃, and 50 mM sucrose). The filter plate was washed with cold washing buffer, and after washing and filtration, Mammalian Protein Extraction Reagent (MPER) was added to the sample wells. After a 10-minute incubation at room temperature with shaking (350 rpm) to lyse the vesicles, samples were eluted into a collection plate by centrifugation. An aliquot of the supernatant was transferred to a white-walled, clearbottomed multiwell plate, and the released radiolabeled probe substrate was analyzed by liquid scintillation counting to measure disintegrations per minute (dpm). All assay conditions were tested in duplicate wells.

For data analysis, corrected uptake (pmol/mg) was calculated by subtracting solvent uptake in the presence of AMP from uptake in the presence of ATP. The results from solvent wells (0 µM test article) were defined as 100% uptake, which was then used to calculate the percentage control transport activity for all other test article concentrations. These results were plotted against test article concentration and fitted to determine an IC₅₀ value (the concentration that produces 50% inhibition of transport activity).

### 4. Metabolic Stability in human and mouse liver microsomes

For the evaluation of phase I metabolic stability, the compound (1 µM) was incubated with either 0.5 mg/mL pooled human or mouse liver microsomes (Xenotech, Kansas City, USA), 2 mM NADPH, 10 mM MgCl2 at 37 °C for 120 min on a microplate shaker (Eppendorf, Hamburg, Germany). The metabolic stability (of testosterone, verapamil and ketoconazole for mouse microsome, and of testosterone, diclofenac and propranolol for human microsomes) was determined in parallel to confirm the enzymatic activity of liver microsomes. The incubation was stopped after defined time points by precipitation of aliquots of enzymes with 2 volumes of cold internal standard solution (15 nM diphenhydramine in 10% methanol/acetonitrile). Samples were stored on ice until the end of the incubation and precipitated protein was removed by centrifugation (15 min, 4 °C, 4,000 g). Concentration of the remaining test compound at the different time points was analyzed by HPLC-MS/MS (Vanquish Flex coupled to a TSQ Altis Plus, Thermo Fisher, Dreieich, Germany) and used to determine half-life (t_{1/2}).

### Bioactivity data

| Compound | MIC (µM) | | | IC50 (µM) | |
|---|---|---|---|---|---|
| | *Mtb* H37 Ra | *Mtb* H37 Rv (IC50) | *Mtb* H37 Rv (IC90) | HepG2 ACC 180 (DSMZ) | CHO-K1 ACC 110 (DSMZ) |
| M57 | 1.563 nM | 12 nM | 27 nM | 31.995±3.8 | 7.94±4.8 |
| M58 | 3.906 nM | 10 nM | 20 nM | > 37 | 7.41±2.3 |
| M60 | 7.813 nM | 13 nM | 23 nM | > 37 | 9.51±0.1 |
| M61 | 31.25 nM | 58 nM | 100 nM | > 37 | 11.19±0.6 |
| M59 | 0.5 | 0.31 | 0.53 | > 37 | > 37 |
| M62 | 0.25 | 40 nM | 73 nM | > 37 | > 37 |
| M83 | 30 nM | 19 nM | 36 nM | > 37 | > 37 |
| M82 | 30 nM | 29 nM | 98 nM | > 37 | > 37 |
| M81 | 30 nM | 14 nM | 25 nM | > 37 | > 37 |
| M104 | 0.64 | 0.4 | 0.7 | > 37 | 19.6 |
| M105 | 0.64 | 0.34 | 0.6 | > 37 | 21.8 |
| M107 | 0.16 | 0.14 | 0.78 | 26.9 | 34.6 |
| M108 | 0.32 | 0.42 | 0.73 | > 37 | 23.5 |
| M109 | 80 nM | 0.1 | 0.23 | 11.2 | 6.5 |
| M111 | 0.32 | 0.15 | 0.85 | > 37 | > 37 |
| M113 | 0.64 | 0.25 | 0.4 | > 37 | > 37 |
| M114 | 40 nM | 25 nM | 67 | 13.76±1.11 | 17.15±4.18 |
| M115 | 20 nM | 23 nM | 67 | > 37 | 14.2 |

### Metabolic stability

| Compound | MLM t1/2 [min] / Clint [µl/mg/min] | Mouse Hepatocytes t1/2 [min] | HLM t1/2 [min] / Clint [µl/mg/min] | Mouse Plasma t1/2 [min] | Human Plasma t1/2 [min] |
|---|---|---|---|---|---|
| M57 | >120 / <11.6 (n=1) | > 120 (n=1) | >120 / <11.6 (n=1) | > 240 | > 240 |
| | 43 ± 18 / 36 ± 15 | | | | |
| M58 | 36.8 ± 2.8 37.9 ± 2.9 | | >120 / <11.6 | > 240 | > 240 |
| M60 | >120 / <11.6 | | >120 / <11.6 | >240 | >240 |
| M61 | >120 / <11.6 | | >120 / <11.6 | >240 | >240 |
| M59 | >120 / <11.6 (n=1) | | >120 / <11.6 | >240 | >240 |
| | 104 / 11.3 (n=1) | | | | |
| M62 | >120 / <11.6 | | >120 / <11.6 | >240 | >240 |
| M83 | 65.7 ± 0.28 / 21.3 ± 0.28 | | | | |
| M82 | >120 / <11.6 (n=1) | | | | |
| M81 | 16.0 / 86.5 (n=1) | | | | |
| M104 | 17.8 ± 3.2 / 79 ± 13 | | | | |
| M105 | >120 / <11.6 | | | | |
| M107 | >120 / <11.6 | | | | |
| M108 | >120 / <11.6 | | | | |
| M109 | >120 / <11.6 | | | | |
| M111 | 4.4 ± 0.42 / 314 ± 28 | | | | |
| M113 | >120 / <11.6 | | | | |
| M114 | | | | | |
| M115 | | | | | |

### BSEP

| Compound | % HEK-BSEP Activity Remaining at 30 µM |
|---|---|
| M57 | 5,2 |
| M58 | 9,2 |
| M60 | 27 |
| M61 | 19.6 |
| M59 | 43,45 |
| M62 | 41 |
| M83 | 30 |
| M82 | 14.2 |
| M81 | 15,05 |
| M104 | 31,85 |
| M105 | 32,8 |
| M107 | 2,2 |
| M108 | 36,55 |
| M109 | 5,05 |
| M111 | 4,3 |
| M113 | 2,8 |
| M114 | 1,7 |
| M115 | 1,7 |

## Claims

1. A compound of formula (I): 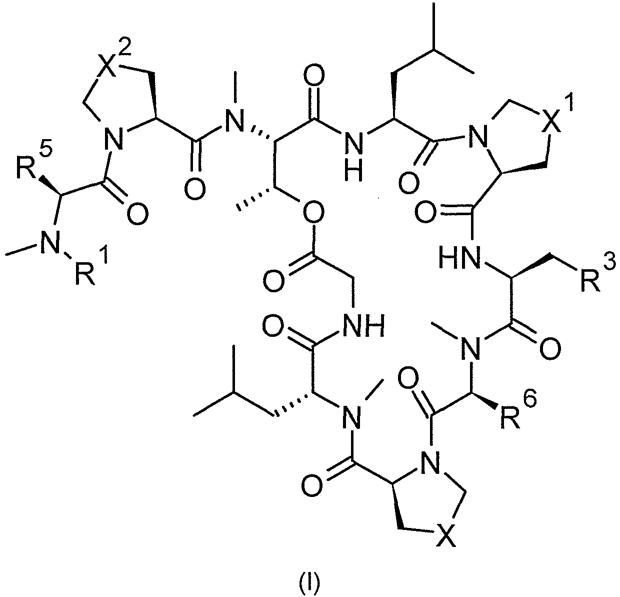 wherein
X is SO₂ or CR⁴R^{4a};
X¹ is SO₂ or CR⁷R^{7a};
X² is SO₂ or CR²R^{2a};
R¹ is a group of formula -C(=O)-C(=O)-R^{1a}; -C(=O)-R^{1a}; or -C(=O)-O-R^{1a};
R^{1a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R² is hydrogen; fluorine; a C₁₋₄ alkyl group; a C₁₋₄ alkyloxy group; a C₁₋₄ alkenyloxy group; or a cyclohexyl group; and R^{2a} is hydrogen; or fluorine; or R² and R^{2a} together with the carbon atom to which they are bound, form a cyclopropyl group;
R³ is a C₁₋₆ alkyl group;
R⁴ is hydrogen; fluorine; a C₁₋₄ alkyl group; a C₁₋₄ alkyloxy group; a C₁₋₄ alkenyloxy group; or a cyclohexyl group; and R^{4a} is hydrogen; or fluorine; or R⁴ and R^{4a} together with the carbon atom to which they are bound, form a cyclopropyl group;
R⁵ is a C₁₋₆ alkyl group;
R⁶ is a C₁₋₆ alkyl group; a group of formula -(CH₂)₄-N₃; or a group of the following formula: 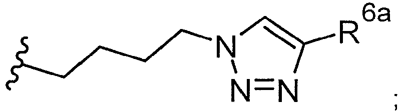
R^{6a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R⁷ is hydrogen; fluorine; a C₁₋₄ alkyl group; a C₁₋₄ alkyloxy group; a C₁₋₄ alkenyloxy group; or a cyclohexyl group; and R^{7a} is hydrogen; or fluorine; or R⁷ and R^{7a} together with the carbon atom to which they are bound, form a cyclopropyl group;
or a salt thereof.

2. A compound of formula (la): 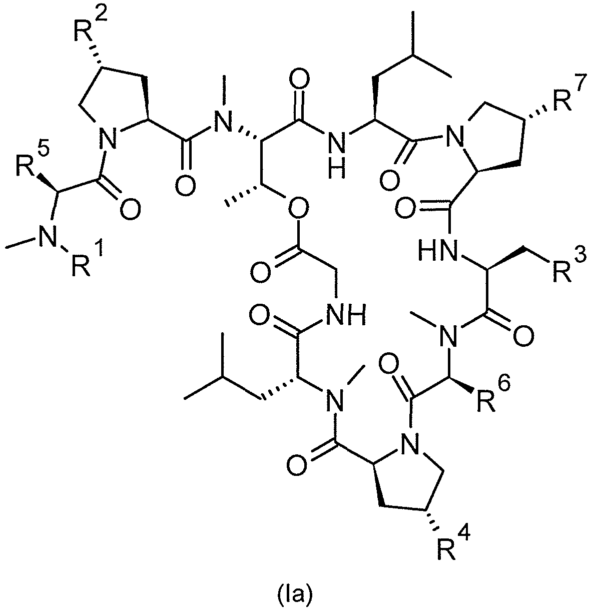 wherein
R¹ is a group of formula -C(=O)-C(=O)-R^{1a}; -C(=O)-R^{1a}; or -C(=O)-O-R^{1a};
R^{1a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R² is hydrogen; a C₁₋₄ alkyl group; a C₁₋₄ alkyloxy group; or a C₁₋₄ alkenyloxy group;
R³ is a C₁₋₆ alkyl group;
R⁴ is hydrogen; a methyl group; a C₁₋₄ alkyloxy group; or a cyclohexyl group;
R⁵ is a C₁₋₆ alkyl group;
R⁶ is a C₁₋₆ alkyl group; a group of formula -(CH₂)₄-N₃; or a group of the following formula: 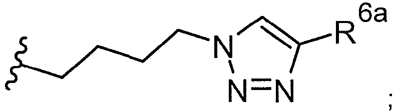
R^{6a} is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R⁷ is hydrogen; or a methyl group;
or a salt thereof.

3. A compound according to claim 1 or 2, wherein R^{1a} is a C₁₋₁₂ alkyl group; a C₁₋₁₂ alkenyl group; a C₃₋₇ cycloalkyl group; a C₁₋₆ alkyl-C₃₋₇ cycloalkyl group; an optionally substituted phenyl group; a C₁₋₆ alkyl-phenyl group, wherein the phenyl may optionally be substituted; or a C₁₋₆ alkyl-heterocycloalkyl group, wherein the heterocycloalkyl group contains 5 or 6 ring atoms that are independently selected from C, N, O and S; especially wherein R^{1a} is a C₁₋₁₂ alkyl group; a C₁₋₁₂ alkenyl group; a C₃₋₇ cycloalkyl group; a C₁₋₆ alkyl-C₃₋₇ cycloalkyl group; or an optionally substituted phenyl group.

4. A compound according to claim 1, 2 or 3, wherein R¹ a group of formula -C(=O)-C(=O)-R^{1a}; especially wherein R^{1a} is an ethyl group.

5. A compound according to claim 1, 2 or 3, wherein R¹ is a group of formula - C(=O)-R^{1a}.; especially wherein R^{1a} is a C₁₋₉ alkyl group; a cyclohexyl group; a group of formula -CH₂-CH₂-CH₂-cyclohexyl; or a 4-fluorophenyl group.

6. A compound according to claim 1, 2 or 3, wherein R¹ is a group of formula - C(=O)-O-R^{1a}; especially wherein R^{1a} is group of formula -CH₂-CH=CH₂.

7. A compound according to any one of the preceding claims, wherein R² is hydrogen; a methyl group; an ethyl group; a methoxy group; or an ethoxy group; especially wherein R² is a methyl group; or an ethyl group.

8. A compound according to any one of the preceding claims, wherein R³ is an isobutyl group; or an isopropyl group.

9. A compound according to any one of the preceding claims, wherein R⁵ is a group of formula -CH(CH₃)₂ or -(CH₂)₃CH₃.

10. A compound according to any one of the preceding claims, wherein R⁶ is a C₁₋₆ alkyl group; especially a group of formula -CH(CH₃)₂.

11. A compound according to any one of the preceding claims 1 to 9, wherein R⁶ is a group of formula -(CH₂)₄-N₃; or a group of the following formula:

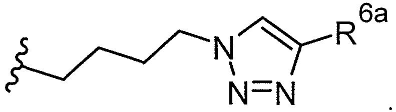

12. A compound according to any one of the preceding claims, wherein R⁷ is hydrogen.

13. A compound according to any one of the preceding claims, wherein R⁴ is a methyl group; or a cyclohexyl group.

14. Pharmaceutical composition comprising a compound according to anyone of the preceding claims and optionally one or more carrier substances and/or one or more adjuvants.

15. Compound according to any one of claims 1 to 13 or pharmaceutical composition according to claim 14 for use in the treatment of a bacterial infection; especially for use in the treatment of tuberculosis.
